# EUROPÄISCHE PATENTSCHRIFT

(11) **EP 2 687 586 B1**
(45) Veröffentlichungstag und Bekanntmachung des Hinweises auf die Patenterteilung: **23.11.2016**
(21) Anmeldenummer: 12176718.0
(22) Anmeldetag: 17.07.2012
(51) Int. Cl.: C11B 9/00, A61L 9/02, A61Q 13/00, C07C 49/603, C11D 3/50, A23L 2/39, A23L 2/56, C11D 3/20, A23G 3/36, A61L 9/01, A61K 8/35

(54) **Verwendung definierter Cyclohexenone als Mittel zum überadditiven Verstärken eines Geruchseindrucks sowie Riech- und/oder Geschmacksstoffkomposition**
Use of defined cyclohexenones as a means for the additive reinforcement of a smell impression and composition of aromas and/or tastes
Utilisation de cyclohexenone définie comme moyen de renforcement supplémentaire d'une impression olfactive ainsi que la composition d'aromates et/ou de parfums

(43) Veröffentlichungstag der Anmeldung: 22.01.2014
(73) Patentinhaber: Symrise AG, 37603 Holzminden (DE)
(72) Erfinder: Ryppa, Claudia, 37603 Holzminden (DE); Hölscher, Bernd, 37620 Halle (DE)
(74) Vertreter: Fabry, Bernd

(56) Entgegenhaltungen:
- EP-A1- 0 133 548
- WO-A2-02/39971
- DE-A1- 2 257 121
- DE-A1- 3 640 591
- US-A- 4 485 828

## Beschreibung

Die vorliegende Erfindung betrifft die Verwendung einer Verbindung der Formel (I) 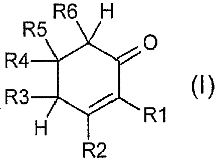 oder einer Mischung bestehend aus oder umfassend zwei, drei, vier, fünf, sechs oder mehr verschiedenen Verbindungen der Formel (I) zum überadditiven Verstärken eines Geruchseindrucks. Die Reste R1 bis R6 haben die nachfolgend definierte Bedeutung.

Die Erfindung betrifft auch neue Riech- und/oder Geschmacksstoffkompositionen, die neben einer Verbindung der Formel (I) oder einer Mischung davon, wie jeweils hier beschrieben, einen, zwei, drei, oder mehr weitere Riech- und/oder Geschmacksstoffe, wobei der beziehungsweise die weiteren Riech- und/oder Geschmacksstoffe keine Verbindungen der Formel (I) sind, enthalten.

Weiterhin betrifft die vorliegende Erfindung parfümierte und/oder aromatisierte Artikel, die eine entsprechende Riechstoff- und/oder Geschmacksstoffmischung gemäß der vorliegenden Erfindung enthalten.

Weiterhin betrifft die vorliegende Erfindung parfümierte und/oder aromatisierte Artikel, die eine entsprechende Riechstoff- und/oder Geschmacksstoffmischung gemäß der vorliegenden Erfindung enthalten.

Die vorliegende Erfindung betrifft ebenfalls Verfahren zur Herstellung einer Verbindung der Formel (I) sowie ein Verfahren zum überadditiven Verstärken eines Geruchseindrucks.

Die Erfindung betrifft schließlich auch neue, besonders vorteilhafte Verbindungen der Formel (I) und entsprechende Mischungen. Zudem wird die Verwendung besagter neuer Verbindungen als Riechstoff, Geschmacksstoff oder Mittel zum überadditiven Verstärken eines Geruchs beschreiben.

Weitere Aspekte der vorliegenden Erfindung ergeben sich aus der nachfolgenden Beschreibung, den Beispielen sowie insbesondere den beigefügten Patentansprüchen.

In der Riechstoffindustrie besteht ein ständiger Bedarf, bestimmte geruchliche Aspekte eines Riechstoffs und/oder Geschmacksstoffs oder einer Riechstoff- und/oder Geschmacksstoffmischung zu betonen (hervorzuheben) und/oder überadditiv zu verstärken. Insbesondere besteht stets Bedarf, florale und/oder fruchtige Geruchseindrücke von Riechstoffen und/oder Geschmacksstoffen oder Riechstoff- und/oder Geschmacksstoffmischungen zu betonen (hervorzuheben) und/oder überadditiv zu verstärken.

Die Aufgabe der vorliegenden Erfindung war es, bestimmte geruchliche Aspekte von Riech- und/oder Geschmacksstoffen oder Riech- und/oder Geschmacksstoffmischungen überadditiv zu verstärken. Gelöst wird diese Aufgabe durch die Verwendung
- einer Verbindung der Formel (I) 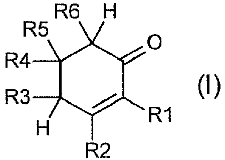 oder
- einer Mischung bestehend aus oder umfassend (vorzugsweise bestehend aus) zwei, drei, vier, fünf, sechs oder mehr verschiedenen Verbindungen der Formel (I), wobei in jeder der Verbindungen der Formel (I) die Reste R1, R2, R3, R4, R5 und R6 jeweils unabhängig voneinander die folgende Bedeutung haben
   R1 bedeutet Wasserstoff, Methyl oder Ethyl,
   R2 bedeutet Wasserstoff, Methyl oder Ethyl,
   R3 bedeutet Wasserstoff, Methyl, Ethyl oder Isopropyl,
   R4 bedeutet Wasserstoff, Methyl, Ethyl, n-Propyl oder Isopropyl,
   R5 bedeutet Wasserstoff oder Methyl,
   R6 bedeutet Wasserstoff, Methyl oder Ethyl,
   mit der Maßgabe, dass R1 nicht Methyl oder Ethyl ist, wenn R6 Methyl oder Ethyl ist,
   zum überadditiven Verstärken eines Geruchseindrucks.

Ein Geruchseindruck ist ein geruchlicher Eindruck, der durch ein oder mehrere Deskriptoren beschrieben werden kann. Im Rahmen des vorliegenden Textes sind solche Geruchseindrücke besonders relevant, die durch einen oder mehrere Deskriptoren beschrieben werden können, die ausgewählt sind aus der Gruppe bestehend aus floral und fruchtig.

Unter einer überadditiven Verstärkung eines Geruchseindrucks (auch bezeichnet als synergistische Verstärkung eines Geruchseindrucks) wird der Effekt verstanden, bei dem es durch Zugabe der Verbindung der Formel (I) zu einer, zwei, drei oder mehr weiteren Riech- und/oder Geschmacksstoffen, wobei der bzw. die weiteren Riech- und/oder Geschmacksstoffe keine Verbindungen der Formel (I) sind, zu einem Geruchseindruck kommt, der stärker ist als die Summe der Einzel-Geruchseindrücke, Besitzt ein (weiterer) Riech- und/oder Geschmacksstoff beispielsweise einen fruchtigen oder floralen Geruchseindruck (neben gegebenenfalls weiteren Geruchseindrücken), so liegt eine überadditive Verstärkung des Geruchseindrucks vor, wenn der fruchtige bzw. florale Gesamt-Geruchseindruck der resultierenden Mischung umfassend die Verbindung der Formel (I) sowie den (weiteren) Riech- und/oder Geschmacksstoff stärker ist als die Summe der floralen bzw. fruchtigen Geruchseindrücke von einerseits der Verbindung der Formel (I) und andererseits dem (weiteren) Riech- und/oder Geschmacksstoff.

Im Rahmen der vorliegenden Erfindung wurden auch neue Verbindungen der obigen Formel (I) sowie entsprechende Mischungen gefunden. Solche sind weiter unten beschrieben.

Es war besonders überraschend, dass sich gerade die hierin beschriebenen Verbindungen der Formel (I) für die Zwecke der vorliegenden Erfindung eignen. Denn die Suche nach geeigneten Mitteln zum überadditiven Verstärken eines Geruchseindrucks, wurde - und wird üblicherweise - durch folgende Gegebenheiten erschwert:
- Die Mechanismen der Geruchswahrnehmung sind (noch) nicht ausreichend bekannt.
- Die Zusammenhänge zwischen der speziellen Geruchswahrnehmung einerseits und der chemischen Struktur des zugehörigen Riechstoffs andererseits sind (noch) nicht hinreichend erforscht.
- Häufig bewirken bereits geringfügige Änderungen am strukturellen Aufbau eines bekannten Riechstoffs oder eines Mittels zum überadditiven Verstärken eines Geruchseindrucks, starke Änderungen der sensorischen (oder anderer) Eigenschaften. Diese können zudem die Verträglichkeit für den menschlichen Organismus beeinträchtigen.
- Der Mechanismus zum Verstärken von Geruchseindrücken ist (noch) nicht ausreichend erforscht. Somit kann keine Voraussage getroffen werden, ob und wann eine Verbindung in der Lage ist, einen Geruchseindruck einer anderen Substanz zu verstärken.

Es ist nicht vorhersehbar, wie sich - ausgehend von einer vorgegebenen Grundstruktur - strukturelle Änderungen auf die Wechselwirkungen einer jeweiligen Verbindung mit anderen Riech- oder Geschmacksstoffen, deren Geruch z.B. verstärkt werden soll, auswirken. Nicht vorhersehbar sind somit die resultierenden sensorischen (sowie gegebenenfalls auch weitere) Eigenschaften einer entsprechenden Riech- und/oder Geschmacksstoffkomposition.

In Abhängigkeit davon, welche Bedeutung die Reste R1 bis R6 in der Verbindung der Formel (I) aufweisen, können unterschiedliche Stereozentren in der Verbindung der Formel (I) vorhanden sein. Eine Verbindung der Formel (I) oder Mischungen davon können dabei jeweils als reine Konfigurationsisomere, bei denen alle Stereozentren die identische Konstitution aufweisen, oder als Mischungen von Konfigurationsisomeren vorliegen. Sofern eine Mischung verschiedener Konfigurationsisomere vorliegt, wird diese Mischung im Rahmen des vorliegenden Textes nicht als Mischung bezeichnet, sofern die Reste R1 bis R6 in den Konfigurationsisomeren die gleiche Bedeutung aufweisen.

Überraschenderweise besitzen einige der Verbindungen der Formel (I) bzw. deren Mischungen besonders ausgeprägte Eigenschaften zur überadditiven Verstärkung eines Geruchseindrucks. Entsprechend ist die erfindungsgemäße Verwendung einer einzelnen Verbindung oder einer Mischung besonders bevorzugt, wobei die einzelne Verbindung der Formel (I) bzw. eine, mehrere oder sämtliche Verbindungen der Formel (I) ausgewählt ist bzw. jeweils unabhängig voneinander ausgewählt sind aus der Gruppe bestehend aus den folgenden Verbindungen (1) bis (61), vorzugsweise ausgewählt aus der Gruppe bestehend aus den Verbindungen (2), (4), (13), (23), (29) und (43):

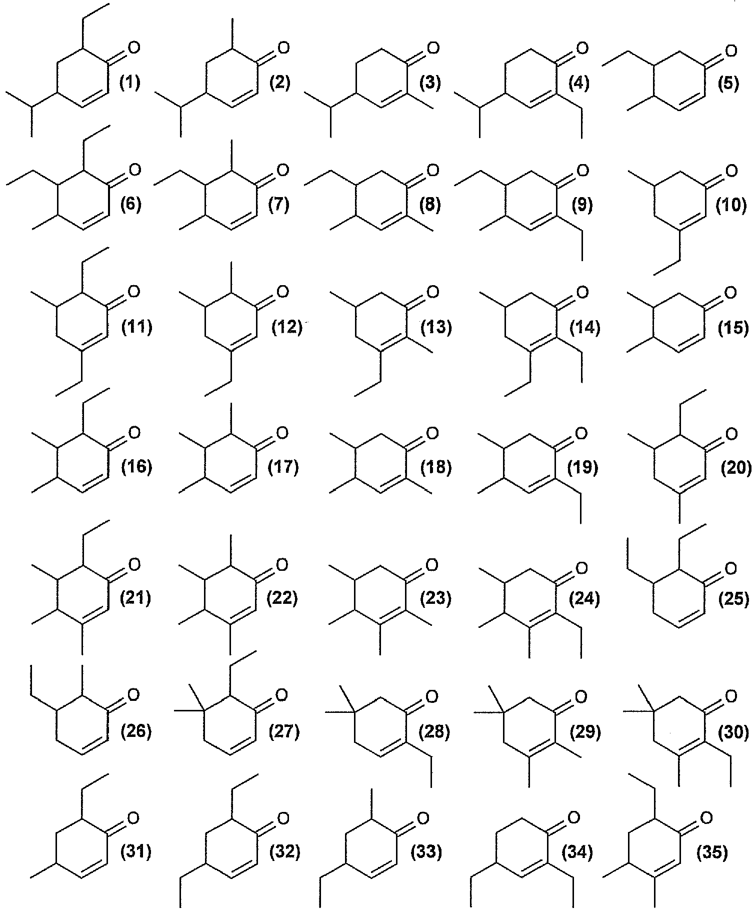

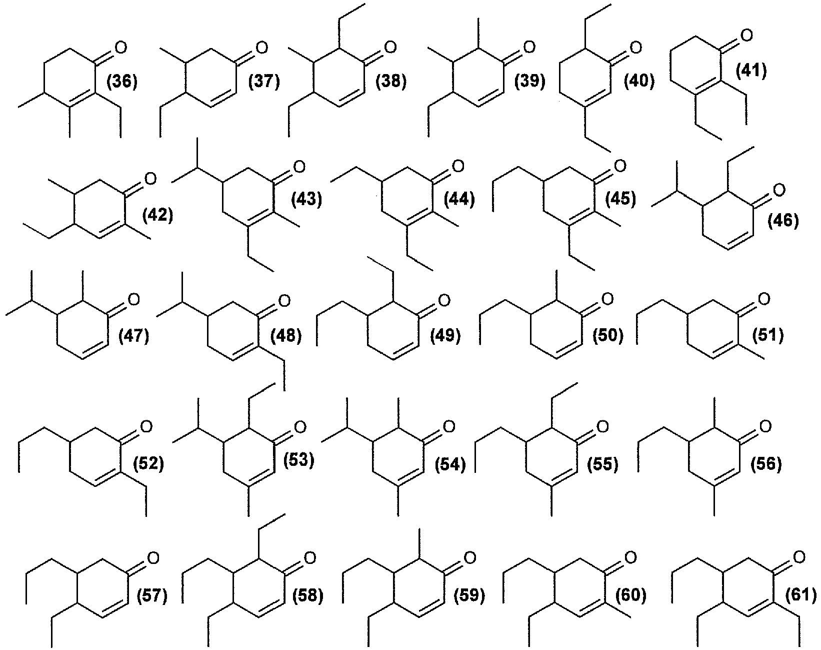

Überraschenderweise hat sich gezeigt, dass die Verbindung der Formel (I) oder eine Mischung bestehend aus oder umfassend zwei, drei, vier, fünf, sechs oder mehr verschiedene Verbindungen der Formel (I) wie oben beschrieben, insbesondere florale und/oder fruchtige Geruchseindrücke überadditiv verstärken können. Eine erfindungsgemäße Verwendung als Geruchsverstärker zum überadditiven Verstärken floraler und/oder fruchtiger Geruchseindrücke ist daher besonders bevorzugt.

Durch die Verwendung der Verbindung der Formel (I) oder einer Mischung bestehend aus oder umfassend zwei, drei, vier, fünf, sechs oder mehr verschiedene Verbindungen der Formel (I) wie oben beschrieben, lassen sich bereits in geringer Dosierung in den resultierenden Riech- und/oder Geschmacksstoffkompositionen florale und/oder fruchtige Duftnoten überadditiv verstärken, wobei der geruchliche Gesamteindruck zudem in der Regel auffallend harmonisiert und die Ausstrahlung wahrnehmbar erhöht sind (vgl. Beispiel 42).

Die Verbindung der Formel (I) oder eine Mischung bestehend aus oder umfassend zwei, drei, vier, fünf, sechs oder mehr verschiedene Verbindungen der Formel (I) eignen sich hervorragend als Mittel zum überadditiven Verstärken eines Geruchseindrucks, insbesondere eines floralen und/oder fruchtigen Geruchseindrucks.

In resultierenden Kompositionen sind einige Geruchseindrücke häufig nicht vorteilhaft. Besonders bevorzugt ist entsprechend eine erfindungsgemäße Verwendung, wobei der zu verstärkende Geruchseindruck nicht ausgewählt ist aus der Gruppe bestehend aus nussig, haselnussig, pistazieartig, kakaoartig, karamellig, fleischig, röstgetreideartig, schokoladig und gewürzartig und/oder wobei die Verbindung der Formel (I) oder die entsprechende Mischung (wie soeben definiert) keinen Geruchseindruck vermittelt, der ausgewählt ist aus der Gruppe bestehend aus nussig, haselnussig, pistazieartig, kakaoartig, karamellig, fleischig, röstgetreideartig, schokoladig und gewürzartig.

Der Fachmann wird in einer erfindungsgemäßen Verwendung einer Verbindung der Formel (I) oder einer Mischung bestehend aus oder umfassend zwei, drei, vier, fünf, sechs oder mehr verschiedener Verbindungen der Formel (I), den Anteil der Verbindung der Formel (I) oder der Mischung der Verbindungen der Formel (I) so wählen, dass der von ihm gewünschte Effekt des überadditiven Verstärkens einer Geruchsnote erreicht wird, wobei er vorzugsweise Sorge trägt, einerseits keine zu großen Mengen der Verbindung der Formel (I) oder der Mischung der Verbindungen der Formel (I) einzusetzen, welche den sensorischen Gesamteindruck einer entstehenden Riech- und/oder Geschmacksstoffkomposition dominieren könnte und andererseits nicht lediglich eine so geringe Menge der Verbindung der Formel (I) oder der Mischung der Verbindungen der Formel (I) vorzusehen, dass eine Verstärkung geruchlicher Aspekte nicht beziehungsweise nicht in signifikantem Maße zu spüren ist. Da die zum überadditiven Verstärken verwendeten Verbindungen der Formel (I) einen Eigengeruch aufweisen können, besteht natürlich auch die Möglichkeit, diesen Eigengeruch der Verbindungen der Formel (I) in einer Riech- und/oder Geschmacksstoffkomposition (wie sie nachfolgend beschrieben wird) auszunutzen, sodass neben dem gewünschten Effekt des überadditiven Verstärkens einer Geruchsnote auch der Eigengeruch der verwendeten Verbindung der Formel (I) zur Geltung kommt.

In Anbetracht des Standes der Technik war es besonders überraschend, dass nunmehr neue, besonders wertvolle Mittel zum überadditiven Verstärken eines Geruchseindrucks identifiziert werden konnten. Darüber hinaus verfügen die erfindungsgemäß zu verwendenden Verbindungen der Formel (I), insbesondere die neuen Verbindungen der Formel (I), eigenständige olfaktorische Eigenschaften. Dabei weisen insbesondere die neuen Verbindungen der Formel (I) Geruchsnoten auf, die überraschend sind, siehe dazu unten. Eine geruchsverstärkende Wirkung der erfindungsgemäß zu verwendenden Verbindungen der Formel (I) wurde bisher noch nicht beschrieben.

Eine Auswahl bekannter Cyclohexenone ist nachfolgend zusammengefasst:

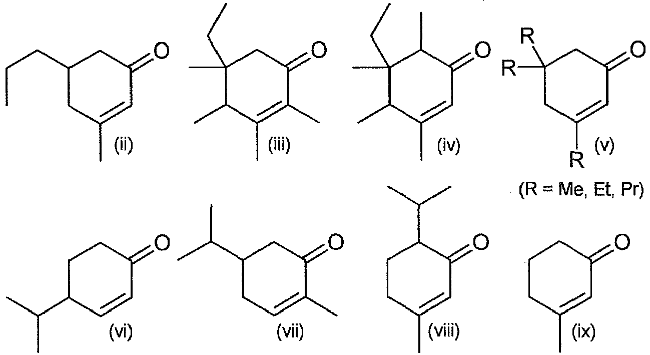

Bekannte Cyclohexenone mit bereits bekannten sensorischen Eigenschaften sind:
- Livescone (ii),
- Substanzen (iii) und (iv) (Federal Register 1967, 32, 7946),
- Isophoron (v, R = Me),
- Crypton (vi) (DE 2551172 A1),
- Carvotanaceton (vii) (J. Chem. Soc., Trans. 1898, 73, 852),
- Piperitone (viii) und
- Seudenone (ix).

Die organoleptischen Eigenschaften von Verbindung (ix) (Seudenone) werden in Perfumer & Flavorist 1998, 23, 56 als süß, nussig, phenolisch, Walnuss, fruchtig und Mandel beschrieben.

In DE 25 511 72 A1 wird Verbindung (2) (wie oben beschrieben) als stark, balsamisch, holzig, lakritzeartig, leicht fruchtig, in DE 36 40 591 A1 Verbindung (3) als süß, krautig, mit Anisnote und Verbindung (4) als blumig, krautig, süßlich, holzig beschrieben. In eigenen Untersuchungen hat es sich überraschenderweise gezeigt, dass Verbindung (2) einen Geruch besitzt, wie er in DE 25 511 72 A1 nicht beschrieben ist. Die Geruchsbeschreibung ist gemäß den eigenen Untersuchungen: frisch, süß, würzig, aromatisch, fruchtig, Grapefruit, Wermuttee. (Vergleiche Beispiel 2).

In Liebigs Ann. 1911, 379, 215 wird die Substanz (3) als pinocarvonartig (gemäß S. Arctander, Perfume and Flavor Materials: camphrig, minzig) beschrieben.

In Chem. Ber. 1948, 81, 197 wird Verbindung (30) als Verbindung mit einem terpenähnlichen Geruch beschrieben.

In J. Econ. Entomol. 1971, 64, 970 wird die Verwendung der Verbindung (ix) als Lockstoff für Borkenkäfer beschrieben.

Verbindungen der allgemeinen Formel (v) (Isophoron wenn R=Me) werden in WO 8500096 A1 als Abwehrmittel für Vögel beschrieben.

Einige Verbindungen der allgemeinen Formel (I) sind als Edukte oder Intermediate für bzw. in verschiedenen Reaktionen bekannt, ohne, dass Geruchs- oder Geschmackseigenschaften beschrieben werden. Beispielsweise wird Verbindung (5) für die Darstellung von Benzoquinonen verwendet (Org. Biomol. Chem. 2005, 3, 3479), Verbindungen (8) und (13) entstehen bei säurekatalysierten Umlagerung von Campher (Tetrahedron 1976, 32, 1699) und Carvomenthon (Bull. Soc. Chim. Fr. 1978, 255). Durch alpha-Alkylierung von Isophoron (Compt. Rend. 1953, 237, 910) oder Isophoroncarboxylat (Bull. Soc. Chim. Fr. 1954, 690) gelangt man zu den Verbindungen (29) und (30), welche u. a. als Ausgangsmaterialien zur Darstellung lichtsensibler Stoffe (US 5356769 A) oder zum Aufbau von Stereoidgerüsten (J. Am. Chem. Soc. 1993, 115, 504) verwendet werden. Siehe auch die nachfolgend aufgeführte Patentliteratur: WO 2012041820 A1, WO 2009066193 A1, WO 2009044310 A1, WO 2007135582 A1, WO 2004000776 A1, WO 2009039675 A1, JP 60045544, JP 60032745, JP 200302722 A, CH 603071 A5 und US 4418087 A.

Verbindungen die nicht unter die allgemeine Formel (I) fallen, aber einzelne strukturelle Übereinstimmungen mit den erfindungsgemäßen Verbindungen der allgemeinen Formel (I) besitzen, finden sich in der nachfolgend aufgeführten Patentliteratur: US 4326997 A, US 4326996 A und CH 611159 A.

In DE 22 57 121 werden die Verbindungen 5-Ethylcyclohexen-2-on und 6-Ethylcyclohexen-2-on als Mittel zur Verbesserung, Steigerung oder Modifizierung von organoleptischen Eigenschaften beschrieben. Zudem werden Cyclohexenone einer allgemeinen Formel II als Bestandteil einer Zusammensetzung angegeben, die zusätzlich mindestens ein Pyrazin oder Pyrazinderivat enthält, Es werden bestimmte Cyclohexenone als Geschmacksmodifizierer beschrieben, wobei die Geschmacksnoten der jeweiligen Verbindungen selbst nicht beschrieben werden.

Wie oben beschrieben, betrifft die vorliegende Erfindung auch neue Verbindungen der Formel (I) sowie entsprechende Mischungen. Dementsprechend betrifft ein weiterer Aspekt der vorliegenden Erfindung
(A) eine erfindungsgemäße Verbindung der Formel (I) 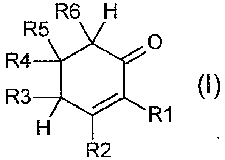 oder
(B) eine Mischung bestehend aus oder umfassend zwei, drei, vier, fünf, sechs oder mehr verschiedene erfindungsgemäße Verbindungen der Formel (I),
   wobei die Verbindung der Formel (I) bzw. eine, mehrere oder sämtliche Verbindungen der Formel (I) ausgewählt ist bzw. jeweils unabhängig voneinander ausgewählt sind aus der Gruppe bestehend aus den folgenden Verbindungen: 6-Ethyl-4-isopropylcyclohex-2-en-1-on (1), 5,6-Diethyl-4-methylcyclohex-2-en-1-on (6), 5-Ethyl-4,6-dimethylcyclohex-2-en-1-on (7), 2,5-Diethyl-4-methylcyclohex-2-en-1-on (9), 3-Ethyl-5-methylcyclohex-2-en-1-on (10), 3,6-Diethyl-5-methylcyclohex-2-en-1-on (11), 3-Ethyl-5,6-dimethylcyclohex-2-en-1-on (12), 2,3-Diethyl-5-methylcyclohex-2-en-1-on (14), 6-Ethyl-4,5-dimethylcyclohex-2-en-1-on (16), 4,5,6-Trimethylcyclohex-2-en-1-on (17), 2,4,5-Trimethylcyclohex-2-en-1-on (18), 2-Ethyl-4,5-dimethylcyclohex-2-en-1-on (19), 6-Ethyl-3,5-dimethylcyclohex-2-en-1-on (20), 6-Ethyl-3,4,5-trimethylcyclohex-2-en-1-on (21), 3,4,5,6-Tetramethylcyclohex-2-en-1-on (22), 2,3,4,5-Tetramethylcyclohex-2-en-1-on (23), 2-Ethyl-3,4,5-trimethylcyclohex-2-en-1-on (24), 5,6-Diethylcyclohex-2-en-1-on (25), 5-Ethyl-6-methylcyclohex-2-en-1-on (26), 6-Ethyl-5,5-dimethylcyclohex-2-en-1-on (27), 2-Ethyl-5,5-dimethylcyclohex-2-en-1-on (28), 6-Ethyl-4-methylcyclohex-2-en-1-on (31), 4,6-Diethylcyclohex-2-en-1-on (32), 4-Ethyl-6-methylcyclohex-2-en-1-on (33), 2,4-Diethylcyclohex-2-en-1-on (34), 6-Ethyl-3,4-dimethylcyclohex-2-en-1-on (35), 2-Ethyl-3,4-dimethylcyclohex-2-en-1-on (36), 4-Ethyl-5-methylcyclohex-2-en-1-on (37), 4,6-Diethyl-5-methylcyclohex-2-en-1-on (38), 4-Ethyl-5,6-dimethylcyclohex-2-en-1-on (39), 3,6-Diethylcyclohex-2-en-1-on (40), 2,3-Diethytcyctohex-2-en-1-on (41), 4-Ethyl-2,5-dimethytcyctohex-2-en-1-on (42), 3-Ethyl-5-isopropyl-2-methylcyclohex-2-en-1-on (43), 3,5-Diethyl-2-methylcyclohex-2-en-1-on (44), 3-Ethyl-2-methyl-5-n-propylcyclohex-2-en-1-on (45), 6-Ethyl-5-isopropylcyclohex-2-en-1-on (46), 5-lsopropyl-6-methylcyclohex-2-en-1-on (47), 2-Ethyl-5-isopropylcyclohex-2-en-1-on (48), 6-Ethyl-5-n-propylcyclohex-2-en-1-on (49), 6-Methyl-5-n-propylcyclohex-2-en-1-on (50), 2-Methyl-5-n-propylcyclohex-2-en-1-on (51), 2-Ethyl-5-n-propylcyclohex-2-en-1-on (52), 6-Ethyl-5-isopropyl-3-methylcyclohex-2-en-1-on (53), 5-lsopropyl-3,6-dimethylcyclohex-2-en-1-on (54), 6-Ethyl-3-methyl-5-n-propylcyclohex-2-en-1-on (55), 3,6-Dimethyl-5-n-propylcyclohex-2-en-1-on (56), 4-Ethyl-5-n-propylcyclohex-2-en-1-on (57), 4,6-Diethyl-5-n-propylcyclohex-2-en-1-on (58), 4-Ethyl-6-methyl-5-n-propylcyclohex-2-en-1-on (59), 4-Ethyl-2-methyl-5-n-propylcyclohex-2-en-1-on (60) und 2,4-Diethyl-5-n-propylcyclohex-2-en-1-on (61), vorzugsweise ausgewählt sind aus der Gruppe bestehend aus 2,3,4,5-Tetramethylcyclohex-2-en-1-on (23) und 3-Ethyl5-isopropyl-2-methylcyclohex-2-en-1-on (43).

Die in Klammern gesetzten Nummern hinter der IUPAC-Bezeichnung der einzelnen Verbindungen beziehen sich auf die oben-aufgeführte Strukturformel. Sofern eine IUPAC-Bezeichnung im Einzelfall nicht einer aufgeführten Strukturformel entspricht, sollen beide aufgeführten Verbindungen (gemäß Strukturformel und gemäß IUPAC-Bezeichnung) berücksichtigt werden.

Die neuen Verbindungen der Formel (I) sowie entsprechende Mischungen sind besonders gut zum überadditiven Verstärken eines Geruchs geeignet, insbesondere zum überadditiven Verstärken eines floralen und/oder fruchtigen Geruchseindrucks.

Eine erfindungsgemäße Mischung (B) umfassend zwei, drei, vier, fünf, sechs oder mehr verschiedene erfindungsgemäße Verbindungen der Formel (I), kann nach Art eines Riechstoffverstärker-Konzentrats ausgebildet sein, welches dazu vorgesehen ist, Basis-Zubereitungen zugesetzt zu werden, um den Geruchseindruck der Basis-Zubereitung überadditiv zu verstärken, beispielsweise unter Ausbildung einer besonders wertvollen Riech- und/oder Geschmacksstoffkomposition. Eine bevorzugte erfindungsgemäße Mischung umfasst entsprechend keinen Riech- und/oder Geschmacksstoff, der keine Verbindung der Formel (I) wie oben definiert ist, und auch keinen Riechstoffverstärker, der keine Verbindung der Formel (I) wie oben definiert ist.

Die Offenlegungsschrift DE 22 57 121 offenbart auf Seite 20 unter Verweis auf weitere Veröffentlichungen eine Reihe von Verbindungen, nämlich Cyclohexen-2-on, 2-Methylcyclohexen-2-on, 3-Methylcyclohexen-2-on, 4-Methylcyclohexen-2-on, 5-Methylcyclohexen-2-on, 6-Methylcyclohexen-2-on, 2,6-Dimethylcyclohexen-2-on, 3,4-Dimethylcyclohexen-2-on, 3,5-Dimethylcyclohexen-2-on, 3,6-Dimethylcyclohexen-2-on, 4,6-Dimethylcyclohexen-2-on, 2,3-Dimethylcyclohexen-2-on, 2,4-Dimethylcyclohexen-2-on, 2,5-Dimethylcyclohexen-2-on, 4,5-Dimethylcyclohexen-2-on, 5,6-Dimethylcyclohexen-2-on, 2,6-Dimethylcyclohexen-2-on, 2-Ethylcyclohexen-2-on, 3-Ethylcyclohexen-2-on, 4-Ethylcyclohexen-2-on, 5-Ethylcyclohexen-2-on und 6-Ethylcyclohexen-2-on. Die jeweils gewählte Bezeichnung erscheint dabei nicht immer korrekt. Die offenbarten Verbindungen sind ebenso wenig Gegenstand der vorliegenden Erfindung wie die entsprechenden Verbindungen, die in den zugrundeliegenden Veröffentlichungen offenbart sind. Vorzugsweise sind eine, zwei oder mehr, bevorzugt sämtliche Verbindungen der Formel (I) in der Mischung nicht ausgewählt aus der Gruppe der besagten Verbindungen. Eine angegebene Verbindung der Formel (I) umfasst in vielen Fällen mehrere Konfigurationsisomere; im vorliegenden Text wird regelmäßig sprachlich nicht weiter zwischen diesen Konfigurationsisomeren unterschieden. Jede Bezugnahme auf eine Verbindung der Formel (I), die mehrere Konfigurationsisomere umfasst, ist im vorliegenden Text als Bezugnahme sowohl auf jedes einzelne der besagten Konfigurationsisomeren als auch als Bezugnahme auf jede einzelne der denkbaren Mischungen von zwei oder (sofern vorhanden) mehr der besagten Konfigurationsisomeren zu verstehen. Beispielsweise entspricht somit die Bezugnahme auf 6-Ethyl-4-isopropylcyclohex-2-en-1-on einer Bezugnahme auf jedes einzelne der Konfigurationsisomeren des 6-Ethyl-4-isopropylcycloshex-2-en-1-on sowie auf jede beliebige Mischung dieser Konfigurationsisomeren. In den Beispielen weiter unten ergeben sich spezifische Informationen zu den eingesetzten bzw. untersuchten Verbindungen aus den angegebenen analytischen Daten.

Überraschenderweise hat sich gezeigt, dass die neuen Verbindungen der Formel (I) (wie oben beschrieben) jeweils auch einen besonders interessanten Geruch und/oder Geschmack aufweisen. Dies ist trotz einer strukturellen Ähnlichkeit dieser neuen Verbindungen mit den bekannten Verbindungen überraschend, denn aus der Struktur einer bekannten ersten Verbindung mit bekannten sensorischen Eigenschaften und aus deren struktureller Ähnlichkeit zur Struktur einer zweiten Verbindung kann nicht auf den Geruch oder Geschmack dieser zweiten Verbindung geschlossen werden. Aus der Literatur sind zahlreiche Beispiele bekannt, in denen bereits durch geringste Variationen der chemischen Struktur (z.B. Austausch von Methylgruppen durch Wasserstoff) zunächst geruchslose Substanzen anschließend einen Geruch aufweisen. Zu der Unmöglichkeit der Geruchsvorhersage siehe C. S. Sell, Angew. Chem. 2006, 118, 6402. Daher ist es in der vorliegenden Situation überraschend, dass die neuen Verbindungen der Formel (I) einen Geruch und/oder Geschmack aufweisen und dass dieser Geruch und/oder Geschmack eigenständige organoleptische, insbesondere olfaktorische und/oder gustatorische, Eigenschaften besitzt, die sich deutlich von den bekannten Verbindungen abheben und diese sogar übertreffen. Insbesondere die in den neuen Verbindungen häufig anzutreffenden Geruchsnoten würzig, Safran, Cumarin, aromatisch, Leder und/oder Tabak unterscheiden sich stark von den aus dem Stand der Technik bekannten Geruchsnoten für Cyclohex-2-en-1-on-Verbindungen.

Die vorliegende Erfindung betrifft somit auch die Verwendung der entsprechenden Verbindungen (1), (2), (3), (4), (5), (8), (9), (10), (12), (13), (14), (15), (18), (19), (23), (24), (28), (29), (30), (31), (32), (33), (34), (37), (40), (41), (42), (43), (44), (45), (48), (51), (52), (57), (60) und (61) als Riechstoff mit einer, zwei, drei, vier, fünf oder sämtlichen Geruchsnoten ausgewählt aus der Gruppe bestehend aus Anis, würzig, Safran, Cumarin, aromatisch, Leder und Tabak.

Besonders bevorzugt ist die kombinierte, d.h. gleichzeitige Verwendung der Verbindungen (1), (2), (3), (4), (5), (8), (9), (10), (12), (13), (14), (15), (18), (19), (23), (24), (28), (29), (30), (31), (32), (33), (34), (37), (40), (41), (42), (43), (44), (45), (48), (51), (52), (57), (60) und (61) zum überadditiven Verstärken eines Geruchseindrucks (vorzugsweise zum überadditiven Verstärken eines floralen und/oder fruchtigen Geruchseindrucks) und als Riechstoff mit einer, zwei, drei, vier, fünf oder sämtlichen Geruchsnoten ausgewählt aus der Gruppe bestehend aus würzig, Anis, Safran, Cumarin, aromatisch, Leder und Tabak.

Überraschenderweise besitzen einige der neuen, insbesondere der vorstehend als bevorzugt bezeichneten Verbindungen eine besonders wertvolle würzige Geruchsnote, vgl. hierzu die Beispiele weiter unten.

Die vorliegende Erfindung betrifft somit auch die Verwendung der entsprechenden Verbindungen (2), (3), (4), (5), (8), (9), (10), (12), (13), (14), (18), (19), (23), (24), (28), (29), (30), (32), (33), (34), (42), (43), (44), (45), (48), (51), (57) und (61) als Riechstoff mit einer würzigen Note.

Überraschenderweise besitzen einige der neuen, insbesondere der vorstehend als bevorzugt bezeichneten Verbindungen eine besonders wertvolle Safran-Geruchsnote, vgl. hierzu die Beispiele weiter unten.

Die vorliegende Erfindung betrifft somit auch die Verwendung der entsprechenden Verbindungen (4), (9), (12), (13), (14), (19), (23), (24), (28), (41) und (30) als Riechstoff mit einer Safran-Note.

Überraschenderweise besitzen einige der neuen, insbesondere der vorstehend als bevorzugt bezeichneten Verbindungen eine besonders wertvolle Cumarin-Geruchsnote, vgl. hierzu die Beispiele weiter unten.

Die vorliegende Erfindung betrifft somit auch die Verwendung der entsprechenden Verbindungen (8), (15), (18) und (34) als Riechstoff mit einer Cumarin-Note.

Überraschenderweise besitzen einige der neuen, insbesondere der vorstehend als bevorzugt bezeichneten Verbindungen eine besonders wertvolle aromatische Geruchsnote, vgl. hierzu die Beispiele weiter unten.

Die vorliegende Erfindung betrifft somit auch die Verwendung der entsprechenden Verbindungen (2), (8), (9), (10), (18) und (42) als Riechstoff mit einer aromatischen Note.

Überraschenderweise besitzen einige der neuen, insbesondere der vorstehend als bevorzugt bezeichneten Verbindungen eine besonders wertvolle Leder-Geruchsnote, vgl. hierzu die Beispiele weiter unten.

Die vorliegende Erfindung betrifft somit auch die Verwendung der entsprechenden Verbindungen (13), (23), (29), (30), (43) und (44) als Riechstoff mit einer Leder-Note.

Überraschenderweise besitzen einige der neuen, insbesondere der vorstehend als bevorzugt bezeichneten Verbindungen eine besonders wertvolle Tabak-Geruchsnote, vgl. hierzu die Beispiele weiter unten.

Die vorliegende Erfindung betrifft somit auch die Verwendung der entsprechenden Verbindungen (9), (15), (23), (24), (29), (30), (40), (41), (43) und (44) als Riechstoff mit einer Tabak-Note.

Überraschenderweise besitzen einige der neuen, insbesondere der vorstehend als bevorzugt bezeichneten Verbindungen eine besonders wertvolle grüne Geruchsnote, vgl. hierzu die Beispiele weiter unten.

Die vorliegende Erfindung betrifft somit auch die Verwendung der entsprechenden Verbindungen (1), (12), (13), (31), (34), (43) und (45) als Riechstoff mit einer grünen Note.

Überraschenderweise besitzen einige der neuen, insbesondere der vorstehend als bevorzugt bezeichneten Verbindungen eine besonders wertvolle Anis-Geruchsnote, vgl. hierzu die Beispiele weiter unten.

Die vorliegende Erfindung betrifft somit auch die Verwendung der entsprechenden Verbindungen (9), (45), (51), (52), (57), (60) und (61) als Riechstoff mit einer Anis-Note.

Überraschenderweise besitzen einige der Verbindungen der Formel (I), insbesondere der neuen, vorstehend als bevorzugt bezeichneten Verbindungen der Formel (I) eine besonders wertvolle Geschmacksnote, insbesondere eine Geschmacksnote ausgewählt aus der Gruppe bestehend aus krautig, Krauseminze, kühlend, holzig, frisch und scharf, vgl. hierzu die Beispiele weiter unten.

Die vorliegende Erfindung betrifft somit auch die Verwendung der erfindungsgemäß zu verwendenden Verbindungen der Formel (I) als Geschmacksstoff, vorzugsweise der vorstehend als bevorzugt bezeichneten zu verwendenden Verbindungen der Formel (I) und/oder der erfindungsgemäßen neuen Verbindungen der Formel (I), vorzugsweise als Geschmacksstoff mit einer Geschmacksnote ausgewählt aus der Gruppe bestehend aus krautig, Krauseminze, kühlend, holzig, frisch und scharf.

Überraschenderweise besitzen einige der Verbindungen der Formel (I), insbesondere der neuen, vorstehend als bevorzugt bezeichneten Verbindungen der Formel (I) eine besonders wertvolle krautige Geschmacksnote, vgl. hierzu die Beispiele weiter unten.

Die vorliegende Erfindung betrifft somit auch die Verwendung der entsprechenden Verbindungen (2), (4) und (43) als Geschmacksstoff mit einer krautigen Note.

Überraschenderweise besitzen einige der Verbindungen der Formel (I), insbesondere der neuen, vorstehend als bevorzugt bezeichneten Verbindungen der Formel (I) eine besonders wertvolle Krauseminze-Geschmacksnote, vgl. hierzu die Beispiele weiter unten.

Die vorliegende Erfindung betrifft somit auch die Verwendung der entsprechenden Verbindungen (2), (29) und (10) als Geschmacksstoff mit einer Krauseminze-Note.

Überraschenderweise besitzen einige der Verbindungen der Formel (I), insbesondere der neuen, vorstehend als bevorzugt bezeichneten Verbindungen der Formel (I) eine besonders wertvolle kühlende Geschmacksnote, vgl. hierzu die Beispiele weiter unten.

Die vorliegende Erfindung betrifft somit auch die Verwendung der entsprechenden Verbindungen (2) und (23) als Geschmacksstoff mit einer kühlenden Note.

Überraschenderweise besitzen einige der Verbindungen der Formel (I), insbesondere der neuen, vorstehend als bevorzugt bezeichneten Verbindungen der Formel (I) eine besonders wertvolle holzige Geschmacksnote, vgl. hierzu die Beispiele weiter unten.

Die vorliegende Erfindung betrifft somit auch die Verwendung der entsprechenden Verbindungen (4), (13), (23) und (43) als Geschmacksstoff mit einer holzigen Note.

Überraschenderweise besitzen einige der Verbindungen der Formel (I), insbesondere der neuen, vorstehend als bevorzugt bezeichneten Verbindungen der Formel (I) eine besonders wertvolle frische Geschmacksnote, vgl. hierzu die Beispiele weiter unten.

Die vorliegende Erfindung betrifft somit auch die Verwendung der entsprechenden Verbindungen (13), (29), (43), (15), (10), (18) und (28) als Geschmacksstoff mit einer frischen Note.

Besonders bevorzugt ist die kombinierte, d.h. gleichzeitige Verwendung der erfindungsgemäß zu verwendenden Verbindungen zum überadditiven Verstärken eines Geruchseindrucks (vorzugsweise zum überadditiven Verstärken eines floralen und/oder fruchtigen Geruchseindrucks) und als Geschmacksstoff (vorzugsweise als Geschmacksstoff mit einer Note ausgewählt aus der Gruppe bestehend aus krautig, Krauseminze, kühlend, holzig, frisch und scharf). Zu bevorzugten erfindungsgemäß zu verwendenden Verbindungen gelten die obigen Ausführungen entsprechend.

Ganz besonders bevorzugt ist die kombinierte, d.h. gleichzeitige Verwendung der Verbindungen (1), (2), (3), (4), (5), (8), (9), (10), (12), (13), (14), (15), (18), (19), (23), (24), (28), (29), (30), (31), (32), (33), (34), (37), (40), (41), (42), (43), (44), (45), (48), (51), (52), (57), (60) und (61) zum überadditiven Verstärken eines Geruchseindrucks (vorzugsweise zum überadditiven Verstärken eines floralen und/oder fruchtigen Geruchseindrucks) und als Riechstoff mit einer, zwei, drei, vier, fünf oder sämtlichen Geruchsnoten ausgewählt aus der Gruppe bestehend aus würzig, Safran, Cumarin, aromatisch, Leder und Tabak und als Geschmacksstoff (vorzugsweise als Geschmacksstoff mit einer frischen und/oder scharfen Note)
Bevorzugt ist auch die kombinierte, d.h. gleichzeitige Verwendung der Verbindungen (1), (2), (3), (4), (5), (8), (9), (10), (12), (13), (14), (15), (18), (19), (23), (24), (28), (29), (30), (31), (32), (33), (34), (37), (40), (41), (42), (43), (44), (45), (48), (51), (52), (57), (60) und (61) als Riechstoff mit einer, zwei, drei, vier, fünf oder sämtlichen Geruchsnoten ausgewählt aus der Gruppe bestehend aus würzig, Safran, Cumarin, aromatisch, Leder und Tabak und als Geschmacksstoff (vorzugsweise als Geschmacksstoff mit einer frischen und/oder scharfen Note).

Besonders bevorzugt ist die Verwendung der erfindungsgemäß zu verwendenden Verbindungen der Formel (I), vorzugsweise der vorstehend als bevorzugt bezeichneten zu verwendenden Verbindungen der Formel (I) und/oder der erfindungsgemäßen neuen Verbindungen der Formel (I), als Geschmacksstoff, wobei es sich bei der Geschmacksnote nicht um einen fleischartigen, getreideartigen, schokoladeartigen, kakaoartigen, kaffeeartigen, nussartigen, schwefelartigen, erdigen und/oder fettigen Geschmack handelt.

Ein weiterer Aspekt der vorliegenden Erfindung betrifft erfindungsgemäße Riech- und/oder Geschmacksstoffkompositionen, umfassend oder bestehend aus
(a) einer Verbindung der Formel (I) oder einer Mischung aus zwei, drei, vier, fünf, sechs oder mehr verschiedenen Verbindungen der Formel (I), wie jeweils oben definiert, vorzugsweise wie jeweils weiter oben bei den erfindungsgemäßen Verbindungen bzw. Mischungen definiert,
   und
(b) einem, zwei, drei oder mehr weiteren Riech- und/oder Geschmacksstoffen, wobei der bzw. die weiteren Riech- und/oder Geschmacksstoffe keine Verbindungen der Formel (I) sind, wobei der bzw. ein, zwei, drei, mehr oder sämtliche der weiteren Riech- und/oder Geschmacksstoffe gemäß Bestandteil (b) einen floralen und/oder fruchtigen Geruch aufweisen.

Wie weiter oben bereits bei der erfindungsgemäßen Verwendung einer Verbindung der Formel (I) oder einer Mischung der Verbindungen der Formel (I) beschrieben, eignen sich die Verbindungen beziehungsweise die Mischungen insbesondere als Geruchsverstärker zum überadditiven Verstärken eines floralen und/oder fruchtigen Geruchseindrucks. Dementsprechend sind erfindungsgemäße Riech- und/oder Geschmacksstoffkompositionen besonders bevorzugt, bei denen der bzw. ein, zwei, drei, mehr oder sämtliche der weiteren Riech- und/oder Geschmacksstoffe gemäß Bestandteil (b) einen floralen und/oder einen fruchtigen Geruch aufweisen.

In resultierenden Riech- und/oder Geschmacksstoffkompositionen sind einige Geruchseindrücke häufig nicht vorteilhaft. Besonders bevorzugt ist entsprechend eine erfindungsgemäße Riech- und/oder Geschmacksstoffkomposition, wobei der zu verstärkende Geruchseindruck der weiteren Riech- und/oder Geschmacksstoffe gemäß Bestandteil (b) nicht ausgewählt ist aus der Gruppe bestehend aus nussig, haselnussig, pistazieartig, kakaoartig, karamellig, fleischig, röstgetreideartig, schokoladig und gewürzartig und/oder

wobei die Verbindung der Formel (I) oder die entsprechende Mischung keinen Geruchseindruck vermittelt, der ausgewählt ist aus der Gruppe bestehend aus nussig, haselnussig, pistazieartig, kakaoartig, karamellig, fleischig, röstgetreideartig, schokoladig und gewürzartig.

Dies gilt entsprechend für erfindungsgemäße Mischungen, die wie ausgeführt in manchen Ausgestaltungen neben Verbindungen der Formel (I) noch weitere Bestandteile umfassen.

In eigenen Untersuchungen hat es sich gezeigt, dass erfindungsgemäße Riech- und/oder Geschmacksstoffkompositionen insbesondere bevorzugt sind, bei denen der Bestandteil (b) keine Riech- und/oder Geschmacksstoffe enthält, die eine Pyrazingrundstruktur aufweisen.

Gemäß einem weiteren, bevorzugten Aspekt der vorliegenden Erfindung sind erfindungsgemäße Riech- und/oder Geschmacksstoffkompositionen besonders bevorzugt, bei denen Bestandteil (a) einen oder mehrere Geruchseindrücke des Bestandteils (b) verstärkt, wobei vorzugsweise ein floraler und/oder ein fruchtiger Geruchseindruck des Bestandteils (b) verstärkt wird. Eine solche Komposition wird auch als "geruchsverstärkte Komposition" bezeichnet; vgl. hierzu die Beispiele 37 bis 86.

Für eine erfindungsgemäße Riechstoff- oder Geschmacksstoffkomposition (wie oben beschrieben, vorzugsweise wie oben als bevorzugt bezeichnet) gilt gemäß einer bevorzugten Ausgestaltung, dass die Gesamtmenge an Verbindungen der Formel (I) im Bereich von 0,0001 bis 90 Gew.-%, vorzugsweise im Bereich von 0,01 bis 70 Gew.-%, bevorzugt im Bereich von 0,1 bis 50 Gew.-%, besonders bevorzugt im Bereich von 0,1 bis 30 Gew.-%, ganz besonders bevorzugt im Bereich von 0,1 bis 10 Gew.-% liegt, bezogen auf das Gesamtgewicht der Riech- und/oder Geschmacksstoffkomposition. Eine erfindungsgemäße Riechstoff- oder Geschmacksstoffkomposition, welche Verbindungen der Formel (I) in einer Gesamtmenge von mehr als 50 bis maximal 90 Gew.-% enthält, wird auch als Geruchsverstärkerkonzentrat bezeichnet und ist als solches bevorzugt. Eine erfindungsgemäße Riechstoff- oder Geschmacksstoffkomposition, welche Verbindungen der Formel (I) in einer Gesamtmenge von 0,0001 bis 50 Gew.-% enthält, ist üblicherweise eine Komposition, in der die Gesamtmenge an Verbindungen der Formel (I) ausreicht, den Geruchseindruck von einem oder mehreren Riechstoffen der Komponente (b) überadditiv zu verstärken, der bzw. die keine Verbindungen der Formel (I) sind, Eine solche Komposition ist eine bevorzugte "geruchsverstärkte Komposition"; vgl. hierzu erneut die Beispiele 37 bis 86.

Je nach gewünschter Anwendung kann es vorteilhaft und demnach erfindungsgemäß bevorzugt sein, die Gesamtmenge an Bestandteil (a) so zu wählen, dass die jeweils resultierende Riech- und/oder Geschmacksstoffkomposition keinen Eigengeruch und/oder Eigengeschmack der Komponente (a) aufweist, aber ein Geruchseindruck, vorzugsweise ein floraler und/oder fruchtiger Geruchseindruck, zumindest einer, zwei, drei, vier, fünf, sechs oder mehr der Komponenten des Bestandteils (b) verstärkt wird.

Erfindungsgemäße Riech- oder Geschmacksstoffkompositionen können in flüssiger Form, unverdünnt oder mit einem Lösungsmittel verdünnt zur Parfümierung oder Aromatisierung eingesetzt werden. Sofern die erfindungsgemäßen Riech- und/oder Geschmacksstoffkompositionen mit einem oder mehreren Lösungsmittel verdünnt vorliegen ist das eine oder sind die mehreren Lösungsmitteln ausgewählt aus der Gruppe bestehend aus: Ethanol, Isopropanol, Diethylenglycolmonoethylether, Glycerin, Propylenglycol, 1,2-Butylenglycol, Dipropylenglycol, Diethylphthalat, Triethylcitrat, Isopropylmyristat, Triacetin und Pflanzenöl.

Es ist zudem im Einzelfall bevorzugt, in den erfindungsgemäßen Riech- und/oder Geschmacksstoffkompositionen ein oder mehrere Lösungsmittel vorzusehen. Diese Lösungsmittel sind dann vorzugweise so ausgewählt, dass sie in der im Einzelfall vorliegenden Konzentration physiologisch unbedenklich sind. Aufgrund ihrer beträchtlichen Toxizität ist der Einsatz einzelner oder mehrerer der folgenden Lösungsmittel in einer erfindungsgemäßen Riech- und/oder Geschmacksstoffkomposition nicht bevorzugt: Hexan, Toluol, Benzol, Tetrahydrofuran, Pentan, Dimethylformamid, Diisopropylether, Dichlormethan, Dichlorethan, Chloroform, Tetrachlorkohlenstoff, N-Methylpyrrolidon, Pyridin, Dimethylacetamid, Dimethoxyethan, Dioxan, Diethylether und Methanol.

In Rahmen dieser Erfindung werden Verbindungen der Formel (I) (wie oben beschrieben) nicht als Lösungsmittel angesehen.

Es hat sich gezeigt, dass weitere positive Eigenschaften der erfindungsgemäßen Riech- und/oder Geschmacksstoffkompositionen vorliegen, sofern die erfindungsgemäße Riech- und/oder Geschmacksstoffkompositionen entsprechende weitere Additive umfasst. Geeignete Additive sind beispielsweise: Konservierungsmittel, Abrasiva, Antiakne-Mittel, Mittel gegen Hautalterung, antibakterielle Mittel, Anticellulitis-Mittel, Antischuppen-Mittel, entzündungshemmende Mittel, irritationsverhindernde Mittel, irritationshemmende Mittel, antimikrobielle Mittel, Antioxidantien, Adstringentien, schweißhemmende Mittel, antiseptische Mittel, Antistatika, Binder, Puffer, Trägermaterialien, Chelatbildner, Zellstimulantien, reinigende Mittel, pflegende Mittel, Enthaarungsmittel, oberflächenaktive Substanzen, deodorierende Mittel, Antiperspirantien, Weichmacher, Emulgatoren, Enzyme, ätherische Öle, Fasern, Filmbildner, Fixateure, Schaumbildner, Schaumstabilisatoren, Substanzen zum Verhindern des Schäumens, Schaumbooster, Fungizide, gelierende Mittel, gelbildende Mittel, Haarpflegemittel, Haarverformungsmittel, Haarglättungsmittel, feuchtigkeitsspendende Mittel, anfeuchtende Substanzen, feuchthaltende Substanzen, bleichende Mittel, stärkende Mittel, fleckenentfernende Mittel, optisch aufhellende Mittel, imprägnierende Mittel, schmutzabweisende Mittel, reibungsverringernde Mittel, Gleitmittel, Feuchtigkeitscremes, Salben, Trübungsmittel, plastifizierende Mittel, deckfähige Mittel, Politur, Glanzmittel, Polymere, Pulver, Proteine, rückfettende Mittel, abschleifende Mittel, Slilcone, hautberuhigende Mittel, hautreinigende Mittel, hautpflegende Mittel, hautheilende Mittel, Hautaufhellungsmittel, hautschützende Mittel, hauterweichende Mittel, kühlende Mittel, hautkühlende Mittel, wärmende Mittel, hautwärmende Mittel, Stabilisatoren, UVabsorbierende Mittel, UV-Filter, Waschmittel, Weichspüler, suspendierende Mittel, Hautbräunungsmittel, Verdickungsmittel, Vitamine, Öle, Wachse, Fette, Phospholipide, gesättigte Fettsäuren, ein- oder mehrfach ungesättigte Fettsäuren, alpha-Hydroxysäuren, Polyhydroxyfettsäuren, Verflüssiger, Farbstoffe, farbschützende Mittel, Pigmente, Antikorrosiva, Aromen, Geschmacksstoffe, Riechstoffe, Polyole, Tenside, Elektrolyte, organische Lösungsmittel oder Silikonderivate. Die Kombination aus erfindungsgemäßer Riech- und/oder Geschmacksstoffkompositionen und Additiv stellt einen beispielhaften erfindungsgemäßen Artikel dar.

Des Weiteren können erfindungsgemäße Riech- und/oder Geschmacksstoffkompositionen, an einen Trägerstoff adsorbiert sein, der sowohl für eine feine Verteilung der Riech- oder Geschmacksstoffe im Produkt als auch für eine kontrollierte Freisetzung bei der Anwendung sorgt. Derartige Träger können poröse anorganische Materialien wie Leichtsulfat, Kieselgele, Zeolithe, Gipse, Tone, Tongranulate, Gasbeton usw. oder organische Materialien wie Hölzer, Cellulose-basierende Stoffe, Zucker, Dextrine (z.B. Maltodextrin) oder Kunststoffe wie PVC, Polyvinylacetate oder Polyurethane sein. Die Kombination aus erfindungsgemäßer Komposition und Trägerstoff stellt einen beispielhaften erfindungsgemäßen Artikel dar.

Riech- und/oder Geschmacksstoffkompositionen, können insbesondere auch mikroverkapselt, sprühgetrocknet, als Einschluss-Komplexe oder als Extrusions-Produkte (d. h. erfindungsgemäße Artikel) vorliegen und in dieser Form z. B. einem zu parfümierenden oder zu aromatisierenden Artikel hinzugefügt werden.

Gegebenenfalls können die Eigenschaften der derart modifizierten Kompositionen durch sog. "Coaten" mit geeigneten Materialien im Hinblick auf eine gezieltere Duftfreisetzung weiter optimiert werden, wozu vorzugsweise wachsartige Kunststoffe wie z.B. Polyvinylalkohol verwendet werden. Die resultierenden Produkte stellen wiederum erfindungsgemäße Artikel dar.

Die Mikroverkapselung der erfindungsgemäßen Riech- und/oder Geschmacksstoffkompositionen kann beispielsweise durch das sogenannte Koazervationsverfahren mit Hilfe von Kapselmaterialien z.B. aus polyurethanartigen Stoffen oder Weichgelatine, erfolgen. Die sprühgetrockneten erfindungsgemäßen Riech- und/oder Geschmacksstoffkompositionen können beispielsweise durch Sprühtrocknung einer die erfindungsgemäßen Riech- und/oder Geschmacksstoffkomposition enthaltenden Emulsion, bzw. Dispersion hergestellt werden, wobei als Trägerstoffe modifizierte Stärken, Proteine, Dextrin und pflanzliche Gummen verwendet werden können. Einschluss-Komplexe können z.B. durch Eintragen von Dispersionen von der Riech- und/oder Geschmacksstoffkomposition und Cyclodextrinen oder Harnstoffderivaten in ein geeignetes Lösungsmittel, z.B. Wasser, hergestellt werden. Extrusions-Produkte können durch Verschmelzen der Riech- und/oder Geschmacksstoffkompositionen mit einem geeigneten wachsartigen Stoff und durch Extrusion mit nachfolgender Erstarrung, ggf. in einem geeigneten Lösungsmittel, z.B. Isopropanol, erhalten werden.

Die erfindungsgemäßen Riech- und/oder Geschmacksstoffkompositionen können in konzentrierter Form, in Lösungen oder in oben beschriebener modifizierter Form verwendet werden für die Herstellung von erfindungsgemäßen parfümierten und/oder aromatisierten Artikel wie z.B. Parfüm-Extraits, Eau de Parfums, Eau de Toilettes, Rasierwässer, Eau de Colognes, Pre-shave-Produkte, Splash-Colognes und parfümierten Erfrischungstüchern sowie die Parfümierung von sauren, alkalischen und neutralen Reinigungsmitteln, wie z.B. Fußbodenreinigern, Fensterglasreinigern, Geschirrspülmittel, Bad- und Sanitärreinigern, Scheuermilch, festen und flüssigen WC-Reinigern, pulver- und schaumförmigen Teppichreinigern, Textilerfrischern, Bügelhilfen, flüssigen Waschmitteln, pulverförmigen Waschmitteln, Wäschevorbehandlungsmitteln wie Bleichmittel, Einweichmittel und Fleckenentfernern, Wäscheweichspülern, Waschseifen, Waschtabletten, Desinfektionsmitteln, Oberflächendesinfektionsmitteln sowie von Luftverbesserern in flüssiger, gelartiger oder auf einem festen Träger aufgebrachter Form, Aerosolsprays, Wachsen und Polituren wie Möbelpolituren, Fußbodenwachsen, Schuhcremes sowie Körperpflegemitteln wie z.B. festen und flüssigen Seifen, Duschgelen, Shampoos, Rasierseifen, Rasierschäumen, Badeölen, kosmetischen Emulsionen vom ÖI-in-Wasser-, vom Wasserin-ÖI- und vom Wasser-in-ÖI-in-Wasser-Typ wie z.B. Hautcremes- und -lotionen, Gesichtscremes und -lotionen, Sonnenschutzcremes- und -lotionen, After-sun-Cremes und - lotionen, Handcremes und -lotionen, Fußcremes und -lotionen, Enthaarungscremes und - lotionen, After-shave-Cremes und -lotionen, Bräunungscremes und -lotionen, Haarpflegeprodukten wie z.B. Haarsprays, Haargelen, festigenden Haarlotionen, Haarspülungen, permanenten und semipermanenten Haarfärbemitteln, Haarverformungsmitteln wie Kaltwellen und Haarglättungsmitteln, Haarwässern, Haarcremes und -lotionen, Deodorantien und Antiperspirantien wie z.B. Achselsprays, Roll-ons, Deosticks, Deocremes, Produkten der dekorativen Kosmetik wie z.B. Lidschatten, Nagellacke, Make-ups, Lippenstifte, Mascara sowie von Kerzen, Lampenölen, Räucherstäbchen, Insektiziden, Repellentien und Treibstoffen.

Die erfindungsgemäße Riech- und/oder Geschmacksstoffkomposition kann in aromatisierten oder zu aromatisierende Produkte eingearbeitet werden, insbesondere der Ernährung, der Mundpflege oder dem Genuss dienende Zubereitungen. Solche Produkte umfassen regelmäßig Bestandteile, die andere als auf die Aroma- oder Duftentwicklung bezogene Funktionen haben (z. B. Farbstoffe) und/oder sind ein bereits fertiges handelbares Produkt (z. B. Parfümöl).

Der Ernährung oder dem Genuss dienende Zubereitungen sind z.B. Backwaren (z.B. Brot, Trockenkekse, Kuchen, sonstiges Gebäck), Süßwaren (z.B. Schokoladen, Schokoladenriegelprodukte, sonstige Riegelprodukte, Fruchtgummi, Hart- und Weichkaramellen, Kaugummi), alkoholische oder nicht-alkoholische Getränke (z.B. Kaffee, Tee, Wein, weinhaltige Getränke, Bier, bierhaltige Getränke, Liköre, Schnäpse, Weinbrände, fruchthaltige Limonaden, isotonische Getränke, Erfrischungsgetränke, Nektare, Obst- und Gemüsesäfte, Frucht- oder Gemüsesaftzubereitungen), Instantgetränke (z.B. Instant-Kakao-Getränke, Instant-Tee-Getränke, Instant-Kaffeegetränke), Fleischprodukte (z.B. Schinken, Frischwurst- oder Rohwurstzubereitungen, gewürzte oder marinierte Frisch- oder Pökelfleischprodukte), Eier oder Eiprodukte (Trockenei, Eiweiß, Eigelb), Getreideprodukte (z.B. Frühstückscerealien, Müsliriegel, vorgegarte Fertigreis-Produkte), Milchprodukte (z.B. Milchgetränke, Milcheis, Joghurt, Kefir, Frischkäse, Weichkäse, Hartkäse, Trockenmilchpulver, Molke, Butter, Buttermilch, teilweise oder ganz hydrolisierte Milchproteinhaltige Produkte), Produkte aus Sojaprotein oder anderen Sojabohnen-Fraktionen (z.B. Sojamilch und daraus gefertigte Produkte, Sojalecithin-haltige Zubereitungen, fermentierte Produkte wie Tofu oder Tempe oder daraus gefertigte Produkte, Sojasoßen), Fruchtzubereitungen (z.B. Konfitüren, Fruchteis, Fruchtsoßen, Fruchtfüllungen), Gemüsezubereitungen (z.B. Ketchup, Soßen, Trockengemüse, Tiefkühlgemüse, vorgegarte Gemüse, in Essig eingelegte Gemüse, eingekochte Gemüse), Knabberartikel (z.B. gebackene oder frittierte Kartoffelchips oder Kartoffelteigprodukte, Brotteigprodukte, Extrudate auf Mais- oder Erdnussbasis), Produkte auf Fett- und Ölbasis oder Emulsionen derselben (z.B. Mayonnaise, Remoulade, Dressings, Würzzubereitungen), sonstige Fertiggerichte und Suppen (z.B. Trockensuppen, Instant-Suppen, vorgegarte Suppen), Gewürze, Würzmischungen sowie insbesondere Aufstreuwürzungen (englisch: Seasonings), die beispielsweise im Snackbereich Anwendung finden. Nach Einarbeiten der a) erfindungsgemäßen Verbindung der Formel (I) bzw. deren Mischung und/oder b) der erfindungsgemäßen Riech- und/oder Geschmacksstoffkomposition sind diese Zubereitungen erfindungsgemäße Artikel.

Zubereitungen können z. B. als Halbfertigware oder als Würzmischung vorliegen.

Zubereitungen können insbesondere als Halbfertigware zur Herstellung weiterer der Ernährung oder dem Genuss dienenden Zubereitungen dienen, insbesondere in sprühgetrockneter Form. Zubereitungen können auch in Form von Kapseln, Tabletten (nichtüberzogene sowie überzogene Tabletten, z.B. magensaftresistente Überzüge), Dragees, Granulaten, Pellets, Feststoffmischungen, Dispersionen in flüssigen Phasen, als Emulsionen, als Pulver, als Lösungen, als Pasten oder als andere schluck- oder kaubare Zubereitungen als Nahrungsergänzungsmittel vorliegen.

Der Mundpflege dienende Zubereitungen sind insbesondere Mund- und/oder Zahnpflegemittel wie Zahnpasten, Zahngele, Zahnpulver, Mundwässer, Kaugummis und andere Mundpflegemittel.

Weitere übliche Wirk-, Grund-, Hilfs- und Zusatzstoffe für der Ernährung, der Mundpflege oder dem Genuss dienende erfindungsgemäße Zubereitungen können in Mengen von 5 bis 99,999999 Gew.-%, vorzugsweise 10 bis 80 Gew.-%, bezogen auf das Gesamtgewicht der Zubereitung, enthalten sein. Ferner können die Zubereitungen Wasser in einer Menge bis zu 99,999999 Gew.-%, vorzugsweise 5 bis 80 Gew.-%, bezogen auf das Gesamtgewicht der Zubereitung, aufweisen.

Die Zubereitungen (als Beispiele erfindungsgemäßer Artikel), enthaltend a) die erfindungsgemäßen Verbindung der Formel (I) bzw. deren Mischung und/oder b) die erfindungsgemäßen Riech- und/oder Geschmacksstoffkomposition, werden gemäß einer bevorzugten Ausgestaltung hergestellt, indem die erfindungsgemäße Verbindung bzw. deren Mischungen und/oder erfindungsgemäßen Riech- und/oder Geschmacksstoffkomposition als Substanz, als Lösung (z.B. in Ethanol, Wasser oder 1,2-Propylenglycol) oder in Form eines Gemisches mit einem festen oder flüssigen Trägerstoff (z.B. Maltodextrin, Stärke, Silicagel), sonstigen Aromen oder Geschmacksstoffen und gegebenenfalls weiteren Hilfsmitteln und/oder Stabilisatoren (z.B. natürliche oder künstliche Polysaccharide und/oder Pflanzengummen wie modifizierte Stärken oder Gummi Arabicum) in eine der Ernährung, der Mundpflege oder dem Genuss dienende Basis-Zubereitung eingearbeitet werden. Vorteilhafterweise können als Lösung und/oder Suspension oder Emulsion vorliegende Zubereitungen auch durch Sprühtrocknung in eine feste Zubereitung (Halbfertigware) überführt werden.

Die sprühgetrockneten festen Zubereitungen (als Beispiel erfindungsgemäßer Artikel) sind als Halbfertigwaren besonders gut zur Herstellung von weiteren Zubereitungen geeignet. In den sprühgetrockneten festen Zubereitungen sind vorzugsweise 50 bis 95 % Gew.-% Trägerstoffe, insbesondere Maltodextrin und/oder Stärke, 5 bis 40 % Hilfsstoffe, bevorzugt natürliche oder künstliche Polysaccharide und/oder Pflanzengummen wie modifizierte Stärken oder Gummi Arabicum enthalten.

Gemäß einer weiteren bevorzugten Ausführungsform werden zur Herstellung von Zubereitungen a) die erfindungsgemäße Verbindung der Formel (I) bzw. deren Mischung und/oder b) erfindungsgemäße Riech- und/oder Geschmacksstoffkomposition und gegebenenfalls andere Bestandteile der erfindungsgemäßen Zubereitung zunächst in Emulsionen, in Liposomen, z.B. ausgehend von Phosphatidylcholin, in Microsphären, in Nanosphären oder auch in Kapseln, Granulaten oder Extrudaten aus einer für Lebens- und Genussmittel geeigneten Matrix, z.B. aus Stärke, Stärkederivaten (z.B. modifizierte Stärke), Cellulose oder Cellulosederivaten (z.B. Hydroxypropylcellulose), anderen Polysacchariden (z.B. Dextrin, Alginat, Curdlan, Carageenan, Chitin, Chitosan, Pullulan), natürlichen Fetten, natürlichen Wachsen (z.B. Bienenwachs, Carnaubawachs), aus Proteinen, z.B. Gelatine oder sonstigen Naturprodukten (z.B. Schellack) eingearbeitet. Dabei können je nach Matrix die Produkte durch Sprühtrocknung, Sprühgranulation, Schmelzgranulation, Koazervation, Koagulation, Extrusion, Schmelzextrusion, Emulsionsverfahren, Beschichtung (Coating) oder andere geeignete Verkapselungsverfahren und gegebenenfalls eine geeignete Kombination der vorgenannten Verfahren erhalten werden. In einem weiteren bevorzugten Herstellungsverfahren für eine erfindungsgemäße Zubereitung wird die erfindungsgemäße Verbindung zunächst mit einem oder mehreren geeigneten Komplexbildnern, beispielsweise mit Cyclodextrinen oder Cyclodextrinderivaten, bevorzugt alpha- oder beta-Cyclodextrin, komplexiert und in dieser komplexierten Form eingesetzt.

Besonders bevorzugt ist eine Zubereitung, bei der die Matrix so gewählt ist, dass die erfindungsgemäße Verbindung verzögert von der Matrix freigegeben wird, so dass eine langanhaltende Wirkung erzielt wird. Besonders bevorzugt ist insoweit eine Fett-, Wachs-, Polysaccharid- oder Proteinmatrix.

Als weitere Bestandteile für der Ernährung oder dem Genuss dienende Zubereitungen können übliche Grund-, Hilfs- und Zusatzstoffe für Nahrungs- oder Genussmittel verwendet werden, z.B. Wasser, Gemische frischer oder prozessierter, pflanzlicher oder tierischer Grund- oder Rohstoffe (z.B. rohes, gebratenes, getrocknetes, fermentiertes, geräuchertes und/oder gekochtes Fleisch, Knochen, Knorpel, Fisch, Gemüse, Früchte, Kräuter, Nüsse, Gemüse- oder Fruchtsäfte oder -pasten oder deren Gemische), verdauliche oder nicht verdauliche Kohlenhydrate (z.B. Saccharose, Maltose, Fructose, Glucose, Dextrine, Amylose, Amylopektin, Inulin, Xylane, Cellulose, Tagatose), Zuckeralkohole (z.B. Sorbit, Erythritol), natürliche oder gehärtete Fette (z.B. Talg, Schmalz, Palmfett, Kokosfett, gehärtetes Pflanzenfett), Öle (z.B. Sonnenblumenöl, Erdnussöl, Maiskeimöl, Olivenöl, Fischöl, Sojaöl, Sesamöl), Fettsäuren oder deren Salze (z.B. Kaliumstearat), proteinogene oder nicht-proteinogene Aminosäuren und verwandte Verbindungen (z.B. gamma-Aminobuttersäure, Taurin), Peptide (z.B. Glutathion), native oder prozessierte Proteine (z.B. Gelatine), Enzyme (z.B. Peptidasen), Nukleinsäuren, Nucleotide, Geschmackskorrigenzien für unangenehme Geschmackseindrücke, weitere Geschmacksmodulatoren für weitere, in der Regel nicht unangenehme Geschmackseindrücke, andere geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Emulgatoren (z.B. Lecithine, Diacylglycerole, Gummi Arabicum), Stabilisatoren (z.B. Carageenan, Alginat), Konservierungsstoffe (z.B. Benzoesäure, Sorbinsäure), Antioxidantien (z.B. Tocopherol, Ascorbinsäure), Chelatoren (z.B. Citronensäure), organische oder anorganische Säuerungsmittel (z.B. Äpfelsäure, Essigsäure, Citronensäure, Weinsäure, Phosphorsäure), Bitterstoffe (z.B. Chinin, Coffein, Limonin, Amarogentin, Humolone, Lupolone, Catechine, Tannine), mineralische Salze (z.B. Natriumchlorid, Kaliumchlorid, Magnesiumchlorid, Natriumphosphate), die enzymatische Bräunung verhindernde Stoffe (z.B. Sulfit, Ascorbinsäure), etherische Öle, Pflanzenextrakte, natürliche oder synthetische Farbstoffe oder Farbpigmente (z.B. Carotinoide, Flavonoide, Anthocyane, Chlorophyll und deren Derivate), Gewürze, trigeminal wirksame Stoffe oder Pflanzenextrakte, enthaltend solche trigeminal wirksamen Stoffe, synthetische, natürliche oder naturidentische Geschmacksstoffe oder Riechstoffe sowie Geruchskorrigenzien.

Zahnpflegemittel (als Basis für der Mundpflege dienende Zubereitungen), die a) die erfindungsgemäße Verbindung der Formel (I) bzw. deren Mischung und/oder b) erfindungsgemäße Riech- und/oder Geschmacksstoffkomposition enthalten, umfassen im Allgemeinen ein abrasives System (Schleif- oder Poliermittel), wie z.B. Kieselsäuren, Calciumcarbonate, Calciumphosphate, Alumiuniumoxide und/oder Hydroxylapatite, oberflächenaktive Substanzen wie z.B. Natriumlaurylsulfat, Natriumlaurylsarcosinat und/oder Cocamidopropylbetain, Feuchthaltemitteln wie z.B. Glycerin und/oder Sorbit, Verdickungsmittel, wie z.B. Carboxymethylcellulose, Polyethylenglycole, Carrageenan und/oder Laponite^{®}, Süßstoffe, wie z.B. Saccharin, Geschmackskorrigenzien für unangenehme Geschmackseindrücke, Geschmackskorrigenzien für weitere, in der Regel nicht unangenehme Geschmackseindrücke, geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Kühlwirkstoffen wie z.B. Menthol, Mentholderivate (z.B. L-Menthol, L-Menthyllactat, L-Menthylalkylcarbonate, Menthonketale, Menthancarbonsäureamide), 2,2,2-Trialkylessigsäureamiden (z.B. 2,2-Diisopropylpropionsäuremethylamid), Icilin und Icilin-Derivate, Stabilisatoren und aktive Wirkstoffe, wie z.B. Natriumfluorid, Natriummonofluorphosphat, Zinndifluorid, quartären Ammoniumfluoriden, Zinkcitrat, Zinksulfat, Zinnpyrophosphat, Zinndichlorid, Mischungen verschiedener Pyrophosphate, Triclosan, Cetylpyridiniumchlorid, Aluminiumlactat, Kaliumcitrat, Kaliumnitrat, Kaliumchlorid, Strontiumchlorid, Wasserstoffperoxid, Aromen und/oder Natriumbicarbonat oder Geruchskorrigenzien.

Kaugummis (als weiteres Beispiel für der Mundpflege dienende Zubereitungen), welche a) die erfindungsgemäße Verbindung der Formel (I) bzw. deren Mischung und/oder b) erfindungsgemäße Riech- und/oder Geschmacksstoffkomposition enthalten, umfassen im allgemeinen eine Kaugummibase, d.h. eine beim Kauen plastisch werdende Kaumasse, Zucker verschiedener Arten, Zuckeraustauschstoffe, andere süß schmeckende Stoffe, Zuckeralkoholen, Geschmackskorrigenzien für unangenehme Geschmackseindrücke, andere Geschmacksmodulatoren für weitere, in der Regel nicht unangenehme Geschmackseindrücke, geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Feuchthaltemittel, Verdicker, Emulgatoren, Aromen und Stabilisatoren oder Geruchskorrigenzien.

Bevorzugt können die Zubereitungen auch neben der erfindungsgemäß einzusetzenden Verbindung eine (zusätzliche) Aromakomposition enthalten, um den Geschmack und/oder Geruch der Zubereitung abzurunden und zu verfeinern. Geeignete (zusätzliche) Aromakompositionen enthalten z.B. synthetische, natürliche oder naturidentische Aroma-, Riech- und Geschmacksstoffe sowie geeignete Hilfs- und Trägerstoffe.

Nach Einarbeiten der a) erfindungsgemäßen Verbindung der Formel (I) bzw. deren Mischung und/oder b) der erfindungsgemäßen Riech- und/oder Geschmacksstoffkomposition sind diese Zubereitungen erfindungsgemäße Artikel wie nachfolgend definiert.

Die erfindungsgemäß zu verwendenden Verbindungen der Formel (I) bzw. entsprechende Mischungen davon oder eine erfindungsgemäße Riechstoff- und/oder Geschmacksstoffkomposition (wie oben beschrieben) können vorteilhafterweise auch zur Parfümierung und/oder Aromatisierung bestimmter Artikel eingesetzt werden bzw. Bestandteil solcher Artikel sein. Insbesondere eine erfindungsgemäße Riechstoff- und/oder Geschmacksstoffkomposition, vorzugsweise eine Riechstoff- und/oder Geschmacksstoffkomposition wie hierin als bevorzugt bezeichnet, ist im Rahmen der vorliegenden Erfindung als Bestandteil eines parfümierten und/oder aromatisierten Artikels einsetzbar.

Dementsprechend betrifft die vorliegende Erfindung auch einen parfümierten und/oder aromatisierten Artikel, der eine erfindungsgemäße Riechstoff- und/oder Geschmacksstoffkomposition (wie hierin beschrieben) umfasst.

In einer bevorzugten Ausgestaltungsform ist der erfindungsgemäße parfümierte und/oder aromatisierte Artikel ausgewählt aus der Gruppe bestehend aus: Parfüm-Extraits, Eau de Parfums, Eau de Toilettes, Rasierwässern, Eau de Colognes, Pre-shave-Produkten, Splash-Colognes, parfümierten Erfrischungstüchern, sauren, alkalischen und neutralen Reinigungsmitteln, Textilerfrischern, Bügelhilfen, flüssigen Waschmitteln, pulverförmigen Waschmitteln, Wäschevorbehandlungsmitteln, Wäscheweichspülern, Waschseifen, Waschtabletten, Desinfektionsmitteln, Oberflächendesinfektionsmitteln, Luftverbesserern, Aerosolsprays, Wachsen und Polituren, Körperpflegemitteln, Handcremes und -lotionen, Fußcremes und -lotionen, Enthaarungscremes und -lotionen, After-shave-Cremes und - lotionen, Bräunungscremes und -lotionen, Haarpflegeprodukten, Deodorantien und Antiperspirantien, Produkten der dekorativen Kosmetik, Kerzen, Lampenölen, Räucherstäbchen, Insektiziden, Repellentien und Treibstoffen.

Erfindungsgemäße Artikel umfassen in vielen Fällen einen Träger oder ein Substrat, der bzw. das in direktem Kontakt mit der oder den Verbindungen der Formel (I) bzw. der Riech- und/oder Geschmacksstoffkomposition steht. Derartige Träger können poröse anorganische Materialien wie Leichtsulfat, Kieselgele, Zeolithe, Gipse, Tone, Tongranulate, Gasbeton usw. oder organische Materialien wie Hölzer, Cellulose-basierende Stoffe, Zucker, Dextrine (z.B. Maltodextrin) oder Kunststoffe wie PVC, Polyvinylacetate oder Polyurethane sein.

Ein weiterer Aspekt der vorliegenden Erfindung betrifft ein Verfahren zum Verstärken eines Geruchseindruckes, vorzugsweise eines floralen und/oder fruchtigen Geruchseindruckes, umfassend folgenden Schritt:
Inkontaktbringen oder Mischen
   - einer Verbindung der Formel (I)
      wie oben definiert, insbesondere wie oben bei den neuen Verbindungen der Formel (I) definiert,
   - einer Mischung aus zwei, drei, vier, fünf, sechs oder mehr verschiedenen Verbindungen der Formel (I),
      wie oben definiert, insbesondere wie oben bei den neuen Verbindungen der Formel (I) definiert,
      oder
   - einer Riech- und/oder Geschmacksstoffkomposition wie oben definiert, mit einem Erzeugnis.

Bevorzugte Erzeugnisse sind die oben beschriebenen erfindungsgemäß bevorzugten Artikel bzw. Zubereitungen oder eine, zwei, drei oder mehr weiterer Riech- und/oder Geschmacksstoffe, wobei der bzw. die weiteren Riech- und/oder Geschmacksstoffe keine Verbindungen der Formel (I) sind und vorzugsweise einen floralen und/oder einen fruchtigen Geruch aufweisen.

Bevorzugte Substanzen, die im Rahmen der vorliegenden Erfindung in einem erfindungsgemäßen Artikel, einer erfindungsgemäßen Riechstoff- und/oder Geschmacksstoffkomposition, vorzugsweise als Bestandteil (b) einer erfindungsgemäßen Riechstoff- und/oder Geschmacksstoffkomposition, enthalten sind bzw. enthalten sein können, finden sich z. B. in S. Arctander, Perfume and Flavor Materials, Vol. I und II, Montclair, N. J. 1969, Eigenverlag, oder K. Bauer et al., Common Fragrance and Flavor Materials, 4th Edition, Wiley-VCH, Weinheim 2001.

Im Einzelnen seien genannt:
Extrakte aus natürlichen Rohstoffen wie Etherische Öle, Concretes, Absolues, Resine, Resinoide, Balsame, Tinkturen wie z. B.

Ambratinktur; Amyrisöl; Angelicasamenöl; Angelicawurzelöl; Anisöl; Baldrianöl; Basilikumöl; Baummoos-Absolue; Bayöl; Beifußöl; Benzoeresin; Bergamotteöl; Bienenwachs-Absolue; Birkenteeröl; Bittermandelöl; Bohnenkrautöl; Buccoblätteröl; Cabreuvaöl; Cadeöl; Calmusöl; Campheröl; Canangaöl; Cardamomenöl; Cascarillaöl; Cassiaöl; Cassie-Absolue; Castoreum-Absolue; Cedernblätteröl; Cedernholzöl; Cistusöl; Citronellöl; Citronenöl; Copaivabalsam; Copaivabalsamöl; Corianderöl; Costuswurzelöl; Cuminöl; Cypressenöl; Davanaöl; Dillkrautöl; Dillsamenöl; Eau de brouts-Absolue; Eichenmoos-Absolue; Elemiöl; Estragonöl; Eucalyptus-citriodora-Öl; Eucalyptusöl; Fenchelöl; Fichtennadelöl; Galbanumöl; Galbanumresin; Geraniumöl; Grapefruitöl; Guajakholzöl; Gurjunbalsam; Gurjunbalsamöl; Helichrysum-Absolue; Helichrysumöl; Ingweröl; Iriswurzel-Absolue; Iriswurzelöl; Jasmin-Absolue; Kalmusöl; Kamillenöl blau; Kamillenöl römisch; Karottensamenöl; Kaskarillaöl; Kiefernadelöl; Krauseminzöl; Kümmelöl; Labdanumöl; Labdanum-Absolue; Labdanumresin; Lavandin-Absolue; Lavandinöl; Lavendel-Absolue; Lavendelöl; Lemongrasöl; Liebstocköl; Limetteöl destilliert; Limetteöl gepresst; Linaloeöl; Litseacubeba-Öl; Lorbeerblätteröl; Macisöl; Majoranöl; Mandarinenöl; Massoirindenöl; Mimosa-Absolue; Moschuskörneröl; Moschustinktur; Muskateller-Salbei-Öl; Muskatnussöl; Myrrhen-Absolue; Myrrhenöl; Myrtenöl; Nelkenblätteröl; Nelkenblütenöl; Neroliöl; Olibanum-Absolue; Olibanumöl; Opopanaxöl; Orangenblüten-Absolue; Orangenöl; Origanumöl; Palmarosaöl; Patchouliöl; Perillaöl; Perubalsamöl; Petersilienblätteröl; Petersiliensamenöl; Petitgrainöl; Pfefferminzöl; Pfefferöl; Pimentöl; Pineöl; Poleyöl; Rosen-Absolue; Rosenholzöl; Rosenöl; Rosmarinöl; Salbeiöl dalmatinisch; Salbeiöl spanisch; Sandelholzöl; Selleriesamenöl; Spiklavendelöl; Sternanisöl; Styraxöl; Tagetesöl; Tannennadelöl; Tea-tree-Öl; Terpentinöl; Thymianöl; Tolubalsam; Tonka-Absolue; Tuberosen-Absolue; Vanilleextrakt; Veilchenblätter-Absolue; Verbenaöl; Vetiveröl; Wacholderbeeröl; Weinhefenöl; Wermutöl; Wintergrünöl; Ylangöl; Ysopöl; Zibet-Absolue; Zimtblätteröl; Zimtrindenöl sowie Fraktionen davon, bzw. daraus isolierten Inhaltsstoffen;

Einzel-Riechstoffe aus der Gruppe
der Kohlenwasserstoffe, wie z.B. 3-Caren; alpha-Pinen; beta-Pinen; alpha-Terpinen; gamma-Terpinen; p-Cymol; Bisabolen; Camphen; Caryophyllen; Cedren; Farnesen; Limonen; Longifolen; Myrcen; Ocimen; Valencen; (E,Z)-1,3,5-Undecatrien; Styrol; Diphenylmethan;
der aliphatischen Alkohole wie z.B. Hexanol; Octanol; 3-Octanol; 2,6-Dimethylheptanol; 2-Methyl-2-heptanol; 2-Methyl-2-octanol; (E)-2-Hexenol; (E)- und (Z)-3-Hexenol; 1-Octen-3-ol; Gemisch von 3,4,5,6,6-Pentamethyl-3/4-hepten-2-ol und 3,5,6,6-Tetramethyl-4-methyleneheptan-2-ol; (E,Z)-2,6-Nonadienol; 3,7-Dimethyl-7-methoxyoctan-2-ol; 9-Decenol; 10-Undecenol; 4-Methyl-3-decen-5-ol;
der aliphatischen Aldehyde und deren Acetale wie z.B. Hexanal; Heptanal; Octanal; Nonanal; Decanal; Undecanal; Dodecanal; Tridecanal; 2-Methyloctanal; 2-Methylnonanal; (E)-2-Hexenal; (Z)-4-Heptenal; 2,6-Dimethyl-5-heptenal; 10-Undecenal; (E)-4-Decenal; 2-Dodecenal;2,6,10-Trimethyl-9-undecenal; 2,6,10-Trimethyl-5,9-undecadienal; Heptanaldiethylacetal; 1,1-Dimethoxy-2,2,5-trimethyl-4-hexen; Citronellyloxyacetaldehyd; 1-(1-Methoxy-propoxy)-(E/Z)-3-hexen;
der aliphatischen Ketone und deren Oxime wie z.B. 2-Heptanon; 2-Octanon; 3-Octanon; 2-Nonanon; 5-Methyl-3-heptanon; 5-Methyl-3-heptanonoxim; 2,4,4,7-Tetramethyl-6-octen-3-on; 6-Methyl-5-hepten-2-on;
der aliphatischen schwefelhaltigen Verbindungen wie z.B. 3-Methylthio-hexanol; 3-Methylthiohexylacetat; 3-Mercaptohexanol; 3-Mercaptohexylacetat; 3-Mercaptohexylbutyrat; 3-Acetylthiohexylacetat; 1-Menthen-8-thiol;
der aliphatischen Nitrile wie z.B. 2-Nonensäurenitril; 2-Undecensäurenitril; 2-Tridecensäurenitril; 3,12-Tridecadiensäurenitril; 3,7-Dimethyl-2,6-octadien-säurenitril; 3,7-Dimethyl-6-octensäurenitril;
der Ester von aliphatischen Carbonsäuren wie z.B. (E)- und (Z)-3-Hexenylformiat; Ethylacetoacetat; Isoamylacetat; Hexylacetat; 3,5,5-Trimethylhexylacetat; 3-Methyl-2-butenylacetat; (E)-2-Hexenylacetat; (E)- und (Z)-3-Hexenylacetat; Octylacetat; 3-Octylacetat; 1-Octen-3-ylacetat; Ethylbutyrat; Butylbutyrat; Isoamylbutyrat; Hexylbutyrat; (E)- und (Z)-3-Hexenyl-isobutyrat; Hexylcrotonat; Ethylisovalerianat; Ethyl-2-methylpentanoat; Ethylhexanoat; Allylhexanoat; Ethylheptanoat; Allylheptanoat; Ethyloctanoat; Ethyl-(E,Z)-2,4-decadienoat; Methyl-2-octinat; Methyl-2-noninat; Allyl-2-isoamyloxyacetat; Methyl-3,7-dimethyl-2,6-octadienoat;4-Methyl-2-pentyl-crotonat;
der acyclischen Terpenalkohole wie z. B. Citronellol; Geraniol; Nerol; Linalool; Lavadulol; Nerolidol; Farnesol; Tetrahydrolinalool; Tetrahydrogeraniol; 2,6-Dimethyl-7-octen-2-ol; 2,6-Dimethyloctan-2-ol; 2-Methyl-6-methylen-7-octen-2-ol; 2,6-Dimethyl-5,7-octadien-2-ol; 2,6-Dimethyl-3,5-octadien-2-ol; 3,7-Dimethyl-4,6-octadien-3-ol; 3,7-Dimethyl-1,5,7-octatrien-3-ol 2,6-Dimethyl-2,5,7-octatrien-1-ol; sowie deren Formiate, Acetate, Propionate, Isobutyrate, Butyrate, Isovalerianate, Pentanoate, Hexanoate, Crotonate, Tiglinate und 3-Methyl-2-butenoate;
der acyclischen Terpenaldehyde und -ketone wie z. B. Geranial; Neral; Citronellal; 7-Hydroxy-3,7-dimethyloctanal; 7-Methoxy-3,7-dimethyloctanal; 2,6,10-Trimethyl-9-undecenal; Geranylaceton; sowie die Dimethyl- und Diethylacetale von Geranial, Neral, 7-Hydroxy-3,7-dimethyloctanal;
der cyclischen Terpenalkohole wie z. B. Menthol; Isopulegol; alpha-Terpineol; Terpinenol-4; Menthan-8-ol; Menthan-1-ol; Menthan-7-ol; Borneol; Isoborneol; Linalooloxid; Nopol; Cedrol; Ambrinol; Vetiverol; Guajol; sowie deren Formiate, Acetate, Propionate, Isobutyrate, Butyrate, Isovalerianate, Pentanoate, Hexanoate, Crotonate, Tiglinate und 3-Methyl-2-butenoate;
der cyclischen Terpenaldehyde und -ketone wie z. B. Menthon; Isomenthon; 8-Mercaptomenthan-3-on; Carvon; Campher; Fenchon; alpha-Ionon; beta-Ionon; alpha-n-Methylionon; beta-n-Methylionon; alpha-Isomethylionon; beta-Isomethylionon; alpha-Iron; alpha-Damascon; beta-Damascon; beta-Damascenon; delta-Damascon; gamma-Damascon; 1-(2,4,4-Trimethyl-2-cyclohexen-1-yl)-2-buten-1-on; 1,3,4,6,7,8a-Hexahydro-1,1,5,5-tetramethyl-2H-2,4a-methanonaphthalen-8(5H)-on;2-Methyl-4-(2,6,6-trimethyl-1-cyclohexen-1-yl)-2-butenal; Nootkaton; Dihydronootkaton; 4,6,8-Megastigmatrien-3-on; alpha-Sinensal; beta-Sinensal; acetyliertes Cedernholzöl (Methylcedrylketon);
der cyclischen Alkohole wie z.B. 4-tert.-Butylcyclohexanol; 3,3,5-Trimethylcyclohexanol; 3-Isocamphylcyclohexanol; 2,6,9-Trimethyl-Z2,Z5,E9-cyclododecatrien-1-ol; 2-Isobutyl-4-methyltetrahydro-2H-pyran-4-ol;
der cycloaliphatischen Alkohole wie z.B. alpha,3,3-Trimethylcyclohexylmethanol;1-(4-Isopropylcyclohexyl)ethanol; 2-Methyl-4-(2,2,3-trimethyl-3-cyclopent-1-yl)butanol; 2-Methyl-4-(2,2,3-trimethyl-3-cyclopent-1-yl)-2-buten-1-ol; 2-Ethyl-4-(2,2,3-trimethyl-3-cyclopent-1-yl)-2-buten-1-ol; 3-Methyl-5-(2,2,3-trimethyl-3-cyclopent-1-yl)-pentan-2-ol; 3-Methyl-5-(2,2,3-trimethyl-3-cyclopent-1-yl)-4-penten-2-ol; 3,3-Dimethyl-5-(2,2,3-trimethyl-3-cyclopent-1-yl)-4-penten-2-ol; 1-(2,2,6-Trimethylcyclohexyl)pentan-3-ol; 1-(2,2,6-Trimethylcyclohexyl)hexan-3-ol;
der cyclischen und cycloaliphatischen Ether wie z.B. Cineol; Cedrylmethylether; Cyclododecylmethylether;1,1-Dimethoxycyclododecan; (Ethoxymethoxy)cyclododecan; alpha-Cedrenepoxid; 3a,6,6,9a-Tetramethyldodecahydronaphtho[2,1-b]furan; 3a-Ethyl-6,6,9a-trimethyldodecahydronaphtho[2,1-b]furan; 1,5,9-Trimethyl-13-oxabicyclo[10.1.0]trideca-4,8-dien; Rosenoxid; 2-(2,4-Dimethyl-3-cyclohexen-1-yl)-5-methyl-5-(1-methylpropyl)-1,3-dioxan;
der cyclischen und makrocyclischen Ketone wie z.B. 4-tert.-Butylcyclohexanon; 2,2,5-Trimethyl-5-pentylcyclopentanon; 2-Heptylcyclopentanon; 2-Pentylcyclopentanon; 2-Hydroxy-3-methyl-2-cyclopenten-1-on; 3-Methyl-cis-2-penten-1-yl-2-cyclopenten-1-on; 3-Methyl-2-pentyl-2-cyclopenten-1-on; 3-Methyl-4-cyclopentadecenon; 3-Methyl-5-cyclopentadecenon; 3-Methylcyclopentadecanon; 4-(1-Ethoxyvinyl)-3,3,5,5-tetramethylcyclohexanon; 4-tert.-Pentylcyclohexanon; 5-Cyclohexadecen-1-on; 6,7-Dihydro-1,1,2,3,3-pentamethyl-4(5H)-indanon; 8-Cyclohexadecen-1-on; 9-Cycloheptadecen-1-on; Cyclopentadecanon; Cyclohexadecanon;
der cycloaliphatischen Aldehyde wie z.B. 2,4-Dimethyl-3-cyclohexencarbaldehyd; 2-Methyl-4-(2,2,6-trimethyl-cyclohexen-1-yl)-2-butenal; 4-(4-Hydroxy-4-methylpentyl)-3-cyclohexencarbaldehyd; 4-(4-Methyl-3-penten-1-yl)-3-cyclohexencarbaldehyd;
der cycloaliphatischen Ketone wie z. B. 1-(3,3-Dimethylcyclohexyl)-4-penten-1-on; 2,2-Dimethyl-1-(2,4-dimethyl-3-cyclohexen-1-yl)-1-propanon; 1-(5,5-Dimethyl-1-cyclohexen-1-yl)-4-penten-1-on; 2,3,8,8-Tetramethyl-1,2,3,4,5,6,7,8-octahydro-2-naphtalenylmethylketon; Methyl-2,6,10-trimethyl-2,5,9-cyclododecatrienylketon; tert.-Butyl-(2,4-dimethyl-3-cyclohexen-1-yl)keton;
der Ester cyclischer Alkohole wie z.B. 2-tert-Butylcyclohexylacetat; 4-tert-Butylcyclohexylacetat; 2-tert-Pentylcyclohexylacetat; 4-tert-Pentylcyclohexylacetat; 3,3,5-Trimethylcyclohexylacetat; Decahydro-2-naphthylacetat; 2-Cyclopentylcyclopentylcrotonat; 3-Pentyltetrahydro-2H-pyran-4-ylacetat; Decahydro-2,5,5,8a-tetramethyl-2-naphthylacetat; 4,7-Methano-3a,4,5,6,7,7a-hexahydro-5, bzw. 6-indenylacetat; 4,7-Methano-3a,4,5,6,7,7a-hexahydro-5, bzw. 6-indenylpropionat; 4,7-Methano-3a,4,5,6,7,7a-hexahydro-5, bzw. 6-indenylisobutyrat; 4,7-Methanooctahydro-5, bzw. 6-indenylacetat;
der Ester cycloaliphatischer Alkohole wie z.B.1-Cyclohexylethylcrotonat;
der Ester cycloaliphatischer Carbonsäuren wie z. B. Allyl-3-cyclohexylpropionat; Allylcyclohexyloxyacetat; cis- und trans-Methyldihydrojasmonat; cis- und trans-Methyljasmonat; Methyl-2-hexyl-3-oxocyclopentancarboxylat; Ethyl-2-ethyl-6,6-dimethyl-2-cyclohexencarboxylat; Ethyl-2,3,6,6-tetramethyl-2-cyclohexencarboxylat; Ethyl-2-methyl-1,3-dioxolan-2-acetat;
der araliphatischen Alkohole wie z.B. Benzylalkohol; 1-Phenylethylalkohol; 2-Phenylethylalkohol; 3-Phenylpropanol; 2-Phenylpropanol; 2-Phenoxyethanol; 2,2-Dimethyl-3-phenylpropanol; 2,2-Dimethyl-3-(3-methylphenyl)propanol; 1,1-Dimethyl-2-phenylethylalkohol; 1,1-Dimethyl-3-phenylpropanol; 1-Ethyl-1-methyl-3-phenylpropanol; 2-Methyl-5-phenylpentanol; 3-Methyl-5-phenylpentanol; 3-Phenyl-2-propen-1-ol; 4-Methoxybenzylalkohol; 1-(4-Isopropylphenyl)ethanol;
der Ester von araliphatischen Alkoholen und aliphatischen Carbonsäuren wie z.B. Benzylacetat; Benzylpropionat; Benzylisobutyrat; Benzylisovalerianat; 2-Phenylethylacetat; 2-Phenylethylpropionat; 2-Phenylethylisobutyrat; 2-Phenylethylisovalerianat; 1-Phenylethylacetat; alpha-Trichlormethylbenzylacetat; alpha,alpha-Dimethylphenylethylacetat; alpha,alpha-Dimethylphenylethylbutyrat; Cinnamylacetat; 2-Phenoxyethylisobutyrat; 4-Methoxybenzylacetat;
der araliphatischen Ether wie z.B. 2-Phenylethylmethylether; 2-Phenylethylisoamylether; 2-Phenylethyl-1-ethoxyethylether; Phenylacetaldehyddimethylacetal; Phenylacetaldehyddiethylacetal; Hydratropaaldehyddimethylacetal; Phenylacetaldehydglycerinacetal; 2,4,6-Trimethyl-4-phenyl-1,3-dioxan; 4,4a,5,9b-Tetrahydroindeno[1,2-d]-m-dioxin; 4,4a,5,9b-Tetrahydro-2,4-dimethylindeno[1,2-d]-m-dioxin;
der aromatischen und araliphatischen Aldehyde wie z. B. Benzaldehyd; Phenylacetaldehyd; 3-Phenylpropanal; Hydratropaaldehyd; 4-Methylbenzaldehyd; 4-Methylphenylacetaldehyd; 3-(4-Ethylphenyl)-2,2-dimethylpropanal; 2-Methyl-3-(4-isopropylphenyl)propanal; 2-Methyl-3-(4-tert-butylphenyl)propanal; 2-Methyl-3-(4-isobutylphenyl)propanal; 3-(4-tert.-Butylphenyl)propanal; Zimtaldehyd; alpha-Butylzimtaldehyd; alpha-Amylzimtaldehyd; alpha-Hexylzimtaldehyd; 3-Methyl-5-phenylpentanal; 4-Methoxybenzaldehyd; 4-Hydroxy-3-methoxybenzaldehyd; 4-Hydroxy-3-ethoxybenzaldehyd; 3,4-Methylendioxybenzaldehyd; 3,4-Dimethoxybenzaldehyd; 2-Methyl-3-(4-methoxyphenyl)propanal; 2-Methyl-3-(4-methylendioxyphenyl)propanal;
der aromatischen und araliphatischen Ketone wie z.B. Acetophenon; 4-Methylacetophenon; 4-Methoxyacetophenon; 4-tert.-Butyl-2,6-dimethylacetophenon; 4-Phenyl-2-butanon; 4-(4-Hydroxyphenyl)-2-butanon; 1-(2-Naphthalenyl)ethanon;2-Benzofuranylethanon;(3-Methyl-2-benzofuranyl)ethanon; Benzophenon; 1,1,2,3,3,6-Hexamethyl-5-indanylmethylketon; 6-tert.-Butyl-1,1-dimethyl-4-indanylmethylketon; 1-[2,3-dihydro-1,1,2,6-tetramethyl-3-(1-methylethyl)-1H-5-indenyl]ethanon; 5',6',7',8'-Tetrahydro-3',5',5',6',8',8'-hexamethyl-2-acetonaphthon;
der aromatischen und araliphatischen Carbonsäuren und deren Ester wie z.B. Benzoesäure; Phenylessigsäure; Methylbenzoat; Ethylbenzoat; Hexylbenzoat; Benzyl-benzoat; Methylphenylacetat; Ethylphenylacetat; Geranylphenylacetat; Phenylethyl-phenylacetat; Methylcinnmat; Ethylcinnamat; Benzylcinnamat; Phenylethylcinnamat; Cinnamylcinnamat; Allylphenoxyacetat; Methylsalicylat; Isoamylsalicylat; Hexylsalicylat; Cyclohexylsalicylat; Cis-3-Hexenylsalicylat; Benzylsalicylat; Phenylethylsalicylat; Methyl-2,4-dihydroxy-3,6-dimethylbenzoat; Ethyl-3-phenylglycidat; Ethyl-3-methyl-3-phenylglycidat;
der stickstoffhaltigen aromatischen Verbindungen wie z.B. 2,4,6-Trinitro-1,3-dimethyl-5-tert.-butylbenzol; 3,5-Dinitro-2,6-dimethyl-4-tert.-butylacetophenon; Zimtsäurenitril; 3-Methyl-5-phenyl-2-pentensäurenitril; 3-Methyl-5-phenylpentansäurenitril; Methylanthranilat; Methy-N-methylanthranilat; Schiff'sche Basen von Methylanthranilat mit 7-Hydroxy-3,7-dimethyloctanal, 2-Methyl-3-(4-tert.-butylphenyl)propanal oder 2,4-Dimethyl-3-cyclohexencarbaldehyd; 6-Isopropylchinolin; 6-Isobutylchinolin; 6-sec.-Butylchinolin;2-(3-Phenylpropyl)pyridin; Indol; Skatol;
der Phenole, Phenylether und Phenylester wie z.B. Estragol; Anethol; Eugenol; Eugenylmethylether; Isoeugenol; Isoeugenylmethylether; Thymol; Carvacrol; Diphenylether; beta-Naphthylmethylether; beta-Naphthylethylether; beta-Naphthylisobutylether; 1,4-Dimethoxybenzol; Eugenylacetat; 2-Methoxy-4-methylphenol; 2-Ethoxy-5-(1-propenyl)phenol; p-Kresylphenylacetat;
der heterocyclischen Verbindungen wie z.B. 2,5-Dimethyl-4-hydroxy-2H-furan-3-on; 2-Ethyl-4-hydroxy-5-methyl-2H-furan-3-on; 3-Hydroxy-2-methyl-4H-pyran-4-on; 2-Ethyl-3-hydroxy-4H-pyran-4-on;
der Lactone wie z.B. 1,4-Octanolid; 3-Methyl-1,4-octanolid; 1,4-Nonanolid; 1,4-Decanolid; 8-Decen-1,4-olid; 1,4-Undecanolid; 1,4-Dodecanolid; 1,5-Decanolid; 1,5-Dodecanolid;4-Methyl-1,4-decanolid; 1,15-Pentadecanolid; cis- und trans-11-Pentadecen-1,15-olid; cis- und trans-12-Pentadecen-1,15-olid; 1,16-Hexadecanolid; 9-Hexadecen-1,16-olid; 10-Oxa-1,16-hexadecanolid; 11-Oxa-1,16-hexadecanolid; 12-Oxa-1,16-hexadecanolid;
Ethylen-1,12-dodecandioat; Ethylen-1,13-tridecandioat; Cumarin; 2,3-Dihydrocumarin; Octahydrocumarin.

Wie im Folgenden näher erläutert wird (vgl. Syntheseweg gemäß Schema 1 sowie Beispiel 1), lässt sich die Verbindung der Formel (I) in besonders wirtschaftlicher Weise synthetisieren. 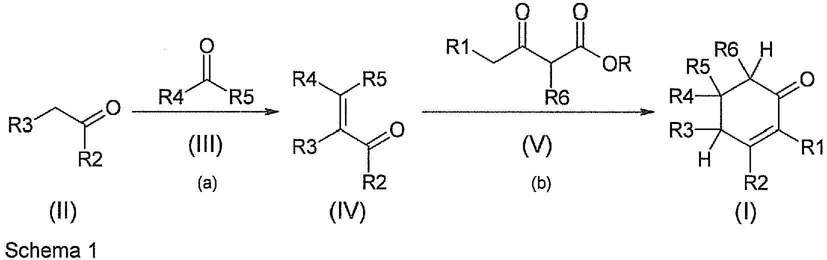
a) Bu₂NH; b) kat. NaOH, ROH; H₂SO₄
   Ausgehend von alpha,beta-ungesättigten Ketonen oder Aldehyden der allgemeinen Formel (IV) und 1,3-Dicarbonylverbindungen der allgemeinen Formel (V) werden durch eine basenkatalysierte Cyclisierungsreaktion mit anschließender Verseifung und Decarboxylierung Verbindungen der allgemeinen Formel (I) hergestellt. Nicht verfügbare Verbindungen der allgemeinen Formel (IV) lassen sich durch basenkatalysierte Aldolkondensation aus Verbindungen der allgemeinen Formeln (II) und (III) darstellen. Wobei die Bedeutung der Reste R1, R2, R3, R4, R5 und R6 in den Verbindungen der Formel (II), (III), (IV) und (V) jeweils die gleiche ist wie die Bedeutung der gleich bezeichneten Reste der Verbindung der Formel (I).

Wie oben beschrieben, werden im Rahmen der vorliegenden Erfindung auch neue Verbindungen der Formel (I) sowie entsprechende Mischungen davon angegeben. Die Herstellung dieser neuen Verbindungen der Formel (I) (wie oben beschrieben) erfolgt mittels eines erfindungsgemäßen Verfahrens.

Dementsprechend beschreibt die vorliegende Erfindung auch ein Verfahren zur Herstellung dieser neuen Verbindungen der Formel (I) mit folgenden Schritten:
(i) Bereitstellen oder Herstellen einer Verbindung der Formel (IV) 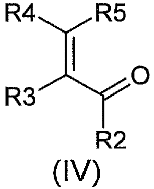 vorzugsweise durch Umsetzen einer Verbindung der Formel (II) und einer Verbindung der Formel (III), so dass eine Verbindung der Formel (IV) resultiert, 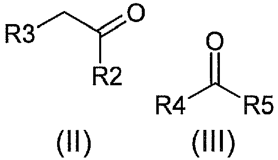 sowie
(ii) Umsetzen der Verbindung der Formel (IV) und einer Verbindung der Formel (V), 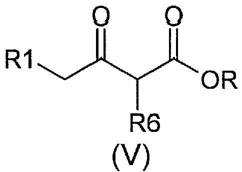 so dass eine Verbindung der Formel (I) resultiert,
   wobei die Bedeutung der Reste R1, R2, R3, R4, R5 und R6 in den Verbindungen der Formel (II), (III), (IV) und (V) jeweils die gleiche ist wie die Bedeutung der gleich bezeichneten Reste der Verbindung der Formel (I).

Bevorzugt ist ein Verfahren zur Herstellung der neuen Verbindungen der Formel (I), bei dem in Schritt (ii) ein Alkalihydroxid, ein Erdalkalihydroxid und/oder ein Alkoholat, vorzugsweise zumindest Natriumhydroxid oder Kaliumhydroxid als Katalysator verwendet wird.

Ebenfalls bevorzugt ist ein Verfahren zur Herstellung der neuen Verbindungen der Formel (I), bei dem die Umsetzung in Schritt (ii) in einem Lösungsmittel, bevorzugt in einem polaren Lösungsmittel, besonders bevorzugt in einem polaren protischen Lösungsmittel, ganz besonders bevorzugt in einem Alkohol durchgeführt wird.

Bensoders bevorzugt ist ein Verfahren zur Herstellung der neuen Verbindungen der Formel (I), bei dem das Umsetzen der Verbindung der Formel (II) mit der Verbindung der Formel (III) in Schritt (ii) in Gegenwart eines sekundären Amins erfolgt, vorzugsweise ausgewählt aus der Gruppe bestehend aus Piperidin, Dibutylamin, Dietylamin und Dimethylamin
und/oder
das Umsetzen der Verbindung der Formel (II) mit der Verbindung der Formel (III) in Schritt (ii) in Gegenwart von Wasser als Lösungsmittel durchgeführt wird.

Alternativ können z. B. die erfindungsgemäßen Verbindungen 13, 43, 44 und 45 (Syntheseweg Schema 2 sowie Beispiel 22), der Formel (VIII) in besonders einfacher Weise synthetisiert werden. 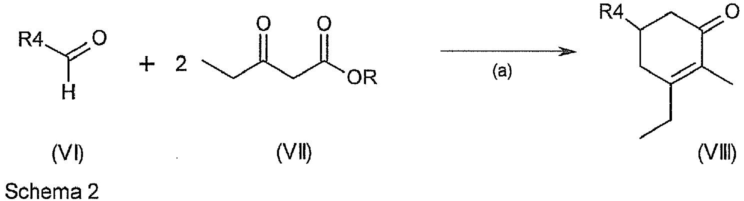
a) Piperidin, NaOH
   Die Verbindungen der allgemeinen Formel (VIII) werden ausgehend von Aldehyden der allgemeinen Formel (VI) und 1,3-Dicarbonylverbindungen der Formel (VII) durch eine basenkatalysierte Cyclisierungsreaktion mit anschließender Verseifung und Decarboxylierung hergestellt. Der Rest R4 der Formel (VIII) hat dabei die Bedeutung: Methyl, Ethyl, n-Propyl und Isopropyl.

Dementsprechend beschreibt die vorliegende Erfindung auch ein alternatives Verfahren zur Herstellung einer Verbindung der Formel (I), vorzugsweise einer der Verbindungen 43, 44 oder 45 wie oben definiert,
wobei für die Reste der Verbindung der Formel (I) gilt, dass
R1 Methyl bedeutet,
R2 Ethyl bedeutet,
R3 Wasserstoff bedeutet,
R4 Methyl, Ethyl, n-Propyl oder Isopropyl bedeutet,
R5 Wasserstoff bedeutet,
R6 Wasserstoff bedeutet,
mit folgenden Schritten:
   (i) Bereitstellen oder Herstellen einer Verbindung der Formel (VI) wobei die Bedeutung des Restes R4 die gleiche ist wie die Bedeutung des gleich bezeichneten Restes der Verbindung der Formel (I),
      sowie
   (ii) Umsetzen der Verbindung der Formel (VI) und einer Verbindung der Formel (VIII), 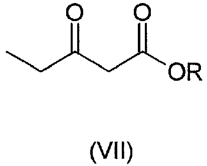
      wobei der Rest R ein verzweigter oder unverzweigter, gesättigter oder ungesättigter Kohlenwasserstoffrest ist, vorzugsweise Methyl, Ethyl, Propyl, Butyl ist,
      so dass eine Verbindung der Formel (I) resultiert,
      und wobei vorzugsweise als Katalysator in Schritt (ii) ein Alkalihydroxid, ein Erdalkalihydroxid und/oder ein Alkoholat, vorzugsweise zumindest Natriumhydroxid oder Kaliumhydroxid verwendet wird
      und wobei vorzugsweise das Umsetzen der Verbindung der Formel (VI) mit der Verbindung der Formel (VII) in Schritt (ii) in Gegenwart eines sekundären Amins erfolgt, vorzugsweise ausgewählt aus der Gruppe bestehend aus Piperidin, Dibutylamin, Dietylamin und Dimethylamin erfolgt.

Nachfolgend wird die vorliegende Erfindung anhand von Beispielen näher erläutert. Sofern nicht anders angegeben ist, beziehen sich alle Angaben auf das Gewicht.

### Beispiel 1: Synthese von 6-Ethyl-4-isopropylcyclohex-2-en-1-on (1)

### Beispiel 1.1: Synthese von 3-Methyl-2-methylenbutanal

Zu einer Lösung aus Isovaleraldehyd (206.7 g, 2.4 mol) und 37%iger wässriger Formaldehydlösung (200.6 g, 2.5 mol) wird bei 80°C Dibutylamin (9.3 g, 70 mmol) zugetropft. Anschließend wird das Reaktionsgemisch 2.5 Stunden am Rückfluss erhitzt. Zur Aufarbeitung wird die Reaktionslösung auf Raumtemperatur abgekühlt und die Phasen getrennt. Erhalten wird 3-Methyl-2-methylenbutanal in Form eines farblosen Öls (240 g, 96%).

¹H-NMR (CDCl₃, 400 MHz): δ 9.54 (s, 1 H), 6.25 (m, 1 H), 5.95 (s, 1 H), 2.80 (m, 1 H), 1.08 ppm (d, J = 7Hz, 6H). ¹³C-NMR (CDCl₃, 100 MHz): δ 194.7, 156.5, 132.2, 26.2, 21.4 ppm. MS (EI): m/z = 27 (38), 41 (100), 55 (60), 69 (59), 83 (28), 98 (39, M^{.+}).

### Beispiel 1.2: Synthese von 6-Ethyl-4-isopropylcyclohex-2-en-1-on (1)

Zu einer 50%igen wässrigen NaOH (8.8 g, 0.11 mol) in Ethanol (90 mL) wurde Ethyl-2-ethylacetoacetat (17.5 g, 0.11 mol) in Ethanol (25 mL) und anschließend 3-Methyl-2-methylenbutanal (10.3 g, 0.11 mol) in Ethanol (25 mL) bei 0°C getropft. Das Reaktionsgemisch wird 1 Stunde am Rückfluss erhitzt und anschließend das Lösungsmittel abdestilliert. Das Reaktionsgemisch wird mit Wasser (170 mL) und MtBE (150 mL) versetzt und die Phasen getrennt. Die organische Phase wird mit 50%iger wässriger Schwefelsäure (8.8 g) neutralisiert, über Natriumsulfat getrocknet und im Vakuum eingeengt. Säulenchromatographische Reinigung an Silicagel (Laufmittel: Cyclohexan/Essigsäureethylester 40/1) ergibt 6-Ethyl-4-isopropylcyclohex-2-en-1-on als Isomerengemisch in Form eines farblosen Öls (2.8 g, 17%).

Hauptverbindung: ¹H-NMR (CDCl₃, 400 MHz): δ 6.81 (m, 1 H), 6.00 (m, 1 H), 2.39 (m, 1 H), 2.17 (m, 1 H), 2.00 (m, 1 H), 1.94 (m, 1 H), 1.82 (m, 1 H), 1.45 (m, 1 H), 1.39 (m, 1 H), 0.96 (d, J = 7Hz, 3H), 0.95 (d, J = 7Hz, 3H), 0.94 ppm (t, J = 7Hz, 3H). ¹³C-NMR (CDCl₃, 100 MHz): δ 201.8, 152.9, 129.9, 47.8, 43.4, 31.7, 30.6, 22.0, 19.3, 19.2, 11.1 ppm. MS (EI): m/z = 27 (9), 41 (19), 55 (11), 67 (14), 81 (12), 95 (100), 110 (7), 123 (12), 138 (15), 151 (2), 166 (3, M⁺).

Nebenverbindung: ¹H-NMR (CDCl₃, 400 MHz): δ 6.84 (m, 1 H), 5.93 (m, 1 H), 2.30 (m, 2H), 1.90 (m, 2H), 1.82 (m, 1 H), 1.72 (m, 1 H), 1.50 (m, 1 H), 1.00 (d, J = 7Hz, 6H), 0.94 ppm (t, J = 7Hz, 3H). ¹³C-NMR (CDCl₃, 100 MHz): δ 202.9, 152.9, 128.5, 46.7, 38.9, 31.4, 29.1, 23.1, 19.9, 11.9 ppm. MS (EI): m/z = 27 (4), 41 (11), 55 (8), 67 (10), 81 (9), 95 (100), 110 (8), 123 (8), 138 (15), 151 (4), 166 (8, M⁺).

Geruchsbeschreibung von 6-Ethyl-4-isopropylcyclohex-2-en-1-on: grün, fruchtig, Grapefruit, Rhabarber, citrisch, frisch, floral.

### Beispiel 2: Synthese von 4-Isopropyl-6-methylcyclohex-2-en-1-on (2)

Synthese analog Beispiel 1.2, ausgehend von 2-Methylacetessigsäureethylester und 3-Methyl-2-methylenbutanal. Fraktionierende Destillation ergibt 4-Isopropyl-6-methylcyclohex-2-en-1-on als Isomerengemisch in Form eines farblosen Öls (34%).

Hauptverbindung: ¹H-NMR (CDCl₃, 400 MHz): δ 6.82 (m, 1H), 6.00 (m, 1H), 2.42 (m, 1H), 2.35 (m, 1 H), 1.97 (m, 1 H), 1.80 (m, 1 H), 1.49 (m, 1 H), 1.14 (d, J = 7Hz, 3H), 0.96 (d, J=7Hz. 3H), 0.94 ppm (d, J = 7Hz, 3H). ¹³C-NMR (CDCl₃, 100 MHz): δ 202.4, 153.3, 129.5, 43.4, 41.6, 34.1, 31.6, 19.4, 19.1, 15.1 ppm. MS (EI): m/z = 27 (13), 43 (23), 55 (9), 67 (17), 82 (21), 95 (100), 110 (42), 137 (4), 152 (15, M⁺).

Nebenverbindung: ¹H-NMR (CDCl₃, 400 MHz): δ 6.89 (m, 1H), 5.95 (m, 1H), 2.30 (m, 1H), 1.98 (m, 1H), 1.81 (m, 2H), 1.15 (d, J = 7Hz, 3H), 1.00 ppm (d, J = 7Hz, 6H). ¹³C-NMR (CDCl₃, 100 MHz): δ 203.0, 153.1, 128.3, 39.3, 39.2, 32.2, 31.4, 20.3, 20.1, 15.8 ppm. MS (EI): m/z = 27 (11), 43 (26), 55 (9), 67 (17), 82 (17), 95 (100), 110 (41), 137 (3), 152 (13, M⁺).

Geruchsbeschreibung von 4-Isopropyl-6-methylcyclohex-2-en-1-on: frisch, süß, würzig, aromatisch, fruchtig, Grapefruit, Wermuttee.

Geschmacksbeschreibung von 4-Isopropyl-6-methylcyclohex-2-en-1-on: krautig, Salbei, balsamisch, Krauseminze, kühlend.

### Beispiel 3: Synthese von 4-Isopropyl-2-methylcyclohex-2-en-1-on (3)

Synthese analog Beispiel 1.2, ausgehend von Methyl-3-oxovalerat und 3-Methyl-2-methylenbutanal. Säulenchromatographische Reinigung an Silicagel (Laufmittel: Cyclohexan/Essigsäureethylester 40/1 bis 30/1) ergibt 4-Isopropyl-2-methylcyclohex-2-en-1-on als Isomerengemisch in Form eines farblosen Öls (17%).

¹H-NMR (CDCl₃, 400 MHz): δ 6.65 (m, 1 H), 2.53 (m, 1 H), 2.32 (m, 1 H), 2.27 (m, 1 H), 1.97 (m, 1 H), 1.78 (m, 3H), 1.76 (m, 1 H), 1.72 (m, 1H), 0.96 (d, J = 7Hz, 3H), 0.94 ppm (d, J = 7Hz, 3H). ¹³C-NMR (CDCl₃, 100 MHz): δ 200.4, 149.5, 135.5, 42.9, 37.7, 31.8, 25.7, 19.7, 19.4, 16.1 ppm. MS (EI): m/z = 27 (18), 41 (46), 53 (19), 67 (23), 81 (46), 95 (100), 110 (79), 123 (4), 137 (8), 152 (44, M^{.+}).

Geruchsbeschreibung von 4-Isopropyl-2-methylcyclohex-2-en-1-on: würzig, krautig, fettig, floral, Cumin.

### Beispiel 4: Synthese von 2-Ethyl-4-isopropylcyclohex-2-en-1-on (4)

Synthese analog Beispiel 1.2, ausgehend von Ethyl-3-oxohexanoat und 3-Methyl-2-methylenbutanal. Säulenchromatographische Reinigung an Silicagel (Laufmittel: Cyclohexan/Essigsäureethylester 25/1) ergibt 2-Ethyl-4-isopropylcyclohex-2-en-1-on als Isomerengemisch in Form eines farblosen Öls (23%).

¹H-NMR (CDCl₃, 400 MHz): δ 6.60 (m, 1 H), 2.52 (m, 1 H), 2.32 (m, 1 H), 2.28 (m, 1 H), 2.21 (m, 1H), 1.96 (m, 1H), 1.80 (m, 1H), 1.70 (m, 1 H), 1.01 (t, J = 7Hz, 3H), 0.97 (d, J = 7Hz, 3H), 0.95 ppm (d, J = 7Hz, 3H). ¹³C-NMR (CDCl₃, 100 MHz): δ 199.9 147.9, 141.0, 42.7, 37.9, 31.8, 25.4, 22.6, 19.7, 19.4, 13.1 ppm. MS (EI): m/z = 27 (49), 41 (93), 55 (52), 67 (56), 81 (100), 95 (96), 109 (83), 123 (61), 137 (7), 151 (9), 166 (39, M^{.+}).

Geruchsbeschreibung von 2-Ethyl-4-isopropylcyclohex-2-en-1-on: fruchtig, Damascon, Safran, würzig, Kamille.

Geschmacksbeschreibung von 2-Ethyl-4-isopropylcyclohex-2-en-1-on: krautig, Pfeffer, Geranium, holzig, blumig.

### Beispiel 5: Synthese von 5-Ethyl-4-methylcyclohex-2-en-1-on (5)

Synthese analog Beispiel 1.2, ausgehend von Ethylacetoacetat und 2-Methyl-2-pentenal. Fraktionierende Destillation ergibt 5-Ethyl-4-methylcyclohex-2-en-1-on als Isomerengemisch in Form eines farblosen Öls (10%).

Hauptverbindung: ¹H-NMR (CDCl₃, 400 MHz): δ 6.74 (m, 1H), 5.94 (m, 1H), 2.55 (m, 1H), 2.28 (m, 1H), 2.14 (m, 1H), 1.70 (m, 1H), 1.64 (m, 1H), 1.35 (m, 1H), 1.18 (d, J = 7Hz, 3H), 0.91 ppm (t, J = 7Hz, 3H). ¹³C-NMR (CDCl₃, 100 MHz): δ 200.4, 156.2, 128.3, 43.1, 41.7, 35.9, 25.7, 18.5, 10.5 ppm. MS (EI): m/z = 27 (25), 39 (34), 54 (45), 68 (42), 82 (100), 95 (13), 109 (19), 123 (4), 138 (26, M^{.+}).

Nebenverbindung: ¹H-NMR (CDCl₃, 400 MHz): δ 6.96 (m, 1 H), 5.94 (m, 1 H), 2.55 (m, 1 H), 2.35 (m, 2H), 2.06 (m, 1 H), 1.43 (m, 2H), 1.02 (d, J = 7Hz, 3H), 0.93 ppm (t, J = 7Hz, 3H). ¹³C-NMR (CDCl₃, 100 MHz): δ 200.1, 156.1, 128.1, 39.5, 39.2, 33.0, 24.9, 12.0, 11.3 ppm. MS (EI): m/z = 27 (19), 39 (28), 54 (40), 68 (9), 82 (100), 95 (9), 109 (15), 123 (2), 138 (19, M^{.+}).

Geruchsbeschreibung von 5-Ethyl-4-methylcyclohex-2-en-1-on: würzig, Mandel.

### Beispiel 6: Synthese von 5-Ethyl-2,4-dimethylcyclohex-2-en-1-on (8)

Synthese analog Beispiel 1.2, ausgehend von Methyl-3-oxovalerat und 2-Methyl-2-pentenal. Säulenchromatographische Reinigung an Silicagel (Laufmittel: Cyclohexan/Essigsäureethylester 20/1) ergibt 5-Ethyl-2,4-dimethylcyclohex-2-en-1-on als Isomerengemisch in Form eines farblosen Öls (5%).

Hauptverbindung: ¹H-NMR (CDCl₃, 400 MHz): δ 6.48 (m, 1H), 2.56 (m, 1H), 2.25 (m, 1H), 2.11 (m, 1H), 1.75 (m, 3H), 1.64 (m, 2H), 1.26 (m, 1H), 1.14 (d, J = 7Hz, 3H), 0.91 ppm (t, J = 7Hz, 3H). ¹³C-NMR (CDCl₃, 100 MHz): δ 200.5, 151.5, 134.2, 43.6, 42.0, 36.2, 25.7, 18.8, 15.6, 10.6 ppm. MS (EI): m/z = 27 (15), 41 (45), 55 (21), 69 (60), 82 (14), 95 (100), 109 (9), 123 (18), 137 (2), 152 (11, M^{.+}).

Nebenverbindung: ¹H-NMR(CDCl_{3,} 400 MHz): δ 6.70 (m, 1H), 2.50 (m, 1H), 2.30 (m, 2H), 2.04 (m, 1H), 1.75 (m, 3H), 1.35 (m, 2H), 0.99 (d, J = 7Hz, 3H), 0.90 ppm (t, J = 7Hz, 3H). ¹³C-NMR (CDCl₃, 100 MHz): δ 200.2, 151.3, 133.9, 39.6, 33.2, 24.9, 15.8, 12.3, 11.3 ppm. MS (EI): m/z = 27 (17), 41 (42), 55 (25), 69 (58), 82 (8), 95 (100), 109 (17), 123 (33), 137 (2), 152 (17, M^{.+}).

Geruchsbeschreibung von 5-Ethyl-2,4-dimethylcyclohex-2-en-1-on: aromatisch, krautig, würzig, medizinisch, Mandel, Cumarin.

### Beispiel 7: Synthese von 2,5-Diethyl-4-methylcyclohex-2-en-1-on (9)

Synthese analog Beispiel 1.2, ausgehend von Ethyl-3-oxohexanoat und 2-Methyl-2-pentenal. Säulenchromatographische Reinigung an Silicagel (Laufmittel: Cyclohexan/Essigsäureethylester 99/1) ergibt 2,5-Diethyl-4-methylcyclohex-2-en-1-on als Isomerengemisch in Form eines farblosen Öls (10%).

Hauptverbindung: ¹H-NMR (CDCl₃, 400 MHz): δ 6.42 (m, 1 H), 2.86 (m, 1 H), 2.30-2.15 (m, 4H), 1.64 (m, 2H), 1.30 (m, 1H), 1.15 (d, J = 7Hz, 3H), 1.00 (t, J = 7Hz, 3H), 0.99 ppm (t, J = 7Hz, 3H). ¹³C-NMR (CDCl₃, 100 MHz): δ 200.2, 149.9, 139.7, 43.4, 42.3, 36.1, 25.7, 22.1, 18.9, 12.9, 10.6 ppm. MS (EI): m/z = 29 (15), 41 (41), 55 (51), 67 (79), 83 (74), 95 (50), 109 (100), 124 (11), 137 (75), 151 (8), 166 (30, M^{.+}).

Nebenverbindung: ¹H-NMR (CDCl₃, 400 MHz): δ 6.65 (m, 1H), 2.55 (m, 1H), 2.35-2.15 (m, 4H), 2.02 (m, 1H), 1.37 (m, 2H), 1.00 (t, J = 7Hz, 3H), 0.99 (d, J = 7Hz, 3H), 0.92 ppm (t, J = 7Hz, 3H). ¹³C-NMR (CDCl₃, 100 MHz): δ 199.8, 149.7, 139.4, 39.8, 39.4, 33.1, 24.8, 22.2, 12.9, 12.4, 11.3 ppm. MS (EI): m/z = 29 (15), 41 (42), 55 (48), 67 (93), 83 (67), 95 (55), 109 (100), 124 (15), 137 (71), 151 (8), 166 (27, M^{.+}).

Geruchsbeschreibung von 2,5-Diethyl-4-methylcyclohex-2-en-1-on: würzig, aromatisch, Krauseminze, Safran, Anis, Fenchel, Tabak.

### Beispiel 8: Synthese von 3-Ethyl-5-methylcyclohex-2-en-1-on (10)

Synthese analog Beispiel 1.2, ausgehend von Ethylacetoacetat und 4-Hexen-3-on. Fraktionierende Destillation ergibt 3-Ethyl-5-methylcyclohex-2-en-1-on als Isomerengemisch in Form eines farblosen Öls (55%).

¹H-NMR (CDCl₃, 400 MHz): δ 5.87 (m, 1H), 2.43 (m, 1H), 2.31 (m, 1H), 2.24 (m, 2H), 2.17 (m, 1 H), 2.04 (m, 1 H), 2.03 (m, 1 H), 1.10 (t, J = 7Hz, 3H), 1.07 ppm (d, J = 7Hz, 3H). ¹³C-NMR (CDCl₃, 100 MHz): δ 200.3, 167.0, 124.3, 45.6, 38.2, 30.8, 30.2, 21.2, 11.3 ppm. MS (EI): m/z = 39 (19), 53 (15), 67 (30), 81 (37), 96 (100), 109 (2), 123 (6), 138 (42, M^{.+}).

Geruchsbeschreibung von 3-Ethyl-5-methylcyclohex-2-en-1-on: würzig, aromatisch, Liebstöckel, Sellerie, Bockshornklee.

### Beispiel 9: Synthese von 3-Ethyl-5,6-dimethylcyclohex-2-en-1-on (12)

Synthese analog Beispiel 1.2, ausgehend von 2-Methylacetessigsäureethylester und 4-Hexen-3-on. Fraktionierende Destillation ergibt 3-Ethyl-5,6-dimethylcyclohex-2-en-1-on als Isomerengemisch in Form eines farblosen Öls (12%).

Hauptverbindung: ¹H-NMR (CDCl₃, 400 MHz): δ 5.85 (m, 1H), 2.30 (m, 1H), 2.21 (m, 2H), 2.10 (m, 1 H), 1.99 (m, 1H), 1.84 (m, 1 H), 1.15 (d, J = 7Hz, 3H), 1.09 (t, J = 7Hz, 3H), 1.08 ppm (d, J = 7Hz, 3H). ¹³C-NMR (CDCl₃, 100 MHz): δ 201.2, 165.3, 123.8, 47.8, 38.2, 36.2, 30.6, 20.0, 12.4, 11.3 ppm. MS (EI): m/z = 27 (11), 39 (17), 53 (13), 67 (26), 81 (32), 96 (100), 109 (4), 123 (4), 137 (9), 152 (11, M⁺).

Nebenverbindung: ¹H-NMR (CDCl₃, 400 MHz): δ 5.81 (m, 1 H), 2.43 (m, 1H), 2.30 (m, 2H), 2.21 (m, 2H), 2.10 (m, 1 H), 1.12 (t, J = 7Hz, 3H), 1.04 (d, J = 7Hz, 3H), 0.95 ppm (d, J = 7Hz, 3H). ¹³C-NMR (CDCl₃, 100 MHz): δ 203.5, 165.1, 123.3, 45.5, 35.8, 33.3, 30.7, 15.7, 11.3, 10.8 ppm. MS (EI): m/z = 27 (11), 39 (17), 53 (13), 67 (26), 81 (32), 96 (100), 109 (2), 123 (4), 137 (9), 152 (13, M⁺).

Geruchsbeschreibung von 5-Ethyl-5,6-dimethylcyclohex-2-en-1-on: würzig, Safran, grün, natürlich, Minze.

### Beispiel 10: Synthese von 3-Ethyl-2,5-dimethylcyclohex-2-en-1-on (13)

Synthese analog Beispiel 1.2, ausgehend von Methyl-3-oxovalerat und 4-Hexen-3-on. Fraktionierende Destillation ergibt 3-Ethyl-2,5-dimethylcyclohex-2-en-1-on als Isomerengemisch in Form eines farblosen Öls (31%).

¹H-NMR (CDCl₃, 400 MHz): δ 2.47 (m, 1H), 2.36 (m, 1 H), 2.23 (m, 2H), 2.10 (m, 1H), 2.08 (m, 1H), 2.03 (m, 1H), 1.76 (m, 3H), 1.07 (t, J = 7Hz, 3H), 1.04 ppm (m, 3H). ¹³C-NMR (CDCl₃, 100 MHz): δ 199.8, 159.5, 129.8, 45.8, 38.7, 29.8, 28.2, 21.2, 11.6, 10.2 ppm. MS (EI): m/z = 27 (19), 41 (42), 53 (22), 67 (100), 82 (27), 95 (32), 110 (90), 123 (10), 137 (27), 152 (68, M⁺).

Geruchsbeschreibung von 3-Ethyl-2,5-dimethylcyclohex-2-en-1-on: würzig, holzig, Leder, Safran, grün, weich, cremig.

Geschmacksbeschreibung von 3-Ethyl-2,5-dimethylcyclohex-2-en-1-on: medizinisch, frisch, holzig.

### Beispiel 11: Synthese von 2,3-Diethyl-5-methylcyclohex-2-en-1-on (14)

Synthese analog Beispiel 1.2, ausgehend von Ethyl-3-oxohexanoat und 4-Hexen-3-on. Fraktionierende Destillation ergibt 2,3-Diethyl-5-methylcyclohex-2-en-1-on als Isomerengemisch in Form eines farblosen Öls (40%).

¹H-NMR (CDCl₃, 400 MHz): δ 2.64 (m, 1 H), 2.34 (m, 1 H), 2.27 (m, 4H), 2.08 (m, 3H), 1.09 (t, J = 8Hz, 3H), 1.03 (d, J = 6Hz, 3H), 0.92 ppm (t, J = 7Hz, 3H). ¹³C-NMR (CDCl₃, 100 MHz): δ 199.5, 159.1, 136.0, 46.2, 38.5, 29.8, 27.7, 21.2, 18.1, 14.2, 12.4 ppm. MS (EI): m/z = 29 (11), 41 (44), 55 (21), 67 (32), 81 (100), 95 (32), 109 (47), 124 (93), 137 (46), 151 (30), 166 (100, M⁺).

Geruchsbeschreibung von 2,3-Diethyl-5-methylcyclohex-2-en-1-on: würzig, krautig, Safran.

### Beispiel 12: Synthese von 4,5-Dimethylcyclohex-2-en-1-on (15)

Synthese analog Beispiel 1.2, ausgehend von Ethylacetoacetat und Tiglinaldehyd. Fraktionierende Destillation ergibt 4,5-Dimethylcyclohex-2-en-1-on als Isomerengemisch in Form eines farblosen Öls (15%).

Hauptverbindung: ¹H-NMR (CDCl₃, 400 MHz): δ 6.74 (m, 1H), 5.91 (m, 1H), 2.46 (m, 1H), 2.17 (m, 1H), 2.15 (m, 1 H), 1.83 (m, 1H), 1.18 (d, J = 7Hz, 3H), 1.08 ppm (d, J = 7Hz, 3H). ¹³C-NMR (CDCl₃, 100 MHz): δ 200.3, 156.2, 128.5, 45.6, 38.3, 37.1, 19.6, 18.5 ppm. MS (EI): m/z = 27 (27), 39 (40), 54 (53), 68 (61), 82 (100), 96 (19), 109 (10), 124 (41, m⁺).

Nebenverbindung: ¹H-NMR (CDCl₃, 400 MHz): δ 6.56 (m, 1H), 5.94 (m, 1H), 2.55 (m, 1 H), 2.34 (m, 3H), 1.06 (d, J = 7Hz, 3H), 0.99 ppm (d, J = 7Hz, 3H). ¹³C-NMR (CDCl₃, 100 MHz): δ 200.6, 155.4, 128.1, 42.7, 34.7, 32.7, 16.6, 13.4 ppm. MS (EI): m/z = 27 (23), 39 (33), 54 (48), 68 (27), 82 (100), 96 (19), 109 (10), 124 (30, M^{.+}).

Geruchsbeschreibung von 4,5-Dimethylcyclohex-2-en-1-on: Mandel, süß, Tabak, Kirsche, fruchtig, Cumarin.

### Beispiel 13: Synthese von 2,4,5-Trimethylcyclohex-2-en-1-on (18)

Synthese analog Beispiel 1.2, ausgehend von Methyl-3-oxovalerat und Tiglinaldehyd. Fraktionierende Destillation ergibt 2,4,5-Trimethylcyclohex-2-en-1-on als Isomerengemisch in Form eines farblosen Öls (30%).

Hauptverbindung:¹H-NMR (CDCl₃, 400 MHz): δ 6.48 (m, 1H), 2.45 (m, 1H), 2.14 (m, 1H), 2.12 (m, 1H), 1.79 (m, 1H), 1.76 (m, 3H), 1.14 (d, J = 7Hz, 3H), 1.05 ppm (d, J = 7Hz, 3H). ¹³C-NMR (CDCl₃, 100 MHz): δ 200.5, 151.5, 134.4, 45.8, 38.6, 37.5, 19.5, 18.8, 15.6 ppm. MS (EI): m/z = 27 (19), 41 (56), 53 (28), 69 (100), 81 (48), 95 (92), 110 (42), 123 (15), 138 (33, M^{.+}).

Nebenverbindung: ¹H-NMR (CDCl₃, 400 MHz): δ 6.60 (m, 1H), 2.53 (m, 1H), 2.33 (m, 3H), 1.76 (m, 3H), 1.03 (d, J = 7Hz, 3H), 0.95 ppm (d, J = 7Hz, 3H). ¹³C-NMR (CDCl₃, 100 MHz): δ 200.1, 150.6, 133.9, 42.8, 37.1, 34.1, 16.5, 15.7, 13.8 ppm. MS (EI): m/z = 27 (22), 41 (60), 53 (31), 69 (100), 81 (59), 95 (100), 110 (47), 123 (15), 138 (31, M^{.+}).

Geruchsbeschreibung von 2,4,5-Trimethylcyclohex-2-en-1-on: würzig, aromatisch, Kirsche, Cumarin, Mandel.

### Beispiel 14: Synthese von 2-Ethyl-4,5-dimethylcyclohex-2-en-1-on (19)

Synthese analog Beispiel 1.2, ausgehend von Ethyl-3-oxohexanoat und Tiglinaldehyd. Fraktionierende Destillation ergibt 2-Ethyl-4,5-dimethylcyclohex-2-en-1-on als Isomerengemisch in Form eines farblosen Öls (29%).

Hauptverbindung:¹H-NMR (CDCl₃, 400 MHz): δ 6.42 (m, 1H), 2.46 (m, 1H), 2.19 (m, 2H), 2.17-2.10 (m, 2H), 1.77 (m, 1 H), 1.15 (d, J = 7Hz, 3H), 1.05 (d, J = 6Hz, 3H), 1.00 ppm (t, J = 8Hz, 3H). ¹³C-NMR (CDCl₃, 100 MHz): δ 200.1, 149.8, 139.9, 46.1, 38.6, 37.4, 22.1, 19.5, 18.9, 12.9 ppm. MS (EI): m/z = 27 (24), 41 (42), 55 (50), 67 (93), 83 (69), 95 (100), 110 (68), 123 (24), 137 (35), 152 (43, M^{.+}).

Nebenverbindung: ¹H-NMR (CDCl₃, 400 MHz): δ 6.53 (m, 1H), 2.52 (m, 1H), 2.35-2.27 (m, 3H), 2.19 (m, 2H), 1.03 (d, J = 7Hz, 3H), 1.00 (t, J = 7Hz, 3H), 0.93 ppm (m, 3H). ¹³C-NMR (CDCl₃, 100 MHz): δ 199.6, 148.8, 139.4, 43.1, 34.8, 32.9, 22.2, 16.4, 14.0, 12.9 ppm. MS (EI): m/z = 27 (23), 41 (42), 55 (58), 67 (86), 83 (78), 95 (100), 110 (60), 123 (23), 137 (38), 152 (48, M^{.+}).

Geruchsbeschreibung von 2-Ethyl-4,5-dimethylcyclohex-2-en-1-on: würzig, krautig, Safran.

### Beispiel 15: Synthese von 2,3,4,5-Tetramethylcyclohex-2-en-1-on (23)

Synthese analog Beispiel 1.2, ausgehend von Methyl-3-oxovalerat und 3-Methyl-3-penten-2-on. Fraktionierende Destillation ergibt 2,3,4,5-Tetramethylcyclohex-2-en-1-on als Isomerengemisch in Form eines farblosen Öls (12%).

Hauptverbindung: ¹H-NMR (CDCl₃, 400 MHz): δ 2.30 (m, 1H), 2.25 (m, 3H), 1.95 (m, 3H), 1.74 (m, 3H), 1.02 (d, J = 7Hz, 3H), 0.98 ppm (d, J = 6Hz, 3H). ¹³C-NMR (CDCl₃, 100 MHz): δ 199.2, 160.8, 129.8, 41.3, 40.3, 32.2, 20.5, 18.4, 11.1, 10.9 ppm. MS (EI): m/z = 27 (15), 41 (40), 55 (30), 67 (100), 83 (75), 95 (65), 110 (97), 123 (6), 137 (12), 152 (59, Mm^{.+}).

Nebenverbindung: ¹H-NMR (CDCl₃, 400 MHz): δ 2.61 (m, 1 H), 2.16 (m, 1 H), 2.10 (m, 1 H), 1.95 (m, 1 H), 1.91 (m, 3H), 1.75 (m, 3H), 1.22 (d, J = 7Hz, 3H), 1.04 ppm (d, J=6Hz_{,} 3H). ¹³C-NMR (CDCl₃, 100 MHz): δ 198.4, 157.1, 129.9, 43.2, 41.5, 34.8, 20.2, 20.1, 18.1, 10.2 ppm. MS (EI): m/z = 27 (15), 41 (41), 55 (32), 67 (100), 83 (75), 95 (64), 110 (85), 123 (7), 137 (15), 152 (55, M⁺).

Geruchsbeschreibung von 2,3,4,5-Tetramethylcyclohex-2-en-1-on: würzig, Safran, Leder, Tabak.

Geschmacksbeschreibung von 2,3,4,5-Tetramethylcyclohex-2-en-1-on: medizinisch, holzig, stechend, bitter, kühlend.

### Beispiel 16: Synthese von 2-Ethyl-3,4,5-trimethylcyclohex-2-en-1-on (24)

Synthese analog Beispiel 1.2, ausgehend von Ethyl-3-oxohexanoat und 3-Methyl-3-penten-2-on. Fraktionierende Destillation ergibt 2-Ethyl-3,4,5-trimethylcyclohex-2-en-1-on als Isomerengemisch in Form eines farblosen Öls (11%).

Hauptverbindung:¹H-NMR (CDCl₃, 400 MHz): δ 2.35-2.20 (m, 6H), 1.96 (m, 3H), 1.02 (d, J = 7Hz, 3H), 0.97 (d, J = 6Hz, 3H), 0.91 ppm (t, J = BHz, 3H). ¹³C-NMR (CDCl₃, 100 MHz): δ 198.7, 160.4, 135.9, 41.3, 40.5, 32.2, 19.8, 18.5, 18.4, 13.4, 11.2 ppm. MS (EI): m/z = 29 (16), 41 (53), 55 (24), 67 (41), 81 (100), 97 (62), 109 (72), 124 (87), 137 (19), 151 (38), 166 (79, M'⁺).

Nebenverbindung: ¹H-NMR (CDCl₃, 400 MHz): δ 2.60 (m, 1 H), 2.35-2.20 (m, 2H), 2.14 (m, 1 H), 2.08 (m, 1 H), 1.95-1,93 (m, 1 H), 1.92 (m, 3H), 1.22 (d, J = 7Hz, 3H), 1.04 (d, J = 7Hz, 3H), 0.91 ppm (t, J = BHz, 3H). ¹³C-NMR (CDCl₃, 100 MHz): δ 197.9. 156.5, 136.0, 43.2, 41.7, 34.7, 20.0, 19.6, 18.5, 18.2, 13.3 ppm. MS (EI): m/z = 29 (13), 41 (52), 55 (22), 67 (36), 81 (100), 97 (67), 109 (66), 124 (96), 137 (19), 151 (39), 166 (77, M⁺).

Geruchsbeschreibung von 2-Ethyl-3,4,5-trimethylcyclohex-2-en-1-on: frisch, minzig, Tabak, Safran, würzig, Liebstöckel.

### Beispiel 17: Synthese von 2-Ethyl-5,5-dimethylcyclohex-2-en-1-on (28)

Synthese analog Beispiel 1.2, ausgehend von Ethyl-3-oxohexanoat und 3-Methylbut-2-enal. Säulenchromatographische Reinigung an Silicagel (Laufmittel: Cyclohexan/Essigsäureethylester 30/1) ergibt 2-Ethyl-5,5-dimethylcyclohex-2-en-1-on in Form eines farblosen Öls (4%).

¹H-NMR (CDCl₃, 400 MHz): δ 6.57 (m, 1H), 2.28 (s, 2H), 2.24 (m, 2H), 2.22 (m, 2H), 1.02 (s, 6H), 1.01 ppm (t, J = 8Hz, 3H). ¹³C-NMR (CDCl₃, 100 MHz): δ 199.7, 141.6, 140.2, 52.2, 40.1, 34.0, 28.3, 22.1, 12.9 ppm. MS (EI): m/z = 27 (5), 41 (15), 53 (12), 67 (30), 81 (17), 96 (100), 109 (20), 123 (2), 137 (5), 152 (27, M⁺).

Geruchsbeschreibung von 2-Ethyl-5,5-dimethylcyclohex-2-en-1-on: würzig, Liebstöckel, Safran, Apfel.

### Beispiel 18: Synthese von 2,3,5,5-Tetramethylcyclohex-2-en-1-on (29)

Synthese analog Beispiel 1.2, ausgehend von Methyl-3-oxovalerat und Mesityloxid. Fraktionierende Destillation ergibt 2,3,5,5-Tetramethylcyclohex-2-en-1-on in Form eines farblosen Öls (5%).

¹H-NMR (CDCl₃, 400 MHz): δ 2.24 (s, 2H), 2.20 (m, 2H), 1.90 (m, 3H), 1.77 (m, 3H), 1.00 ppm (s, 6H). ¹³C-NMR (CDCl₃, 100 MHz): δ 199.3, 152.5, 129.8, 51.1, 47.0, 32.7, 28.3, 21.6, 10.5 ppm. MS (EI): m/z = 27 (8), 41 (15), 53 (11), 68 (32), 83 (6), 96 (100), 109 (9), 124 (2), 137 (4), 152 (34, M⁺).

Geruchsbeschreibung von 2,3,5,5-Tetramethylcyclohex-2-en-1-on: Tabak, würzig, Leder, animalisch.

Geschmacksbeschreibung von 2,3,5,5-Tetramethylcyclohex-2-en-1-on: medizinisch, frisch, Krauseminze, Getreide, Heu, Klee.

### Beispiel 19: Synthese von 2-Ethyl-3,5,5-trimethyl-cyclohex-2-en-1-on (30)

Synthese analog Beispiel 1.2, ausgehend von Ethyl-3-oxohexanoat und Mesityloxid. Fraktionierende Destillation ergibt 2-Ethyl-3,5,5-trimethyl-cyclohex-2-en-1-on in Form eines farblosen Öls (6%).

¹H-NMR (CDCl₃, 400 MHz): δ 2.31 (m, 2H), 2.23 (s, 2H), 2.21 (m, 2H), 1.92 (m, 3H), 1.00 (s, 6H), 0.93 ppm (t, J = 7Hz, 3H). ¹³C-NMR (CDCl₃, 100 MHz): δ 198.8 152.0, 136.0, 51.4, 47.0, 32.7, 28.2, 21.0, 18.2, 13.5 ppm. MS (EI): m/z = 29 (8), 41 (25), 55 (9), 67 (49), 82 (47), 95 (9), 110 (100), 123 (9), 137 (1), 151 (6), 166 (36, M^{.+}).

Geruchsbeschreibung von 2-Ethyl-3,5,5-trimethylcyclohex-2-en-1-on: Tabak, würzig, Leder, Safran.

### Beispiel 20: Synthese von 6-Ethyl-4-methylcyclohex-2-en-1-on (31)

Synthese analog Beispiel 1.2, ausgehend von wurde Ethyl-2-ethylacetoacetat und Methacrolein. Säulenchromatographische Reinigung an Silicagel (Laufmittel: Cyclohexan/Essigsäureethylester 30/1) ergibt 6-Ethyl-4-methylcyclohex-2-en-1-on in Form eines farblosen Öls (15%).

Hauptverbindung: ¹H-NMR (CDCl₃, 400 MHz): δ 6.71 (m, 1H), 5.94 (m, 1H), 2.60 (m, 1H), 2.19 (m, 1 H), 2.12 (m, 1H), 1.93 (m, 1 H), 1.40 (m, 1H), 1.36 (m, 1H), 1.15 (d, J = 7Hz, 3H), 0.93 ppm (t, J = 7Hz, 3H). ¹³C-NMR (CDCl₃, 100 MHz): δ 201.5, 155.2, 128.8, 47.6, 36.6, 32.2, 21.8, 21.0, 11.0 ppm. MS (El): m/z = 27 (15), 39 (22), 54 (31), 67 (11), 82 (100), 96 (59), 110 (53), 123 (7), 138 (4, M⁺).

Nebenverbindung: ¹H-NMR (CDCl₃, 400 MHz): δ 6.74 (m, 1H), 5.87 (m, 1H), 2.60 (m, 1H), 2.30 (m, 1H), 2.00 (m, 1H), 1.79 (m, 1H), 1.74 (m, 1H), 1.49 (m, 1H), 1.16 (d, J = 7Hz, 3H), 0.96 ppm (t, J = 8Hz, 3H). ¹³C-NMR (CDCl₃, 100 MHz): δ 202.3, 154.6, 127.6, 45.8, 34.6, 28.1, 22.8, 19.7, 11.7 ppm. MS (El): m/z = 27 (14), 39 (21), 54 (32), 67 (9), 82 (100), 96 (46), 110 (42), 123 (4), 138 (6, M⁺).

Geruchsbeschreibung von 6-Ethyl-4-methylcyclohex-2-en-1-on: grün, nussig, lactonisch, fruchtig, Kokos.

### Beispiel 21: Synthese von 4,6-Diethylcyclohex-2-en-1-on (32)

Synthese analog Beispiel 1.2, ausgehend von Ethyl-2-ethylacetoacetat und 2-Ethylacrolein. Säulenchromatographische Reinigung an Silicagel (Laufmittel: Cyclohexan/Essigsäureethylester 30/1) ergibt 4,6-Diethylcyclohex-2-en-1-on als Isomerengemisch in Form eines farblosen Öls (12%).

Hauptverbindung:¹H-NMR (CDCl₃, 400 MHz): δ 6.78 (m, 1H), 5.97 (m, 1H), 2.39 (m, 1H), 2.18 (m, 1 H), 2.12 (m, 1 H), 1.94 (m, 1 H), 1.54 (m, 2H), 1.40 (m, 1 H), 1.30 (m, 1 H), 1.00 (t, J = 7Hz, 3H), 0.94 ppm (t, J = 8Hz, 3H). ¹³C-NMR (CDCl₃, 100 MHz): δ 201.8, 153.9, 129.3, 47.7, 38.8, 33.9, 28.2, 22.0, 11.1 ppm. MS (EI): m/z = 27 (6), 41 (12), 55 (16), 67 (16), 81 (76), 96 (100), 109 (10), 124 (31), 152 (6, M⁺).

Nebenverbindung: ¹³C-NMR (CDCl₃, 100 MHz): δ 202.7, 153.4, 128.0, 46.1, 34.7, 32.0, 27.2, 22.9, 11.8, 11.7 ppm. MS (El): m/z 27 (8), 41 (14), 55 (16), 67 (18), 81 (82), 96 (100), 109 (8), 124 (24), 152 (10, M⁺).

Geruchsbeschreibung von 4,6-Diethylcyclohex-2-en-1-on: würzig, Kümmel, fettig, grün.

### Beispiel 22: Synthese von 4-Ethyl-6-methylcyclohex-2-en-1-on (33)

Synthese analog Beispiel 1.2, ausgehend von 2-Methylacetessigsäureethylester und 2-Ethylacrolein. Säulenchromatographische Reinigung an Silicagel (Laufmittel: Cyclohexan/Essigsäureethylester 30/1) ergibt 4-Ethyl-6-methylcyclohex-2-en-1-on als Isomerengemisch in Form eines farblosen Öls (35%).

Hauptverbindung: ¹H-NMR (CDCl₃, 400 MHz): δ 6.79 (m, 1 H), 5.97 (m, 1 H), 2.42 (m, 1 H), 2.37 (m, 1H), 2.09 (m, 1H), 1.52 (m, 1H), 1.45 (m, 1H), 1.39 (m, 1H), 1.14 (d, J = 7Hz, 3H), 0.99 ppm (t, J = 7Hz, 3H). ¹³C-NMR (CDCl₃, 100 MHz): δ 202.4, 154.2, 128.8, 41.5, 38.7, 37.5, 28.1, 15.0, 11.1 ppm. MS (El): m/z = 27 (14), 41 (17), 53 (20), 67 (18), 81 (100), 96 (81), 110 (9), 138 (19, M⁺).

Nebenverbindung: ¹H-NMR (CDCl₃, 400 MHz): δ 6.85 (m, 1 H), 5.91 (m, 1H), 2.53 (m, 1 H), 2.37 (m, 1H), 2.09 (m, 1H), 1.87 (m, 2H), 1.58 (m, 1H), 1.45 (m, 1 H), 1.15 (d, J = 7Hz, 3H), 1.03 ppm (t, J = 7Hz, 3H). ¹³C-NMR (CDCl₃, 100 MHz): δ 202.8, 153.6, 127.9, 38.6, 35.1, 34.9, 26.7, 15.6, 12.0 ppm. MS (El): m/z = 27 (14), 41 (16), 53 (20), 67 (18), 81 (100), 96 (78), 110(9), 138(17, M⁺).

Geruchsbeschreibung von 4-Ethyl-6-methylcyclohex-2-en-1-on: würzig, Kümmel, grün, wässrig, Blüte.

### Beispiel 23: Synthese von 2,4-Diethylcyclohex-2-en-1-on (34)

Synthese analog Beispiel 1.2, ausgehend von Ethyl-3-oxohexanoat und 2-Ethylacrolein. Säulenchromatographische Reinigung an Silicagel (Laufmittel: Cyclohexan/Essigsäureethylester 30/1) ergibt 2,4-Diethylcyclohex-2-en-1-on als Isomerengemisch in Form eines farblosen Öls (15%).

¹H-NMR (CDCl₃, 400 MHz): δ 6.56 (m, 1 H), 2.50 (m, 1 H), 2.33 (m, 1 H), 2.31 (m, 1 H), 2.20 (m, 2H), 2.07 (m, 1 H), 1.62 (m, 1 H), 1.53 (m, 1 H), 1.44 (m, 1 H), 1.01 (t, J = 7Hz, 3H), 0.99 ppm (t, J = 7Hz, 3H). ¹³C-NMR (CDCl₃, 100 MHz): δ 199.8, 148.6, 140.3, 37.9, 37.5, 28.5, 28.0, 22.5, 12.9, 11.4 ppm. MS (El): m/z = 27 (22), 41 (27), 55 (32), 67 (33), 81 (100), 95 (69), 110 (59), 123 (42), 137 (8), 152 (61, M⁺).

Geruchsbeschreibung von 2,4-Diethylcyclohex-2-en-1-on: grün, technisch, würzig, Cumarin.

### Beispiel 24: Synthese von 4-Ethyl-5-methylcyclohex-2-en-1-on (37)

Synthese analog Beispiel 1.2, ausgehend von Ethylacetoacetat und 2-Ethylcrotonaldehyd. Säulenchromatographische Reinigung an Silicagel (Laufmittel: Cyclohexan/Essigsäureethylester 10/1) ergibt 4-Ethyl-5-methylcyclohex-2-en-1-on als Isomerengemisch in Form eines farblosen Öls (10%).

Hauptverbindung: ¹H-NMR (CDCl₃, 400 MHz): δ 6.85 (m, 1 H), 6.02 (m, 1 H), 2.48 (m, 1 H), 2.16 (m, 1 H), 2.08-1.98 (m, 2H), 1.73 (m, 1 H), 1.54 (m, 1 H), 1.06 (d, J = 6Hz, 3H), 0.96 ppm (t, J = 7Hz, 3H). ¹³C-NMR (CDCl₃, 100 MHz): δ 200.2, 154.3, 129.2, 45.3, 44.4, 33.4, 24.1, 19.5, 10.5 ppm. MS (El): m/z = 27 (13), 41 (20), 55 (31), 68 (46), 81 (100), 96 (31), 109 (15), 123 (6), 138 (17, M⁺).

Nebenverbindung: ¹H-NMR (CDCl₃, 400 MHz): δ 6.83 (m, 1 H), 5.99 (m, 1 H), 2.42-2.30 (m, 4H), 1.58-1.46 (m, 2H), 1.02 (t, J = 7Hz, 3H), 0.94 ppm (d, J = 7Hz, 3H). ¹³C-NMR (CDCl₃, 100 MHz): δ 199.9, 153.5, 128.7, 44.6, 418, 32.3, 23.3, 14.7, 11.9 ppm. MS (El): m/z = 27 (11), 41 (18), 55 (27), 68 (30), 81 (100), 96 (36), 109 (13), 123 (4), 138(15, M⁺).

Geruchsbeschreibung von 4-Ethyl-5-methylcyclohex-2-en-1-on: Mandel, fruchtig, Kirsche, Zimt, balsamisch.

### Beispiel 25: Synthese von 3,6-Diethylcyclohex-2-en-1-on (40)

Synthese analog Beispiel 1.2, ausgehend von Ethyl-2-ethylacetoacetat und 1-Penten-3-on. Säulenchromatographische Reinigung an Silicagel (Laufmittel: Cyclohexan/Essigsäureethylester 30/1) ergibt 3,6-Diethylcyclohex-2-en-1-on als Isomerengemisch in Form eines farblosen Öls (26%).

¹H-NMR (CDCl₃, 400 MHz): δ 5.83 (m, 1H), 2.32 (m, 2H), 2.22 (m, 2H), 2.16-2.05 (m, 2H), 1.86 (m, 1 H), 1.73 (m, 1 H), 1.43 (m, 1 H), 1.10 (t, J = 7Hz, 3H), 0.94 ppm (t, J = 8Hz, 3H). ¹³C-NMR (CDCl₃, 100 MHz): δ 202.0, 166.4, 124.3, 47.2, 30.6, 28.9, 27.2, 22.2, 11.5, 11.3 ppm. MS (El): m/z = 27 (11), 41 (16), 53 (16), 67 (34), 81 (40), 96 (100), 109 (14), 124 (52), 137 (7), 152 (21, M⁺).

Geruchsbeschreibung von 3,6-Diethylcyclohex-2-en-1-on: grün, Minze, Tabak, Liebstöckel.

### Beispiel 26: Synthese von 2,3-Diethylcyclohex-2-en-1-on (41)

Synthese analog Beispiel 1.2, ausgehend von Ethyl-3-oxohexanoat und 1-Penten-3-on. Säulenchromatographische Reinigung an Silicagel (Laufmittel: Cyclohexan/Essigsäureethylester 30/1) ergibt 2,3-Diethylcyclohex-2-en-1-on als Isomerengemisch in Form eines farblosen Öls (15%).

¹H-NMR (CDCl₃, 400 MHz): δ 2.37 (m, 2H), 2.33 (m, 2H), 2.29 (m, 2H), 2.27 (m, 2H), 1.92 (m, 2H), 1.10 (t, J = 8Hz, 3H), 0.94 ppm (t, J = 8Hz, 3H). ¹³C-NMR (CDCl₃, 100 MHz): δ 199.2, 160.0, 136.5, 38.1, 30.1, 27.7, 22.6, 18.2, 14.2, 12.5 ppm. MS (El): m/z = 27 (13), 41 (36), 55 (26), 67 (40), 81 (100), 95 (46), 109 (37), 124 (78), 137 (21), 152 (77, M⁺).

Geruchsbeschreibung von 2,3-Diethylcyclohex-2-en-1-on: Tabak, Safran, fruchtig, Apfel, Damascon.

### Beispiel 27: Synthese von 4-Ethyl-2,5-dimethylcyclohex-2-en-1-on (42)

Synthese analog Beispiel 1.2, ausgehend von Methyl-3-oxovalerat und 2-Ethylcrotonaldehyd. Säulenchromatographische Reinigung an Silicagel (Laufmittel: Cyclohexan/Essigsäureethylester 5/1) ergibt 4-Ethyl-2,5-dimethylcyclohex-2-en-1-on als Isomerengemisch in Form eines farblosen Öls (34%).

Hauptverbindung: ¹H-NMR (CDCl₃, 400 MHz): δ 6.59 (m, 1 H), 2.48 (m, 1 H), 2.14 (m, 1 H), 2.02 (m, 1H), 1.96 (m, 1H), 1.78 (m, 3H), 1.69 (m, 1H), 1.48 (m, 1 H), 1.04 (d, J = 6Hz, 3H), 0.95 ppm (t, J = 7Hz, 3H). ¹³C-NMR (CDCl₃, 100 MHz): δ 200.3, 149.4, 135.1, 45.6, 44.7, 33.9, 24.6, 19.4, 15.8, 10.5 ppm. MS (El): m/z = 27 (7), 41 (31), 55 (14), 69 (55), 81 (17), 95 (100), 110 (20), 123 (18), 137 (6), 152 (9, M⁺).

Nebenverbindung: ¹H-NMR (CDCl₃, 400 MHz): δ 6.56 (m, 1H), 2.37 (m, 4H), 1.78 (m, 3H), 1.47 (m, 2H), 1.00 (d, J = 7Hz, 3H), 0.90 ppm (t, J = 7Hz, 3H). ¹³C-NMR (CDCl₃, 100 MHz): δ 200.0, 148.4, 134.5, 44.8, 42.0, 32.6, 23.7, 15.8, 14.5, 11.9 ppm. MS (El): m/z = 27 (7), 41 (30), 55 (13), 69 (50), 81 (18), 95 (100), 110 (21), 123 (17), 137 (6), 152 (7, M⁺).

Geruchsbeschreibung von 4-Ethyl-2,5-dimethylcyclohex-2-en-1-on: würzig, aromatisch, camphrig, floral, animalisch.

### Beispiel 28: Synthese von 3-Ethyl-5-isopropyl-2-methylcyclohex-2-en-1-on (43)

Zu einer Lösung von Methyl-3-oxovalerat (215 g, 1.36 mol) und Isobutylaldehyd (61.2 g, 0.85 mol) wird Piperidin (7.4 g) in Ethanol (20 mL) bei 0-5°C innerhalb von 20 Minuten getropft. Die Lösung wird über Nacht bei Raumtemperatur gerührt und 2.5 Stunden am Rückfluss erhitzt. Danach wird die Reaktionslösung mit Ethanol (600 mL), Wasser (350 mL) und NaOH (56 g) 8 Stunden am Rückfluss erhitzt und das Lösungsmittel abdestilliert. Bei Raumtemperatur werden Wasser (500 mL) und MTBE (250 mL) zusetzt und die wässrige Phase abgetrennt. Die organische Phase wird 2x mit 5%iger Schwefelsäure und Sodalösung neutral gewaschen und eingeengt. Fraktionierende Destillation ergibt 3-Ethyl-5-isopropyl-2-methylcyclohex-2-en-1-on in Form eines farblosen Öls (156.3 g, 64%).

¹H-NMR (CDCl₃, 400 MHz): δ 2.50 (ddd, J = 16, 4,2 Hz, 1H), 2.37-2.19 (m, 1H), 2.23 (m, 3H), 2.19-2.08 (m, 1H), 2.06 (dd, J = 16, 14Hz, 1 H), 1.76 (dd, J = 2, 1 Hz, 3H), 1.83-1.68 (m, 1 H), 1.63-1.49 (m, 1 H), 1.08 (t, J = 8Hz, 3H), 0.92 (d, J = 7Hz, 3H), 0.91 ppm (d, J = 7Hz, 3H). ¹³C-NMR (CDCl₃, 100 MHz): δ 200.4, 160.0, 129.8, 41.4, 40.9, 34.3, 32.0, 28.4, 19.7, 19.4, 11.7, 10.3 ppm. MS (El): m/z = 41 (30), 67 (62), 83 (19), 95 (22), 110 (100), 123 (12), 137 (28), 151 (2), 165 (8), 180 (87, M⁺).

Geruchsbeschreibung von 3-Ethyl-5-isopropyl-2-methylcyclohex-2-en-1-on: stark, Chinolin, Leder, grün, galbanumartig, würzig, Tabak.

Geschmacksbeschreibung von 3-Ethyl-5-isopropyl-2-methylcyclohex-2-en-1-on: chemisch, medizinisch, frisch, holzig, krautig.

### Beispiel 29: Synthese von 3,5-Diethyl-2-methylcyclohex -2-en-1-on (44)

Synthese analog Beispiel 28, ausgehend von Methyl-3-oxovalerat und Propionaldehyd. Fraktionierende Destillation ergibt 3,5-Diethyl-2-methylcyclohex -2-en-1-on in Form eines farblosen Öls (62%)

¹H-NMR (CDCl₃, 400 MHz): δ 2.52 (ddd, J = 16, 4,2 Hz, 1H), 2.41-2.34 (m, 1H), 2.33-2.19 (m, 2H), 2.12-2.07 (m, 1 H), 2.02 (dd, J = 16, 13Hz,1 H), 1.94-1.82 (m, 1 H), 1.76 (dd, J = 2, 2Hz, 3H), 1.46-1.30 (m, 2H), 1.07 (t, J = 8Hz, 3H), 0.91 ppm (t, J = 8Hz, 3H). ¹³C-NMR (CDCl₃, 100 MHz): δ 200.0, 159.6, 130.0, 43.7, 36.6, 36.3, 28.6, 28.3, 11.7, 11.1, 10.2 ppm. MS (El): m/z = 41 (25), 53 (14), 67 (60), 82 (13), 95 (21), 110 (100), 123 (6), 137 (21 ), 151 (10), 166 (84, M⁺).

Geruchsbeschreibung von 3,5-Diethyl-2-methylcyclohex -2-en-1-on: würzig, Leder, Tabak.

### Beispiel 30: Synthese von 3-Ethyl-2-methyl-5-n-propylcyclohex-2-en-1-on (45)

Synthese analog Beispiel 28, ausgehend von Methyl-3-oxovalerat und Butyraldehyd. Fraktionierende Destillation ergibt 3-Ethyl-2-methyl-5-n-propylcyclohex-2-en-1-on in Form eines farblosen Öls (63%)

¹H-NMR (CDCl₃, 400 MHz): δ 2.54-2.48 (m, 1H), 2.41-2.33 (m, 1H), 2.33-2.18 (m, 2H), 2.12-1.91 (m, 3H), 1.76 (t, J = 2Hz, 3H), 1.42-1.27 (m, 4H), 1.07 (t, J = 8Hz, 3H), 0.91 ppm (t, J = 7Hz, 3H). ¹³C-NMR (CDCl₃, 100 MHz): δ 200.01, 159.6, 130.0, 44.0, 38.1, 37.0, 34.3, 28.3, 19.6, 14.1, 11.7, 10.2 ppm. MS (El): m/z = 41 (26), 55 (14), 67 (53), 81 (13), 95 (17), 110 (100), 123 (8), 137 (31), 151 (4), 165 (7), 180 (68, M⁺).

Geruchsbeschreibung von 3-Ethyl-2-methyl-5-n-propylcyclohex-2-en-1-on: würzig, grün, Sellerie, Anis.

### Beispiel 31: Synthese von 2-Ethyl-5-isopropylcyclohex-2-en-1-on (48)

Synthese analog Beispiel 1.2, ausgehend von Ethyl-2-ethylacetoacetat und 4-Methyl-2-pentenal. Säulenchromatographische Reinigung an Silicagel (Laufmittel: Cyclohexan /Essigsäureethylester 30/1) ergibt 2-Ethyl-5-isopropylcyclohex-2-en-1-on als Isomerengemisch in Form eines farblosen Öls (28%).

¹H-NMR (CDCl₃, 400 MHz): δ 6.70 (m, 1H), 2.53 (m, 1H), 2.39 (m, 1H), 2.20 (m, 2H), 2.16-2.04 (m, 2H), 1.83 (m, 1 H), 1.57 (m, 1 H), 1.00 (t, J = 7Hz, 3H), 0.92 ppm (d, J = 7Hz, 6H). ¹³C-NMR (CDCl₃, 100 MHz): δ 200.3, 143.7, 140.9, 42.4, 41.9, 32.1, 29.9, 22.2, 19.6, 19.5, 12.8 ppm. MS (El): m/z = 27 (9), 41 (22), 55 (18), 67 (31), 81 (33), 96 (100), 109 (33), 123 (31), 137 (4), 151 (5), 166 (36, M⁺).

Geruchsbeschreibung von 2-Ethyl-5-isopropylcyclohex-2-en-1-on: fruchtig, Minze, grün, würzig, Salbei.

### Beispiel 32: Synthese von 2-Methyl-5-n-propylcyclohex-2-en-1-on (51)

Synthese analog Beispiel 1.2, ausgehend von Methyl-3-oxovalerat und (E)-2-Hexenal. Fraktionierende Destillation ergibt 2-Methyl-5-n-propylcyclohex-2-en-1-on als Isomerengemisch in Form eines farblosen Öls (30%).

¹H-NMR (CDCl₃, 400 MHz): δ 6.72 (m, 1H), 2.53 (m, 1H), 2.40 (m, 1H), 2.07 (m, 3H), 1.77 (m, 3H), 1.34 (m, 4H), 0.90 ppm (t, J = 7Hz, 3H). ¹³C-NMR (CDCl₃, 100 MHz): δ 200.3, 144.9, 135.5, 44.7, 38.1, 35.4, 32.6, 19.7, 15.8, 14.1 ppm. MS (El): m/z = 27 (7), 41 (17), 54 (26), 69 (17), 82 (100), 95 (7), 109 (20), 123 (4), 137 (1), 152 (20, M⁺).

Geruchsbeschreibung von 2-Methyl-5-n-propylcyclohex-2-en-1-on: würzig, frisch, süß, Anis, Pfeffer.

### Beispiel 33: Synthese von 2-Ethyl-5-n-propylcyclohex-2-en-1-on (52)

Synthese analog Beispiel 1.2, ausgehend von Ethyl-2-ethylacetoacetat und (E)-2-Hexenal. Säulenchromatographische Reinigung an Silicagel (Laufmittel: Cyclohexan/Essigsäureethylester 30/1) ergibt 2-Ethyl-5-n-propylcyclohex-2-en-1-on als Isomerengemisch in Form eines farblosen Öls (41%).

¹H-NMR (CDCl₃, 400 MHz): δ 6.67 (m, 1 H), 2.53 (m, 1 H), 2.43 (m, 1 H), 2.20 (m, 2H), 2.10-2.02 (m, 3H), 1.34 (m, 4H), 1.00 (t, J = 7Hz, 3H), 0.91 ppm (t, J = 7Hz, 3H). ¹³C-NMR (CDCl₃, 100 MHz): δ 199.9, 143.3, 141.0, 45.0, 38.1, 35.3, 32.6, 22.3, 19.7, 14.1, 12.9 ppm. MS (El): m/z = 27 (7), 41 (18), 55 (15), 67 (32), 81 (23), 96 (100), 109 (22), 123 (42), 137 (5), 151 (3), 166 (27, M⁺).

Geruchsbeschreibung von 2-Ethyl-5-n-propylcyclohex-2-en-1-on: Anis, Lakritze, lactonisch, Blüte.

### Beispiel 34: Synthese von 4-Ethyl-5-n-propylcyclohex-2-en-1-on (57)

Synthese analog Beispiel 1.2, ausgehend von Ethylacetoacetat und 2-Ethyl-2-hexenal. Säulenchromatographische Reinigung an Silicagel (Laufmittel: Cyclohexan/Essigsäureethylester 30/1) ergibt 4-Ethyl-5-n-propylcyclohex-2-en-1-on als Isomerengemisch in Form eines farblosen Öls (15%).

Hauptverbindung: ¹H-NMR (CDCl₃, 400 MHz): δ 6.85 (m, 1H), 5.98 (m, 1H), 2.58 (m, 1H), 2.15 (m, 1H), 2.11 (m, 1H), 1.93 (m, 1H), 1.74-1.23 (m, 6H), 0.97 (t, J = 8Hz, 3H), 0.91 ppm (t, J = 8Hz, 3H). ¹³C-NMR (CDCl₃, 100 MHz): δ 200.2, 154.3, 128.9, 42.4, 41.8, 37.6, 35.4, 24.7, 19.5, 14.2, 10.8 ppm. MS (El): m/z = 27 (16), 41 (30), 55 (42), 68 (57), 81 (100), 95 (91), 109 (46), 123 (12), 137 (16), 166 (22, M⁺).

Nebenverbindung: ¹H-NMR (CDCl₃, 400 MHz): δ 7.00 (m, 1H), 5.99 (m, 1 H), 2.38-2.32 (m, 3H), 2.21-2.18 (m, 1H), 1.74-1.23 (m, 6H), 1.02 (t, J = 8Hz, 3H), 0.91 ppm (m, 3H). ¹³C-NMR (CDCl₃, 100 MHz): δ 200.2, 154.8, 128.9, 41.2, 40.7, 37.4, 32.5, 22.1, 20.0, 14.2, 12.1 ppm. MS (El): m/z = 27 (14), 41 (26), 55 (35), 67 (35), 81 (100), 95 (76), 109 (39), 124 (12), 137 (14), 166 (18, M⁺).

Geruchsbeschreibung von 4-Ethyl-5-n-propylcyclohex-2-en-1-on: würzig, Anis, aldehydisch.

### Beispiel 35: Synthese von 4-Ethyl-2-methyl-5-n-propylcyclohex-2-en-1-on (60)

Synthese analog Beispiel 1.2, ausgehend von Ethylacetoacetat und 2-Ethyl-2-hexenal. Säulenchromatographische Reinigung an Silicagel (Laufmittel: Cyclohexan/Essigsäureethylester 30/1) ergibt 4-Ethyl-2-methyl-5-n-propylcyclohex-2-en-1-on als Isomerengemisch in Form eines farblosen Öls (19%).

Hauptverbindung: ¹H-NMR (CDCl₃, 400 MHz): δ 6.58 (m, 1H), 2.59 (m, 1H), 2.13 (m, 2H), 1.88 (m, 1 H), 1.77 (m, 3H), 1.65 (m, 1H), 1.51 (m, 2H), 1.25 (m, 3H), 0.95 (t, J = 7Hz, 3H), 0.90 ppm (t, J = 7Hz, 3H). ¹³C-NMR (CDCl₃, 100 MHz): δ 200.4, 149.4, 134.7, 42.7, 42.1, 38.0, 35.3, 24.8, 19.6, 15.7, 14.2, 10.8 ppm. MS (El): m/z = 27 (7), 41 (36), 55 (22), 69 (52), 81 (18), 95 (89), 109 (100), 123 (28), 137 (12), 151 (12), 180 (5, M⁺).

Nebenverbindung: ¹H-NMR (CDCl₃, 400 MHz): δ 6.74 (m, 1H), 2.39-2.30 (m, 3H), 2.22-2.17 (m, 1 H), 1.77 (m, 3H), 1.55 (m, 1 H), 1.38 (m, 1 H), 1.25 (m, 4H), 1.00 (t, J = 8Hz, 3H), 0.90 ppm (t, J = 7Hz, 3H). ¹³C-NMR (CDCl₃, 100 MHz): δ 200.1, 149.7, 134.7, 41.2, 41.0, 37.8, 32.3, 22.3, 20.0, 15.9, 14.2, 12.1 ppm. MS (El): m/z = 27 (11), 41 (34), 55 (21), 69 (48), 81 (28), 95 (91), 109 (100), 123 (28), 137 (16), 151 (18), 180 (7, M⁺).

Geruchsbeschreibung von 4-Ethyl-2-methyl-5-n-propylcyclohex-2-en-1-on: süß, technisch, Anis.

### Beispiel 36: Synthese von 2,4-Diethyl-5-n-propylcyclohex-2-en-1-on (61)

Synthese analog Beispiel 1.2, ausgehend von Ethylacetoacetat und 2-Ethyl-2-hexenal. Säulenchromatographische Reinigung an Silicagel (Laufmittel: Cyclohexan/Essigsäure-ethylester 30/1) ergibt 2,4-Diethyl-5-n-propylcyclohex-2-en-1-on als Isomerengemisch in Form eines farblosen Öls (21%).

Hauptverbindung: ¹H-NMR (CDCl₃, 400 MHz): δ 6.52 (m, 1H), 2.58 (m, 1H), 2.16-2.09 (m, 2H), 1.87 (m, 1H), 1.66 (m, 1 H), 1.54 (m, 3H), 1.40 (m, 1H), 1.32-1.18 (m, 3H), 1.01 (t, J = 8Hz, 3H), 0.95 (t, J = 8Hz, 3H), 0.90 ppm (t, J = 7Hz, 3H). ¹³C-NMR (CDCl₃, 100 MHz): δ 199.9, 147.8, 140.3, 42.5, 42.4, 37.8, 35.3, 24.9, 22.3, 19.6, 14.2, 13.0, 10.8 ppm. MS (El): m/z = 29 (21), 41 (41), 55 (70), 67 (28), 81 (100), 95 (42), 109 (89), 123 (100), 137 (59), 151 (56), 165 (26), 194 (21, M⁺).

Nebenverbindung: ¹H-NMR (CDCl₃, 400 MHz): δ 6.68 (m, 1H), 2.39-2.32 (m, 3H), 2.24-2.18 (m, 3H), 1.61 (m, 1H), 1.40 (m, 2H), 1.32-1.18 (m, 3H), 1.01 (t, J = 8Hz, 3H), 1.00 (t, J = 7Hz, 3H), 0.90 ppm (t, J = 7Hz, 3H). ¹³C-NMR (CDCl₃, 100 MHz): δ 199.7, 148.1, 140.2, 41.5, 40.8, 37.6, 32.3, 22.4, 22.3, 20.0, 14.2, 13.0, 12.1 ppm. MS (El): m/z = 29 (21), 41 (41), 55 (68), 67 (28), 81 (94), 95 (38), 109 (100), 123 (100), 137 (58), 151 (51), 165 (26), 194 (19, M⁺).

Geruchsbeschreibung von 2,4-Diethyl-5-n-propyl-cyclohex-2-en-1-on: würzig, fruchtig, Anis, floral, fettig.

### Beispiel 37: Parfümöl-Komposition

Das nachfolgend angegebene Parfümöl kann zur Parfümierung diverser kosmetischer Artikel verwendet werden.

| | |
|---|---|
| Agrumex (2-tert-Butylcyclohexylacetat) (Symrise) | 30,0 |
| Agrunitril (2,6-Dimethyl-5-heptenylcyanid) | 1,0 |
| Aldehyd C11 Undecanal 10% DPG (Undec-10-enal) | 2,5 |
| Aldehyd C12 Laurin (Dodecanal) | 1,0 |
| Aldehyd C14 Sog (gamma-Undecalacton) | 4,5 |
| Allylcyclohexylpropionat | 1,0 |
| Allylheptylat | 4,0 |
| Ambrocenide® 0,1% DPG ((4aR,5R,7aS,9R)-Octahydro-2,2,5,8,8,9a-hexamethyl-4H-4a) (Symrise) | 8,0 |
| Anisaldehyd | 6,0 |
| Anisylnitril 1% DPG | 5,0 |
| Benzaldehyd | 2,0 |
| Benzylacetat | 40,0 |
| Benzylsalicylat | 60,0 |
| Cedernblätteröl | 0,5 |
| Cinarol (2-Methyl-3-phenyl-2-propen-1-ol) | 2,0 |
| Citral FF (3,7-Dimethylocta-2,6-dienal) | 1,0 |
| Citronellol 950 (3,7-Dimethyl-6-octen-1-ol) | 20,0 |
| Damascenon Total 10% DPG (2-(2,6,6-Trimethyl-1,3-cyclohexadien-1-yl)-2E-buten-1-on) | 1,0 |
| Damascon alpha 10% DPG ((E)-1-(2,6,6-Trimethyl-2-cyclohexen-1-yl)-2-buten-1-on) | 3,0 |
| Dihydromyrcenol (2,6-Dimethyl-7-octen-2-ol) | 5,0 |
| Dimethylbenzylcarbinylacetat | 4,0 |
| Ebanol 10% DPG (3-Methyl-5-(2,2,3-trimethyl-3-cyclopenten-1-yl)-4-penten-2-ol) (Givaudan) | 3,0 |
| Ethylmethylbutyrat-2 | 1,5 |
| Ethylvanillin (3-Ethoxy-4-hydroxybenzaldehyd) | 0,5 |
| Eugenol (2-Methoxy-4-allyl-phenol) | 3,0 |
| Eugenolacetat (4-Allyl-2-methoxyphenylacetat) | 1,0 |
| Galaxolid 50% IPM (4,6,6,7,8,8-hexamethyl-1,3,4,6,7,8-hexahydrocyclo-penta[g]benzopyran) (International Flavors & Fragrances Inc.) | 40,0 |
| Geraniol 60 (2,6-Dimethyl-trans-2,6-octadien-8-ol) | 30,0 |
| Hexylzimtaldehyd alpha | 120,0 |
| Indoflor® krist. 10% DPG (4,4a,5,9b-Tetrahydroindeno[1,2-d][1,3] dioxin) (Symrise) | 2,0 |
| Iso E Super (2-Acetyl-1,2,3,4,5,6,7,8-octahydro-2,3,8,8-tetramethyl-naphthalin) (International Flavors & Fragrances Inc.) | 20,0 |
| Isoamylsalicylat | 2,0 |
| Isobutylchinolin 1 % DPG | 8,5 |
| Isoraldein 70 ((E)-3-Methyl-4-(2,6,6-trimethyl-cyclohex-2-enyl)-but-3-en-2-on) (Givaudan) | 15,0 |
| Jasmaprunat (Ethyl (2-methyl-1,3-dioxolan-2-yl)acetat) (Symrise) | 1,0 |
| Ligustral (2,4-Dimethyl-3-cyclohexene-1-carboxaldehyd) | 3,0 |
| Lilial® (2-Methyl-3-(4-tert-butylphenyl)propanal) (Givaudan) | 20,0 |
| Linalool (3,7-Dimethyl-1,6-octadien-3-ol) | 32,0 |
| Menzanate 10% DPG | 2,0 |
| Methylanthranilat 10% DPG | 1,0 |
| Methylcapronat 10% DPG | 1,0 |
| Methylheptincarbonat 10% DPG | 3,0 |
| Mysore Acetat (4,7-Methano-1 H-indenemethanol) | 5,0 |
| Orangenöl | 20,0 |
| Oryclon® Spezial (4-tert-Butylcyclohexylacetat) (Symrise) | 60,0 |
| Phenylethylacetat | 6,0 |
| Phenylethyldimethylcarbinol | 10,0 |
| Rosaphen® (beta-Methyl-phenylpentanol) (Symrise) | 10,0 |
| Rosenoxid inactive 10% DPG (Tetrahydro-4-methyl-2-(2-methylpropenyl)-2H-pyran) | 4,0 |
| Styrolylacetat | 5,0 |
| Terpineol rein | 30,0 |
| Terpinylacetat | 5,0 |
| Vanillin (4-Hydroxy-3-methoxybenzaldehyd) | 1,5 |
| Vertomugal 10% DPG (4-(4-Methyl-3-penten-1-yl)-3-cyclohexencarb-aldehyd) | 8,0 |
| Ylang MC Type Base | 10,0 |
| Ysamber® K (1,1,5,5-Tetramethylhexahydro-spiro [1,3-dioxolane-2,8(5H)-2H-2,4a-methanonaphthalene]) (Symrise) | 8,0 |
| Dipropylenglycol (DPG) | 306,0 |

Das hergestellte Parfümöl wurde in zwei gleiche Teile geteilt. Um Verdünnungseffekte auszuschließen, wurde ein erster Teil des Parfümöls mit 0,5 Gew.-% Dipropylenglycol (DPG) versetzt, bezogen auf die Gesamtmenge des ersten Teils des Parfümöls. Der zweite Teil des Parfümöls wurde mit 0,5 Gew.-% 4-lsopropyl-6-methylcyclohex-2-en-1-on aus Beispiel 2 versetzt, bezogen auf die Gesamtmenge des Teils des Parfümöls. Die resultierenden Kompositionen wurden olfaktorisch miteinander verglichen.

Die Komposition umfassend 0,5 Gew.-% an 4-lsopropyl-6-methylcyclohex-2-en-1-on aus Beispiel 2, wurde von Parfümeuren als strahlender und harmonischer empfunden, wobei besonders die floralen Aspekte als hervorgehoben wahrgenommen wurden. Zusätzlich wurde ein angenehmes Frischegefühl empfunden.

### Beispiele 38: Parfümöl-Komposition

Das nachfolgend angegebene Parfümöl kann zur Parfümierung diverser kosmetischer Produkte verwendet werden.

| | |
|---|---|
| Aldehyd C14 Sog (gamma-Undecalacton) | 2,0 |
| Amarocit® (6,6-Dimethoxy-2,5,5-trimethylhex-2-en) (Symrise) | 3,0 |
| Anethol Supra (4-Propenylanisol) | 1,0 |
| Apriconia Base | 10,0 |
| Benzoe Siam Resin 20% BB | 4,0 |
| Benzylacetat | 1,0 |
| Calone 1951 10% DPG (7-Methyl-2*H*-1,5-benzodioxepin-3(4*H*-on) (Danisco Seillans S.A.) | 1,0 |
| Canthoxal [3-(4-Methoxyphenyl)-2-methylpropanal] | 10,0 |
| Citronellol 950 (3,7-Dimethyl-6-octen-1-ol) | 6,0 |
| Cumarin (2H-1-Benzopyran-2-on) | 17,0 |
| Damascenon Total 10% DPG (2-(2,6,6-Trimethyl-1,3-cyclohexadien-1-yl)-2E-buten-1-on) | 6,0 |
| Damascon alpha 10% DPG ((E)-1-(2,6,6-Trimethyl-2-cyclohexen-1-yl)-2-buten-1-on) | 4,0 |
| Decalacton gamma | 2,0 |
| Dihydrojasmon 10% DPG (2-Pentyl-3-methyl-2-cyclopenten-1-on) | 0,5 |
| Ethylenbrassylat (1,4-Dioxacycloheptadecan-5,17-dion) | 100,0 |
| Ethyllinalool [(6E)-3,7-dimethylnona-1,6-dien-3-ol] | 45,0 |
| Ethylmaltol 10% DPG (2-Ethyl-3-hydroxy-4H-pyran-4-on) | 2,0 |
| Ethylvanillin (3-Ethoxy-4-hydroxybenzaldehyd) | 3,0 |
| Florhydral 10% DPG (3-(3-Isopropylphenyl)butanal) (Givaudan) | 4,0 |
| Florosa® (4-Methyl-2-(2-methylpropyl)tetrahydro-2H-4-pyranol) (Givaudan) | 45,0 |
| Geraniol 60 2,6-Dimethyl-trans-2,6-octadien-8-ol | 2,0 |
| Globalide® (Oxacyclohexadec-12-en-2-on) (Symrise) | 60,0 |
| Globanone® (Cyclohexadec-8-en-1-on) (Symrise) | 40,0 |
| Grapefruitöl | 5,0 |
| Hedion® (Methyldihydrojasmonat) (Firmenich) | 77,5 |
| Hexylsalicylat cis-3 | 15,0 |
| Hydroxycitronellal | 15,0 |
| Iso E Super (2-Acetyl-1,2,3,4,5,6,7,8-octahydro-2,3,8,8-tetramethyl-naphthalin) (International Flavors & Fragrances Inc.) | 170,0 |
| Leafovert® 10% DPG (cis-Hex-3-en-1-yl-methylcarbonat) | 7,0 |
| Lilial® (2-Methyl-3-(4-tert-butylphenyl)propanal) (Givaudan) | 50,0 |
| Macrolide® Supra (15-Cyclopentadecanolid) | 60,0 |
| Mandarinenöl | 5,0 |
| Phenylethylalkohol 10% DPG | 2,0 |
| Polysantol (3,3-Dimethyl-5-(2',2',3'-trimethyl-3'-cyclopenten-1'-yl)-4-penten-2-ol) (Firmenich) | 4,0 |
| Prunella Type Base | 10,0 |
| Rosenoxid high cis 1% DPG (Tetrahydro-4-methyl-2-(2-methylpropenyl)-2H-pyran) | 5,0 |
| Sandalore [5-(2,2,3-Trimethyl-3-cyclopentenyl)-3-methylpentan-2-ol] (Givaudan) | 5,0 |
| Synambran® R 50% IMP [3a,6,6,9a-Tetramethyldodecahydronaphtho[2,1-b]furan] (Symrise) | 8,0 |
| Timberol® (1-(2,2,6-Trimethylcyclohexyl)hexan-3-ol) | 3,0 |
| Vanillin (4-Hydroxy-3-methoxybenzaldehyd) | 15,0 |
| DPG | 55,0 |

Das hergestellte Parfümöl wurde in zwei gleiche Teile geteilt. Um Verdünnungseffekte auszuschließen, wurde ein erster Teil des Parfümöls mit 1,0 Gew.-% Dipropylenglycol (DPG) versetzt, bezogen auf die Gesamtmenge des ersten Teils des Parfümöls. Der zweite Teil des Parfümöls wurde mit 1,0 Gew.-% -Ethyl-2,5-dimethylcyclohex-2-en-1-on aus Beispiel 10 versetzt, bezogen auf die Gesamtmenge des Teils des Parfümöls. Die resultierenden Kompositionen wurden olfaktorisch miteinander verglichen.

Der Zusatz von 1,0 Gew.-% an 3-Ethyl-2,5-dimethylcyclohex-2-en-1-on aus Beispiel 10 verleiht speziell den Kopfnoten der Komposition einen belebenden Eindruck. Es wird eine unerwartete Fruchtigkeit in Richtung Pfirsich impliziert. Dadurch wird die Komposition natürlicher und spritziger und somit das Gesamtbild abgerundet.

### Beispiel 39: Parfümöl-Komposition

Das nachfolgend angegebene Parfümöl kann zur Parfümierung diverser kosmetischer Produkte verwendet werden.

| | |
|---|---|
| Aldehyd C11 Undecanal (Undec-10-enal) | 4,0 |
| Aldehyd C12 MNA (2-Methylundecanal) | 6,0 |
| Aldehyd C18 Sog (5-Pentyldihydrofuran-2(3H)-one) | 1,5 |
| Allylamylglycolat | 1,5 |
| Allylcapronat | 0,5 |
| Ambrettolide (Oxacycloheptadec-10-en-2-one) | 2,0 |
| Ambroxid (Dodecahydro-3a,6,6,9a-tetramethyl naphtho(2,1-b)furan) (Symrise) | 1,5 |
| Amylsalicylat | 31,5 |
| Anethol Supra (4-Propenyl-anisol) | 3,0 |
| Anisaldehyd | 24,5 |
| Benzylalkohol | 1,5 |
| Benzylbenzoat | 0,5 |
| Caryophyllen, rekt. 10 % DPG | 8,0 |
| Cumarin (2H-1-Benzopyran-2-on) | 48,0 |
| Damascon alpha ((E)-1-(2,6,6-Trimethyl-2-cyclohexen-1-yl)-2-buten-1-on) | 0,5 |
| Dimethylbenzylcarbinylbutyrat | 6,0 |
| Dipenten | 4,0 |
| Diphenyloxid | 0,5 |
| Eucalyptusöl | 1,5 |
| Frambinon® (4-(4-Methoxyphenyl)butan-2-on) (Symrise) | 1,0 |
| Geraniol Supra 2,6-Dimethyl-trans-2,6-octadien-8-ol | 6,5 |
| Geranylacetat (3,7-Dimethyl-2,6-octadienacetat) | 1,0 |
| Globalide® (Oxacyclohexadec-12-en-2-on) (Symrise) | 14,5 |
| Guajylacetat | 0,5 |
| Hedion® (Methyldihydrojasmonat) (Firmenich) | 51,0 |
| Heliotropin (3,4-(Methylendioxy)-benzaldehyd) | 8,5 |
| Hexylsalicylat | 156,0 |
| lonol (2,6-Di-tert-butyl-4-methylphenol) | 4,0 |
| lonon beta ((E)-4-(2,6,6-trimethyl-1-cyclohexen-1-yl)-3-buten-2-one) | 71,0 |
| Iso E Super (2-Acetyl-1,2,3,4,5,6,7,8-octahydro-2,3,8,8-tetramethyl-naphthalin) (International Flavors & Fragrances Inc.) | 92,0 |
| Isoraldein 70 ((E)-3-Methyl-4-(2,6,6-trimethyl-cyclohex-2-enyl)-but-3-en-2-on) (Givaudan) | 30,0 |
| Lilial® (2-Methyl-3-(4-tert-butylphenyl)propanal) (Givaudan) | 92,0 |
| Linalool (3,7-Dimethyl-1,6-octadien-3-ol) | 69,0 |
| Linalylacetat (1,5-Dimethyl-1-vinyl-4-hexenylacetat) | 4,5 |
| Nerol (2,6-Dimethyl-2,6-octadien-8-ol) | 2,5 |
| Ocimen | 0,5 |
| Orangenöl | 68,0 |
| Oryclon® Spezial (4-tert-Butylcyclohexylacetat) (Symrise) | 119,0 |
| Patchouliöl | 9,5 |
| Polysantol (3,3-Dimethyl-5-(2',2',3'-trimethyl-3'-cyclopenten-1'-yl)-4-penten-2-ol) (Firmenich) | 1,0 |
| Sandranol® ((2E)-2-Ethyl-4-(2,2,3)-trimethylcyclopent-3-en-1-yl) but-2-en-1-ol) (Symrise) | 10,0 |
| Styrolylacetat | 14,5 |
| Terpineol alpha | 14,0 |
| Tetrahydrolinalool (3,7-Dimethyl octan-3-ol) | 0,5 |
| Undecanon-2 10 % DPG | 2,0 |
| Vanillin (4-Hydroxy-3-methoxybenzaldehyd) | 10,5 |

Das hergestellte Parfümöl wurde in zwei gleiche Teile geteilt. Um Verdünnungseffekte auszuschließen, wurde ein erster Teil des Parfümöls mit 2,0 Gew.-% Dipropylenglycol (DPG) versetzt, bezogen auf die Gesamtmenge des ersten Teils des Parfümöls. Der zweite Teil des Parfümöls wurde mit 2,0 Gew.-% 2,3,4,5-Tetramethylcyclohex-2-en-1-on aus Beispiel 15 versetzt, bezogen auf die Gesamtmenge des Teils des Parfümöls. Die resultierenden Kompositionen wurden olfaktorisch miteinander verglichen.

Der Zusatz von 2,0 Gew.-% an 2,3,4,5-Tetramethylcyclohex-2-en-1-on aus Beispiel 15 bewirkt einen unerwarteten frischen floralen Akkord, verbunden mit einer angenehmen Fruchtigkeit. Somit erhält die Komposition mehr Ausstrahlung und Fülle.

### Beispiel 40: Parfümöl-Komposition

Das nachfolgend angegebene Parfümöl kann zur Parfümierung diverser kosmetischer Produkte verwendet werden.

| | |
|---|---|
| Ambrocenide® 0,1% DPG ((4aR,5R,7aS,9R)-Octahydro-2,2,5,8,8,9a-hexamethyl-4H-4a) (Symrise) | 20,0 |
| Ambroxid (Dodecahydro-3a,6,6,9a-tetramethyl naphtho(2,1-b)furan) (Symrise) | 5,0 |
| Aurelione (Cyclohexadec-8-en-1-on) (Symrise) | 120,0 |
| Beifußöl | 10,0 |
| Bergamotte Base | 80,0 |
| Cedramber | 20,0 |
| Cistusöl 10% DPG | 10,0 |
| Citronenöl | 20,0 |
| Cumarin (2H-1-Benzopyran-2-on) | 20,0 |
| Cyclogalbanat® 10% DPG | 5,0 |
| Dihydromyrcenol (2,6-Dimethyl-7-octen-2-ol) | 60,0 |
| Ebanol (3-Methyl-5-(2,2,3-trimethyl-3-cyclopenten-1-yl)-4-penten-2-ol) (Givaudan) | 15,0 |
| Evernyl® (3-Methoxy-5-methylphenol) (Givaudan) | 5,0 |
| Galbanumöl 10% DPG | 15,0 |
| Geraniumbase | 20,0 |
| Globalide® (Oxacyclohexadec-12-en-2-on) (Symrise) | 60,0 |
| Hedion® (Methyldihydrojasmonat) (Firmenich) | 30,0 |
| Hexylzimtaldehyd alpha | 40,0 |
| Iso E Super (2-Acetyl-1,2,3,4,5,6,7,8-octahydro-2,3,8,8-tetramethyl-naphthalin) (International Flavors & Fragrances Inc.) | 150,0 |
| Isoraldein 70 ((E)-3-Methyl-4-(2,6,6-trimethyl-cyclohex-2-enyl)-but-3-en-2-on) (Givaudan) | 20,0 |
| Krauseminzöl 10% DPG | 10,0 |
| Lavandinöl Grosso | 30,0 |
| Lilial® (2-Methyl-3-(4-tert-butylphenyl)propanal) (Givaudan) | 30,0 |
| Linalool (3,7-Dimethyl-1,6-octadien-3-ol) | 20,0 |
| Linalylacetat (1,5-Dimethyl-1-vinyl-4-hexenylacetat) | 30,0 |
| Lyral® 4-(4-Hydroxy-4-methylpentyl)-3-cyclohexencarboxaldehyd | 40,0 |
| Mandarinenöl | 5,0 |
| Nelkenblütenöl | 10,0 |
| Patchouliöl | 79,5 |
| Vanillin (4-Hydroxy-3-methoxybenzaldehyd) | 20,0 |

Das hergestellte Parfümöl wurde in zwei gleiche Teile geteilt. Um Verdünnungseffekte auszuschließen, wurde ein erster Teil des Parfümöls mit 0,5 Gew.-% Dipropylenglycol (DPG) versetzt, bezogen auf die Gesamtmenge des ersten Teils des Parfümöls. Der zweite Teil des Parfümöls wurde mit 0,5 Gew.-% 2,3,5,5-Tetramethylcyclohex-2-en-1-on aus Beispiel 18 versetzt, bezogen auf die Gesamtmenge des Teils des Parfümöls. Die resultierenden Kompositionen wurden olfaktorisch miteinander verglichen.

Der Zusatz von 0,5 Gew.-% an 2,3,5,5-Tetramethylcyclohex-2-en-1-on aus Beispiel 18 verleiht der Komposition einen überraschenden Frischecharakter. Darüber hinaus werden die Kopfnoten, insbesondere die floralen und fruchtigen Aspekte im besonderen Maße verstärkt, so dass die Komposition mehr Ausdruck und Eleganz gewinnt.

### Beispiel 41: Parfümöl-Komposition

Das nachfolgend angegebene Parfümöl kann zur Parfümierung diverser kosmetischer Produkte verwendet werden.

| | |
|---|---|
| Agrumex (2-tert-Butylcyclohexylacetat) (Symrise) | 40,0 |
| Aldehyd C8 Octanal | 10,0 |
| Ambrocenide® 10% DPG ((4aR,5R,7aS,9R)-Octahydro-2,2,5,8,8,9a-hexamethyl-4H-4a) (Symrise) | 5,0 |
| Damascon alpha 1% DPG ((E)-1-(2,6,6-Trimethyl-2-cyclohexen-1-yl)-2-buten-1-on) | 20,0 |
| Dihydromyrcenol (2,6-Dimethyl-7-octen-2-ol) | 80,0 |
| Dimethylbenzylcarbinylacetat | 15,0 |
| Hedion® (Methyldihydrojasmonat) (Firmenich) | 30,0 |
| Herbaflorat (4,7-Methano-3a,4,5,6,7,7a-hexahydro-5-indenylacetat und/oder 4,7-Methano-3a,4,5,6,7,7a-hexahydro-6-indenylacetat) (Symrise) | 80,0 |
| Herbylpropionat | 40,0 |
| Hexylsalicylat | 100,0 |
| Hexylzimtaldehyd alpha | 78,0 |
| Helvetolide® (2-[1-(3,3-Dimethyl-cyclohexyl)ethoxy]-2-methyl-l-propanol-propanoat (Firmenich) | 10,0 |
| Iso E Super (2-Acetyl-1,2,3,4,5,6,7,8-octahydro-2,3,8,8-tetramethyl-naphthalin) (International Flavors & Fragrances Inc.) | 120,0 |
| Isoraldein 70 ((E)-3-Methyl-4-(2,6,6-trimethyl-cyclohex-2-enyl)-but-3-en-2-on) (Givaudan) | 50,0 |
| Majantol® (2,2-Dimethyl-3-(3-methylphenyl)propanol) | 50,0 |
| Nerolione® (1-(3-Methyl-2-benzofuran-1-yl)ethanon] | 5,0 |
| Oryclon® Spezial (4-tert-Butylcyclohexylacetat) (Symrise) | 100,0 |
| Patchouliöl | 50,0 |
| Phenylethylalkohol | 35,0 |
| Phenoxanol (3-Methyl-5-phenylpentan-1-ol) | 20,0 |
| Symrose® | 50,0 |
| Undecavertol | 10,0 |

Das hergestellte Parfümöl wurde in zwei gleiche Teile geteilt. Um Verdünnungseffekte auszuschließen, wurde ein erster Teil des Parfümöls mit 2,0 Gew.-% Dipropylenglycol (DPG) versetzt, bezogen auf die Gesamtmenge des ersten Teils des Parfümöls. Der zweite Teil des Parfümöls wurde mit 2,0 Gew.-% 3-Ethyl-5-isopropyl-2-methylcyclohex-2-en-1-on aus Beispiel 28 versetzt, bezogen auf die Gesamtmenge des Teils des Parfümöls. Die resultierenden Kompositionen wurden olfaktorisch miteinander verglichen.

Nach Zusatz von 2,0 Gew.-% an 3-Ethyl-5-isopropyl-2-methylcyclohex-2-en-1-on aus Beispiel 28 werden insbesondere blumige Noten intensiviert. Die Komposition entfaltet darüber hinaus einen rosigen Akkord und wird insgesamt mit einer frischen Natürlichkeit und anregenden Sinnlichkeit verbunden.

### Beispiel 42: Deo-Spray

| **Ingredienzien** | **Gew.%** |
|---|---|
| Ethylalkohol 96% | 48,75 |
| Deolite (Dimethylphenylpropanol, 1,5-Pentandiol) (Symrise) | 0,25 |
| Propellant 2.7 bar | 50,00 |
| Parfümölkomposition aus Beispiel 38 | 1,00 |

Der Geruch des Deo-Sprays selbst sowie der in der Nähe der Achselhöhle wahrnehmbare Geruch nach Anwendung des selbigen zeigten einen deutlichen fruchtigen Aspekt, wobei der Gesamteindruck strahlend, natürlich und harmonisch empfunden wurde.

### Beispiel 43: Deo-Stick

| **Ingredienzien** | **Gew.%** |
|---|---|
| Natriumstearat | 6,5 |
| Deolite (Dimethylphenylpropanol, 1,5-Pentandiol) (Symrise) | 0,5 |
| Ethylalkohol 96% | 74,3 |
| Glycerin | 10,0 |
| PPG-3 Mystyl Ether | 7,7 |
| Parfümölkomposition aus Beispiel 39 | 1,0 |

### Beispiel 44: Deo-Roll-On mit Alkohol und Symdeo

| **Ingredienzien** | **Gew.-%** |
|---|---|
| Propan-1,2-diol | 7,00 |
| Ethylalkohol 96% | 50,00 |
| Ultrathix P 100 (Acrylsäure/VP Crosspolymer) (ISP Global Technologies) | 0,60 |
| Entionisiertes Wasser | 39,35 |
| Symdeo MPP | 0,50 |
| EDTA-Na4 | 0,05 |
| Natriumhydroxidlösung, 10 % | 0,50 |
| Cremophor CO 40 | 1,00 |
| Parfümölkomposition aus Beispiel 40 | 1,00 |

### Beispiel 45: Shampoo

| **Ingredienzien** | **Gew.-%** |
|---|---|
| Wasser | 71,9 |
| Plantacare PS 10 (Natriumlaurylsulfat, Laurylglucosid) (Cognis) | 20,0 |
| Euperlan PK 771 (Glycoldistearat, Natriumlaurylsulfat, Cocamid MEA, Laureth-10) (Cognis) | 6,0 |
| Konservierungsmittel | 0,5 |
| Natriumchlorid | 1,4 |
| Zitronensäure | 0,1 |
| Parfümölkomposition aus Beispiel 41 | 0,1 |

### Beispiel 46: Duschgel

| **Ingredienzien** | **Gew.-%** |
|---|---|
| Wasser | 77,436 |
| Plantacare PS 10 (Natriumlaurylsulfat, Laurylglucosid) (Cognis) | 20,000 |
| Phenoxyethanol | 0,500 |
| Natriumchlorid | 1,400 |
| Zitronensäure | 0,164 |
| Parfümölkomposition aus Beispiel 38 | 0,500 |

### Beispiel 47: Gesichtscreme O/W

| **Ingredienzien** | **Gew.-%** |
|---|---|
| Phase A | |
| Dracorin 100 S.E.P. (Glycerylstearat, PEG-100 Stearat) (Symrise) | 2,5 |
| Glycerylstearat | 2,0 |
| Cetearylalkohol | 2,0 |
| Dragoxat 89 (Ethylhexylisononanonat) (Symrise) | 3,0 |
| Mineralöl | 3,0 |
| Neutrales Öl (Capryl-/Caprinsäuretriglycerid) | 4,0 |
| Abil 350 (Dimethicone) (Degussa-Goldschmidt) | 0,5 |
| Tocopherylacetat Phase B | 0,3 |
| Entionisiertes Wasser | 77,7 |
| Keltrol RD (Xanthan Gum) (CP-Kelko) | 0,1 |
| Carbopol Ultrez-10 (Carbomer) (BF Goodrich, Belgium) | 0,1 |
| Glycerin | 3,0 |
| Dragocid Liquid (Phenoxyethanol, Methylparaben, Ethylparaben, Butylparaben, Propylparaben, Isobutylparaben) (Symrise) Phase C | 0,8 |
| Natriumhydroxidlösung, 10 % Phase D | 0,2 |
| Dragoderm (Glycerin, Triticum Vulgare Gluten, Wasser) (Symrise) Phase E | 0,5 |
| Parfümölkomposition aus Beispiel 39 | 0,3 |

### Beispiel 48: Anti Aginp Gesichtscreme O/W, LSF 15

| **Ingredienzien** | **Gew.-%** |
|---|---|
| Phase A | |
| Dracorin CE (Glycerylstearatcitrat) (Symrise) | 3,50 |
| Glycerylstearat | 1,50 |
| Cetylalkohol | 2,00 |
| Softisan 100 (gehärtete Kokosglyceride) (Clariant GmbH) | 1,00 |
| Dragoxat 89 (Ethylhexylisononanonat) (Symrise) | 2,00 |
| Kakaobutter | 1,00 |
| Neo Heliopan 357 (Butylmethoxybenzoylmethan) (Symrise) | 1,50 |
| Neo Heliopan 303 (Octocrylene) (Symrise) | 7,50 |
| Neo Heliopan OS (Ethylhexylsalicylat) (Symrise) | 5,00 |
| Tocopherylacetat | 0,50 |
| Xiameter PMX-0245 Cyclosiloxan (Cyclopentasiloxan) (Dow Corning | 1,00 |
| Dinatrium EDTA | 0,10 |
| Dragosantol 100 (Bisabolol) (Symrise) | 0,10 |
| Tocopherol | 0,10 |
| Keltrol CG-T (Xanthan Gum) (CP-Kelko) | 0,25 |
| Carbopol ETD 2050 (Carbomer) (Noveon) | 0,10 |

| Phase B | |
|---|---|
| Entionisiertes Wasser | 62,92 |
| Neo Heliopan AP, neutralisiert mit Triethanolamin (Dinatriumphenyldibenzimidazoltetrasulfonat) (Symrise) | 1,36 |
| Actipone Amla (Amalki) Fruit GW (Glycerin, Wasser, Phyllanthus Emblica Fruchtextrakt) (Symrise) | 1,00 |
| Glycerin | 3,50 |
| Panthenol | 0,50 |
| Konservierungsmittel | 0,80 |
| Triethanolamin | 0,40 |

| Phase C | |
|---|---|
| DragoBetaGlucan (Wasser, Butylenglycol, Glycerin, Haferkornextrakt) (Symrise) | 1,00 |
| DragoCalm (Wasser, Glycerin, Haferkornextrakt) (Symrise) | 0,50 |
| Farbe I | 0,32 |
| Farbe II | 0,25 |
| Parfümölkomposition aus Beispiel 37 | 0,30 |

### Beispiel 49: Body Lotion O/W

| **Ingredienzien** | **Gew.-%** |
|---|---|
| Phase A | |
| Dracorin GOC (Glycerin Oleat Citrat, Capryl-/Caprinsäuretriglycerid) (Symrise) | 1,50 |
| Sheabutter | 2,00 |
| Dracorin CE (Glycerylstearat Citrat) (Cognis) | 1,00 |
| Glycerylstearat | 3,00 |
| Sweet Almond Öl | 7,00 |
| Isopropylmyristat | 3,00 |

| Phase B | |
|---|---|
| Biotive D-(+)-Trehalose (Symrise) | 0,10 |
| Entionisiertes Wasser | 77,85 |
| Amaze" XT (Dehydroxanthan Gum) (National Starch & Chemical) | 0,40 |
| Glycerin | 1,50 |
| Natriumbenzoat | 0,25 |
| Kaliumsorbat | 0,25 |
| Phase C | |
| SymMatrix (Maltodextrin, Brombeerblattextrakt) (Symrise) | 0,30 |
| Symrelief (Bisabolol, Ingwerwurzelextrakt) (Symrise) | 0,10 |
| Extrapone Aloe Vera GW (Wasser, Glycerin, Aloe Vera) (Symrise) | 1,00 |

| Phase D | |
|---|---|
| Zitronensäure | 0,25 |

| Phase E | |
|---|---|
| Parfümölkomposition aus Beispiel 40 | 0,50 |

### Beispiel 50: Hand- und Körpercreme O/W, heiß-kalt-processing

| **Ingredienzien** | **Gew.-%** |
|---|---|
| Phase A | |
| Dracorin GOC (Glycerin Oleat Citrat, Capryl-/Caprinsäuretriglycerid) (Symrise) | 2,00 |
| PCL Solid (Stearylheptanoat, Stearylcaprylat) (Symrise) | 2,50 |
| Cetearylalkohol | 1,50 |
| Glycerylstearat | 1,00 |
| Dragoxat 89 (Ethylhexylisononanonat) (Symrise) | 3,00 |
| PCL Liquid 100 (Cetearylethylhexanoat) (Symrise) | 7,00 |
| Isodragol (Triisononanoin) (Symrise) | 4,00 |
| Dow Corning 345 Fluid (Cyclomethicon) (Dow Corning GmbH) | 0,50 |

| Phase B | |
|---|---|
| Entionisiertes Wasser | 72,10 |
| Carbopol Ultrez 21 (Acrylate/C10-30 Alkylacrylate Crosspolymer) (Noveon) | 0,20 |
| Keltrol CG-T (Xanthan Gum) (CP-Kelko) | 0,10 |
| Glycerin | 3,00 |
| DragoBetaGlucan (Wasser, Butylenglycol, Glycerin, Haferkornextrakt) (Symrise) | 1,50 |
| Kaliumsorbat | 0,10 |
| Dragocid Liquid (Phenoxyethanol, Methylparaben, Ethylparaben, Butylparaben, Propylparaben, Isobutylparaben) (Symrise) | 0,80 |

| Phase C | |
|---|---|
| Natriumhydroxidlösung, 10 % | 0,50 |
| Parfümölkomposition aus Beispiel 38 | 0,20 |

### Beispiel 51: Haarcoloration, Creambase Blond mit Ammoniak

| **Ingredienzien** | **Gew.-%** |
|---|---|
| Phase A | |
| Oxowax (Cetylalkohol, Oleylalkohol, Cetearylalkohol, Stearinsäure) (LCW) | 21,0 |
| Ceteareth-25 | 4,0 |
| Laureth-8 | 10,0 |
| Natriumcetearylsulfat | 1,0 |
| Polyquaternium-6 (LCW) | 4,0 |
| Wasser | 49,3 |

| Phase B | |
|---|---|
| Ammoniak | 10,2 |

| Phase C | |
|---|---|
| Parfümölkomposition aus Beispiel 39 | 0,5 |

### Beispiel 52: Hairconditioner (Rinse)

| **Ingredienzien** | **Gew.-%** |
|---|---|
| Phase A | |
| Cetearylalkohol | 2,5 |
| Glycerylstearat | 3,0 |
| Cutina HR Powder (Gehärtetes Rizinusöl) (Cognis) | 0,5 |
| Genamin KDM-P (Behentrimoniumchloride) (Clariant) | 2,0 |
| PCL-Liquid 100 (Cetearylethylhexanoat) (Symrise) | 0,5 |

| Phase B | |
|---|---|
| Entionisiertes Wasser | 86,7 |
| Konservierungsmittel | 0,8 |

| Phase C | |
|---|---|
| Dragoderm (Glycerin, Triticum Vulgare Gluten, Wasser) (Symrise) | 3,5 |

| Phase D | |
|---|---|
| Parfümölkomposition aus Beispiel 41 | 0,5 |

### Beispiel 53: Hairconditioner (Leave-on)

| **Ingredienzien** | **Gew.-%** |
|---|---|
| Phase A | |
| Cetearylalkohol | 3,50 |
| Cetrimoniumchlorid | 4,00 |
| PEG-15 Kokospolyamine | 3,00 |

| Phase B | |
|---|---|
| Entionisiertes Wasser | 81,40 |
| Extrapone Henna (Wasser, Propylenglycol, Hennablattextrakt, Glucose) (Symrise) | 1,00 |
| Extrapone Jojoba (Wasser, Propylenglycol, PEG-40 gehärtetes Rizinusöl, 1,2-Hexandiol, Caprylglycol, Jojobasamenöl) (Symrise) | 1,50 |
| Aloe Vera Gel Concentrate 10/1 (Aloe Barbadensis Blattsaft)(Symrise) | 0,50 |
| Panthenol | 1,00 |
| Dragocid Liquid (Phenoxyethanol, Methylparaben, Ethylparaben, Butylparaben, Propylparaben, Isobutylparaben) (Symrise) | 0,80 |
| Desamido Collagen | 1,00 |
| Natriumchlorid | 2,00 |

| Phase C | |
|---|---|
| Parfümölkomposition aus Beispiel 39 | 0,30 |

### Beispiel 54: Sonnencreme O/W "wasserfest" mit Breitband UVA Schutz UVA/UVB Balance

| **Ingredienzien** | **Gew.-%** |
|---|---|
| Phase A | |
| Emulsiphos (Kaliumcetylphosphat, gehärte Palmenglyceride) (Symrise) | 3,50 |
| Cetearylalkohol | 1,00 |
| Neo Heliopan HMS (Homosalate) (Symrise) | 5,00 |
| Neo Heliopan 303 (Octocrylene) (Symrise) | 10,00 |
| Neo Heliopan OS (Ethylhexylsalicylat) (Symrise) | 5,00 |
| Neo Heliopan 357 (Butylmethoxybenzoylmethan) (Symrise) | 5,00 |
| Eusolex T-AVO (Titandioxid, Kieselgel) (Merck) | 4,00 |
| Abil Wax 9801 (Cetyldimethicone) (Degussa-Goldschmidt) | 1,00 |
| Silicare Silicone 41 M65 (Stearyldimethicone) (Clariant) | 1,00 |
| SF1550 (Phenyltrimethicone) (GE Bayer Silicones) | 2,00 |
| Isoadipate (Diisopropyladipat) (Symrise) | 3,00 |
| Tocopherylacetat | 0,50 |
| Antaron-Ganex V-216 (PVP/Hexadecen Copolymer) (ISP Europe) | 0,50 |
| Dinatrium EDTA | 0,10 |
| Keltrol CG-T (Xanthan Gum) (CP-Kelko) | 0,50 |

| Phase B | |
|---|---|
| Entionisiertes Wasser | 47,15 |
| Neo Heliopan Hydro (Phenylbenzimidazolsulfonsäure) (Symrise) | 2,00 |
| Neo Heliopan AP (Dinatriumphenyldibenzimidazoltetrasulfonat) (Symrise) | 2,00 |
| Konservierungsmittel | 0,70 |
| Triethanolamin | 2,50 |
| LaraCare A200 (Galactoarabinan) (Rahn AG) | 0,25 |
| Hydrolite 5 (Pentylenglycol) (Symrise) | 3,00 |
| Parfümölkomposition aus Beispiel 37 | 0,30 |

### Beispiel 55: Rasierschaum

| **Ingredienzien** | **Gew.%** |
|---|---|
| Phase A | |
| Entionisiertes Wasser | 77,3 |
| Triethanolamin | 4,0 |

| Phase B | |
|---|---|
| Edenor L2 SM (Stearinsäure, Palmitinsäure) (Cognis) | 5,3 |
| Laureth-23 | 3,0 |
| Stearylalkohol | 0,5 |

| Phase C | |
|---|---|
| Konservierungsmittel | 0,8 |

| Phase D | |
|---|---|
| Natriumlaurylsulfat | 3,0 |
| Parfümölkomposition aus Beispiel 38 | 1,0 |
| Extrapone Seaweed (Wasser, Propylenglycol, Kaliumiodid, Fucus Vesiculosus Extract) (Symrise) | 1,0 |
| Dragosantol (Bisabolol, Farnesol) (Symrise) | 0,1 |

| Phase E | |
|---|---|
| Propan Butan 4,2 Bar | 4,0 |

### Beispiel 56: Enthaarungsmittel

| **Ingredienzien** | **Gew.-%** |
|---|---|
| Phase A | |
| Cetearylalkohol | 10,0 |
| Ceteareth-12 | 2,0 |
| PCL-Liquid (Cetearylethylhexanoat, Isopropylmyristat) (Symrise) | 3,0 |
| Dragosantol (Bisabolol, Farnesol) (Symrise) | 0,1 |
| Edenor L2 SM (Stearinsäure, Palmitinsäure) (Cognis) | 1,0 |

| Phase B | |
|---|---|
| Entionisiertes Wasser | 52,2 |
| Urea | 5,0 |
| Neo Dragocid Powder (Methylparaben, Sorbinsäure, Dehydroessigsäure, Propylparaben) (Symrise) | 0,2 |

| Phase C | |
|---|---|
| Entionisiertes Wasser | 10,0 |
| Calciumthioglycolat | 6,0 |
| Natriumhydroxidlösung, 10 % | 10,0 |

| Phase D | |
|---|---|
| Parfümölkomposition aus Beispiel 39 | 0,5 |

### Beispiel 57: Weichspülkonzentrat

| **Ingredienzien** | **Gew.-%** |
|---|---|
| Rewoquat WE 18 (Di-(Talg Carboxyethyl), Hydroxyethylmethylammoniummethosulfat) (Goldschmidt Rewo GmbH) | 12,0 |
| Parmetol A 26 (Mischung aus 5-Chloro-2-methyl-2H-isothiazol-3-on und 2-Methyl-2H-isothiazol-3-on) (Hoesch Julius) | 0,1 |
| Calciumchloridlösung, 25 % | 0,4 |
| Wasser | 86,9 |
| Parfümölkomposition aus Beispiel 41 | 0,6 |

### Beispiel 58: Waschpulver

| **Ingredienzien** | **Gew.-%** |
|---|---|
| Natriummetasilicat Pentahydrat | 48,0 |
| Natriumhydrogencarbonat | 15,0 |
| Natriumcarbonat Peroxyhydrat | 15,0 |
| TAED/Natriumcarboxymethylcellulose | 5,0 |
| Oxoalkohol C14-15, 8 EO | 3,0 |
| Natriumlaurylsulfat, C12 | 7,0 |
| Trinopal CBS-X (Derivate von 4,4-Distyrylbiphenyl) (Ciba) | 0,5 |
| Protease | 0,4 |
| alpha-Amylase | 0,3 |
| Natriumsulfat | 5,5 |
| Parfümölkomposition aus Beispiel 37 | 0,3 |

### Beispiel 59: Flüssigwaschmittel

| **Ingredienzien** | **Gew.-%** |
|---|---|
| Wasser | 39,7 |
| Dinatriumdistyrylbiphenyldisulfonat | 0,1 |
| Kokosfettsäure | 7,5 |
| Kaliumhydroxidlösung 50 % (Merck) | 4,3 |
| Propan-1,2-diol | 5,0 |
| Trideceth-9 | 12,0 |
| Parmetol A 26 (Mischung aus 5-Chloro-2-methyl-2H-isothiazol-3-on und 2-Methyl-2H-isothiazol-3-on) (Hoesch Julius) | 0,2 |
| Sulfonsäure, C13-17-sec-alkyl, Natriumsalz | 17,0 |
| Triethanolamin | 2,0 |
| Trinatriumcitratdihydrat | 5,0 |
| HOESCH PHOS DET 32 D (Diethylenetriamine, Pentamethylenphosphonsäure, Natriumsalz) (Hoesch Julius) | 3,0 |
| Ethylalkohol 96% | 3,0 |
| Protease | 0,4 |
| alpha-Amylase | 0,3 |
| Parfümölkomposition aus Beispiel 38 | 0,5 |

### Beispiel 60: Geschirrspülmittelkonzentrat

| **Ingredienzien** | **Gew.-%** |
|---|---|
| Natriumlaurylsulfat | 31,0 |
| Propan-1,2-diol | 6,0 |
| Ethylalkohol 96% | 7,0 |
| Kokosglukosid | 6,0 |
| Kokosbetain | 18,0 |
| Wasser | 31,6 |
| Parfümölkomposition aus Beispiel 40 | 0,4 |

### Beispiel 61: Geschirrspülmittel

| **Ingredienzien** | **Gew.-%** |
|---|---|
| Kokosglukosid | 4,0 |
| Natriumlaurylsulfat | 45,0 |
| Kokosbetain | 8,0 |
| Ethylalkohol 96% | 1,0 |
| Wasser | 41,8 |
| Parfümölkomposition aus Beispiel 41 | 0,2 |

### Beispiel 62: Geschirrspültabs

| **Ingredienzien** | **Gew.-%** |
|---|---|
| Natriumsilicat | 35,00 |
| Trinatriumcitratdihydrat | 20,00 |
| Natriumperborate Monohydrat | 7,00 |
| Tetraacetylethylendiamin Natriumcarboxymethylcellulose | 3,00 |
| Protease | 1,00 |
| alpha-Amylase | 0,25 |
| Natriumcarbonat | 27,65 |
| Sokalan PA 30 CL Granulat (BASF) | 2,00 |
| Sokalan CP 45 Granulat (BASF) | 3,00 |
| Plurafac LF 403 (BASF) | 1,00 |
| Parfümölkomposition aus Beispiel 39 | 0,10 |

### Beispiel 63: Bleichmittel

| **Ingredienzien** | **Gew.-%** |
|---|---|
| Decyldimethylaminoxid | 3,34 |
| Natriumlaurylsulfat | 4,22 |
| Natriumhypochlorit 12,5 % | 30,77 |
| Natriumhydroxydlösung, 30 % | 1,50 |
| Wasser | 60,07 |
| Parfümölkomposition aus Beispiel 37 | 0,10 |

### Beispiel 64: Allzweckreiniger

| **Ingredienzien** | **Gew.-%** |
|---|---|
| Parmetol A 26 (Mischung aus 5-Chloro-2-methyl-2H-isothiazol-3-on und 2-Methyl-2H-isothiazol-3-on) (Hoesch Julius) (Hoesch Julius) | 0,1 |
| Trinatriumcitratdihydrat | 3,0 |
| Natriumlaurylsulfat | 30,0 |
| Trideceth-9 | 5,0 |
| Ethylalkohol 96% | 2,0 |
| Wasser | 59,6 |
| Parfümölkomposition aus Beispiel 39 | 0,3 |

### Beispiel 65: Toilettenreiniger

| **Ingredienzien** | **Gew.-%** |
|---|---|
| Wasser | 93,2 |
| Kelzan ASX-T (Biesterfeld Spezialchemie GmbH) | 0,5 |
| Parafinsulfonat, Natriumsalz | 1,0 |
| Zitronensäure | 5,0 |
| Parfümölkomposition aus Beispiel 38 | 0,3 |

### Beispiel 66: Toilettenstein

| **Ingredienzien** | **Gew.-%** |
|---|---|
| C12/15-pareth-20 | 4,0 |
| Cocamid MEA | 6,0 |
| C10-13, ABS-Na, Pulver | 42,0 |
| Natriumsulfat | 42,0 |
| Parfümölkomposition aus Beispiel 41 | 6,0 |

### Beispiel 67: Seife

| **Ingredienzien** | **Gew.-%** |
|---|---|
| Grundseife HTS (Hirtler GmbH) | 95,8 |
| Titandioxid | 1,0 |
| Wasser | 2,0 |
| Perfümölkomposition aus Beispiel 39 | 1,2 |

### Beispiel 68: Raumluftverbesserer in Alginatgel

| **Ingredienzien** | **Gew.-%** |
|---|---|
| Entionisiertes Wasser | 86,8 |
| 1,2-Benzisothiazol-3(2H)-on | 0,2 |
| Genugel Cl-121 (CP KelcoGermany GmbH) | 2,0 |
| Trideceth-11 | 2,0 |
| Propan-2-ol | 4,0 |
| Perfümöl aus Beispiel 41 | 5,0 |

### Beispiel 69: Raumluftverbesserer Aerosol, water based (w/o)

| **Ingredienzien** | **Gew.-%** |
|---|---|
| C12-15 Pareth-3 | 0,25 |
| Deceth-8, Ziegler Alkohol | 1,00 |
| Entionisiertes Wasser | 22,50 |
| Propellant 4,2 bar | 75,00 |
| Perfümöl aus Beispiel 39 | 1,25 |

### Beispiel 70: Raumluftverbesserer flüssig, Pumpspray

| **Ingredienzien** | **Gew.-%** |
|---|---|
| Entionisiertes Wasser | 90,7 |
| Ethylalkohol 96% | 4,0 |
| Empilan KCL 11/90 (Alkohol C12-15 11 EO) (Stockmeier Chemie GmbH & Co. KG) | 3,0 |
| Propan-2-ol | 1,0 |
| Natriumhydrogencarbonat | 0,2 |
| Parmetol A 26 (Mischung aus 5-Chloro-2-methyl-2H-isothiazol-3-on und 2-Methyl-2H-isothiazol-3-on) (Hoesch Julius) | 0,1 |
| Parfümtilkomposition aus Beispiel 40 | 1,0 |

### Beispiel 71: Raumluftverbesserer (pump/wick)

| **Ingredienzien** | **Gew.-%** |
|---|---|
| Entionisiertes Wasser | 67,32 |
| Sequion 40 NA 32 (Polygon Chemie) | 1,25 |
| Triethanolamin | 0,30 |
| Rewoderm (DiNa-Ricinolamid MEA-Sulfosuccinat) (Goldschmidt AG) | 2,33 |
| Tego Sorb Conc. 50 (Alkohol C12-14, ethoxyliert (2-EO) + Zn-Ricinoleat) (Evonik) | 0,50 |
| Ethylalkohol 96% | 20,00 |
| Solubilizer | 3,00 |
| Milchsäure 90% (2-Hydroxypropionsäure) | 0,30 |
| Parfümölkomposition aus Beispiel 38 | 5,00 |

### Beispiel72: Kerzen

| **Ingredienzien** | **Gew.-%** |
|---|---|
| Kerzenwachs | 95,0 |
| Perfümöl aus Beispiel 37 | 5,0 |

### Beispiel 73: Aroma Typ Pfefferminze

| **Ingredienzien** | **Gew.-%** |
|---|---|
| Anethol (4-Propenyl-anisol) | 9,0 |
| L-Menthol (natürlich oder synthetisch) | 35,0 |
| Pfefferminzöl Piperita Typ (natürlich oder rekonstruiert) | 20,0 |
| Pfefferminzöl Arvensis Typ (natürlich oder rekonstruiert) | 30,0 |

Der Zusatz von 6,0 Gew.-% 4-lsopropyl-6-methylcyclohex-2-en-1-on aus Beispiel 2 verleiht der Aromabase eine zusätzliche Süße und bewirkt dadurch eine Harmonisierung der Base.

### Beispiel 74: Aroma Typ Krauseminze

| **Ingredienzien** | **Gew.-%** |
|---|---|
| Anethol (4-Propenyl-anisol) | 9,0 |
| L-Menthol (natürlich oder synthetisch) | 30,0 |
| Pfefferminzöl Piperita Typ (natürlich oder rekonstruiert) | 5,0 |
| Pfefferminzöl Arvensis Typ (natürlich oder rekonstruiert) | 5,0 |
| L-Carvon | 15,0 |
| Krauseminzöl Cardiaca Typ (natürlich oder rekonstruiert) | 15,0 |
| Krauseminzöl Spicata Typ (natürlich oder rekonstruiert) | 15,0 |

Der Zusatz von 6,0 Gew.-% 2,3,4,5-Tetramethylcyclohex-2-en-1-on aus Beispiel 15 verbessert deutlich das sensorische Profil der Basis verbunden mit einer angenehmen Frische.

### Beispiel 75: Aroma Typ Wintergrün

| **Ingredienzien** | **Gew.-%** |
|---|---|
| Anethol (4-Propenyl-anisol) | 9,0 |
| L-Menthol (natürlich oder synthetisch) | 45,0 |
| Pfefferminzöl Piperita Typ (natürlich oder rekonstruiert) | 2,0 |
| Pfefferminzöl Arvensis Typ (natürlich oder rekonstruiert) | 3,0 |
| Krauseminzöl Spicata Typ (natürlich oder rekonstruiert) | 1,0 |
| Eugenol (2-Methoxy-4-allyl-phenol) | 7,0 |
| Eukalyptol | 5,0 |
| Methylsalicylat | 20,0 |

Der Zusatz von 8,0 Gew.-% 3-Ethyl-2,5-dimethylcyclohex-2-en-1-on aus Beispiel 10 zur Aromabase bewirkt einen deutlichen Boosteffekt der minzigen Noten und vermittelt ein verstärktes Frischegefühl.

### Beispiel 76: Aroma Typ Eucalyptus

| **Ingredienzien** | **Gew.-%** |
|---|---|
| Anethol (4-Propenyl-anisol) | 18,0 |
| L-Menthol (natürlich oder synthetisch) | 50,0 |
| Pfefferminzöl Piperita Typ (natürlich oder rekonstruiert) | 2,0 |
| Pfefferminzöl Arvensis Typ (natürlich oder rekonstruiert) | 3,0 |
| Eukalyptol | 15,0 |
| Eukalyptusöl | 5,0 |

Der Zusatz von 7,0 Gew.-% an 2,3,5,5-Tetramethylcyclohex-2-en-1-on aus Beispiel 18 Aromabase bewirkt eine angenehme Verstärkung der krautigen Noten und vermittelt ein erfrischendes Mundgefühl.

### Beispiel 77: Aroma Typ Zimt

| **Ingredienzien** | **Gew.-%** |
|---|---|
| Zimtaldehyd | 10,0 |
| Anethol (4-Propenyl-anisol) | 9,0 |
| Pfefferminzöl Piperita Typ (natürlich oder rekonstruiert) | 10,0 |
| Pfefferminzöl Arvensis Typ (natürlich oder rekonstruiert) | 15,0 |
| Krauseminzöl Spicata Typ (natürlich oder rekonstruiert) | 8,0 |
| Eugenol (2-Methoxy-4-allyl-phenol) | 2,0 |
| L-Menthol (natürlich oder synthetisch) | 40,0 |

Der Zusatz von 6,0 Gew.-% an 3-Ethyl-5-isopropyl-2-methylcyclohex-2-en-1-on aus Beispiel 28 verleiht der Aromabase einen leichten Schärfeindruck und verstärkt darüber hinaus die Süße, so dass die Base als wesentlich abgerundeter und frischer empfunden wird.

### Beispiel 78: Aroma Typ Eisbonbon

| **Ingredienzien** | **Gew.-%** |
|---|---|
| Isoamylacetat | 2,0 |
| Ethylbutyrat | 0,5 |
| Ethylvanillin (3-Ethoxy-4-hydroxybenzaldehyd) | 2,0 |
| Frambinon™ [4-(4-Hydroxyphenyl)-2-butanon] | 0,5 |
| L-Menthol (natürlich) | 8,0 |
| 1,2-Propylenglycol | 83,0 |
| Verbindung aus Beispiel 15 | 4,0 |

### Beispiel 79: Zahnpasta Silicabase

| **Ingredienzien** | **Gew.-%** |
|---|---|
| Entionisiertes Wasser | 26,53 |
| Sorbitol 70% | 45,00 |
| Solbrol M (Natriumsalz) | 0,15 |
| Trinatriumphosphat | 0,10 |
| Saccharin | 0,20 |
| Natriummonofluorphosphat | 1,12 |
| PEG 1500 | 5,00 |
| Sident 9 (Abrasive Silica) | 10,00 |
| Sident 22 S (Dickende Silica) | 8,00 |
| Natriumcarboxymethylcellulose | 0,90 |
| Titan (IV) oxid | 0,50 |
| Natriumlaurylsulfat | 1,50 |
| Aroma aus Beispiel 73 | 1,00 |

### Beispiel 80: Zahnpasta Phosphatbase

| **Ingredienzien** | **Gew.-%** |
|---|---|
| Entionisiertes Wasser | 36,39 |
| Glycerin | 20,00 |
| Solbrol M (Natriumsalz) | 0,15 |
| Natriummonofluorphosphat | 0,76 |
| Saccharin | 0,20 |
| Dicalciumphosphat-Dihydrat | 36,00 |
| Aerosil® 200 (Silica) | 3,00 |
| Natriumcarboxymethylcellulose | 1,20 |
| Natriumlaurylsulfat (Texapon) | 1,30 |
| Aroma aus Beispiel 75 | 1,00 |

### Beispiel 81: Zahnpasta Calciumcarbonatbase

| **Ingredienzien** | **Gew.-%** |
|---|---|
| Entionisiertes Wasser | 27,5 |
| Saccharin | 0,2 |
| Solbrol M (Natriumsalz) | 0,2 |
| Natriummonofluorphosphat | 0,8 |
| Sorbitol 70% | 29,0 |
| Calciumcarbonat | 35,0 |
| Sident 22 S (Verdickende Silica) | 2,5 |
| Natriumcarboxymethylcellulose | 1,3 |
| Titan (IV) oxid | 0,5 |
| Natriumlaurylsulfat (SLS) | 2,0 |
| Aroma aus Beispiel 74 | 1,00 |

### Beispiel82: Mundwasserkonzentrat

| **Ingredienzien** | **Gew.-%** |
|---|---|
| Ethylalkohol 96% | 42,00 |
| Cremophor RH 455 | 5,00 |
| Entionisiertes Wasser | 48,67 |
| Allantoin | 0,20 |
| Natriumsaccharin 450 | 0,10 |
| Colour L-Blue 5000 (1% in Wasser) | 0,03 |
| Aroma aus Beispiel 75 | 4,00 |

### Beispiel 83: Mundwasser ("ready to use" mit und ohne Alkohol)

| **Ingredienzien** | **I Gew.-%** | **II Gew.%** |
|---|---|---|
| Ethylalkohol 96% | 10,00 | - |
| Cremophor CO 40 | 1,00 | - |
| Cremophor RH 455 | - | 1,80 |
| Benzoesäure | 0,10 | - |
| Entionisiertes Wasser | 83,48 | 87,57 |
| Sorbitol 70% | 5,00 | 10,00 |
| Natriumsaccharin 450 | 0,07 | - |
| Colour L-Blue 5000 (1% in Wasser) | 0,10 | - |
| Natriumfluorid | - | 0,18 |
| Solbrol M Natriumsalz | - | 0,10 |
| Pellitorin-Lösung PLM (enthaltend 10% Pellitorin) | - | 0,15 |
| Aroma aus Beispiel 76 | 0,25 | 0,20 |

### Beispiel 84: Kaugummi mit und ohne Zucker

| **Ingredienzien** | **I Gew.-%** | **II Gew.-%** |
|---|---|---|
| Gum Base (Kaugummibase) | 21,0 | 30,0 |
| Glucosesirup | 16,5 | - |
| Glycerin | 0,5 | - |
| Zucker gepulvert | 60,0 | - |
| Sorbit gepulvert | - | 40, |
| Isomalt gepulvert | - | 9,5 |
| Xylitol | - | 2,0 |
| Mannit D | - | 3,0 |
| Aspartam | - | 0,1 |
| Acesulfam K | - | 0,1 |
| Emulgum™ (Soja-Lecithine, hoher Gehalt an Phospholipiden) | - | 0,3 |
| Sorbitol (70% in Wasser) | - | 13,0 |
| Glycerin | 0,5 | 1,0 |
| Aroma aus Beispiel 77 | 2,0 | 1,0 |

### Beispiel 85: Bonbon (Hardboiled Candy) mit und ohne Zucker

| **Ingredienzien** | **I Gew.-%** | **II Gew.-%** |
|---|---|---|
| Wasser | 2,75 | 2,24 |
| Zucker | 60,1 | - |
| Glucosesirup | 36,9 | - |
| Isomalt | - | 94,98 |
| Xylitol | - | 2,40 |
| Sucralose | - | 0,03 |
| Acesulfam K | - | 0,05 |
| Zitronensäure | - | 0,05 |
| Aroma aus Beispiel 78 | 0,25 | 0,25 |

### Beispiel 86: Instant Getränkepulver

| **Ingredienzien** | **Gew.-%** |
|---|---|
| Zucker (Saccharose) | 81,5 |
| Citronensäure | 11,58 |
| Trinatriumcitrat | 0,70 |
| Tricalciumphosphat | 0,60 |
| Vitamin C | 0,66 |
| Grindsted® JU 543 Stabilizer System (Danisco) | 0,90 |
| Saccharin | 0,56 |
| Citronenaroma, sprühgetrocknet | 1,75 |
| Aroma aus Beispiel 77, sprühgetrocknet auf Maltodextrin (DE 18), Dextrose und Gummi Arabicum, Aromabeladung 35 % | 1,75 |

### Beispiel 87: Geruchsverstärkerkonzentrat

Das nachfolgend angegebene Geruchsverstärkerkonzentrat kann dazu verwendet werden, Basis-Zubereitungen zugesetzt zu werden, um den Geruchseindruck der Basis-Zubereitung überadditiv zu verstärken, beispielsweise unter Ausbildung einer besonders wertvollen Riech- und/oder Geschmacksstoffkomposition.

| **Ingredienzien** | **Gew.%** |
|---|---|
| Rosenoxid | 40 |
| 2,3,5,5-Tetramethylcyclohex-2-en-1-on aus Beispiel 18 | 60 |

### Beispiel 88: Geruchsverstärkerkonzentrat

Das nachfolgend angegebene Geruchsverstärkerkonzentrat kann dazu verwendet werden, Basis-Zubereitungen zugesetzt zu werden, um den Geruchseindruck der Basis-Zubereitung überadditiv zu verstärken, beispielsweise unter Ausbildung einer besonders wertvollen Riech- und/oder Geschmacksstoffkomposition.

| **Ingredienzien** | **Gew.-%** |
|---|---|
| Rosenoxid | 10 |
| 2,3,5,5-Tetramethylcyclohex-2-en-1-on aus Beispiel 18 | 50 |
| 4-Isopropyl-6-methylcyclohex-2-en-1-on aus Beispiel 2 | 40 |

## Patentansprüche

1. Verwendung
- einer Verbindung der Formel (I) 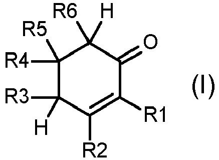 oder
- einer Mischung bestehend aus oder umfassend zwei, drei, vier, fünf, sechs oder mehr verschiedenen Verbindungen der Formel (I),
wobei in jeder der Verbindungen der Formel (I) die Reste R1, R2, R3, R4, R5 und R6 jeweils unabhängig voneinander die folgende Bedeutung haben
R1 bedeutet Wasserstoff, Methyl oder Ethyl,
R2 bedeutet Wasserstoff, Methyl oder Ethyl,
R3 bedeutet Wasserstoff, Methyl, Ethyl oder Isopropyl,
R4 bedeutet Wasserstoff, Methyl, Ethyl, n-Propyl oder Isopropyl,
R5 bedeutet Wasserstoff oder Methyl,
R6 bedeutet Wasserstoff, Methyl oder Ethyl,
mit der Maßgabe, dass R1 nicht Methyl oder Ethyl ist, wenn R6 Methyl oder Ethyl ist zum überadditiven Verstärken eines Geruchseindrucks.

2. Verwendung einer einzelnen Verbindung oder einer Mischung nach Anspruch 1, wobei die einzelne Verbindung der Formel (I) bzw. eine, mehrere oder sämtliche Verbindungen der Formel (I) ausgewählt ist bzw. jeweils unabhängig voneinander ausgewählt sind aus der Gruppe bestehend aus den folgenden Verbindungen (1) bis (61), vorzugsweise ausgewählt aus der Gruppe bestehend aus den Verbindungen (2), (4), (13), (23), (29), (43) und (51):

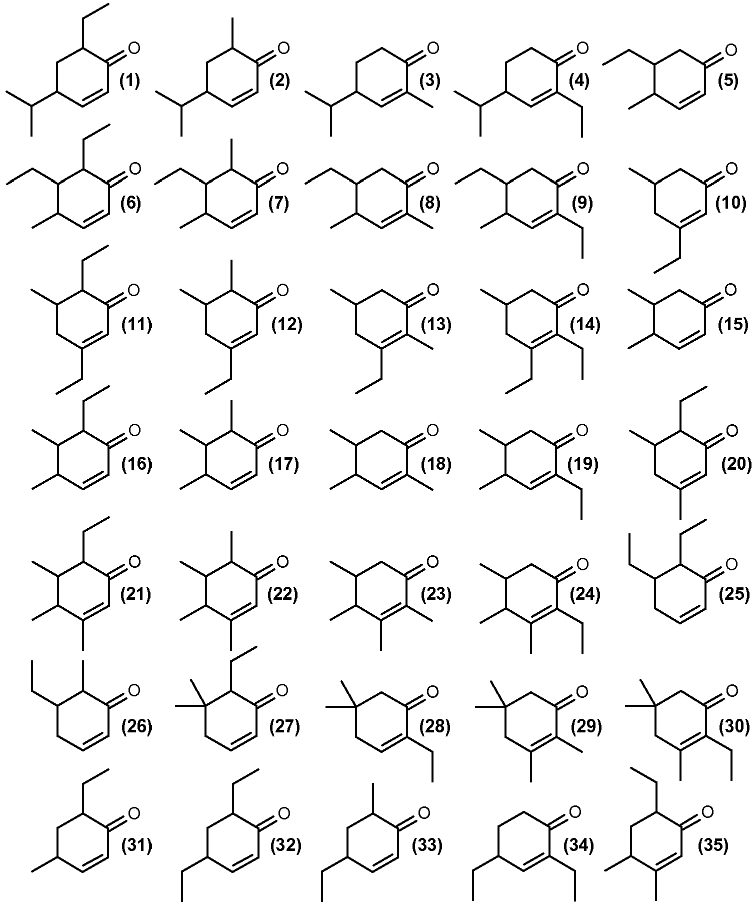

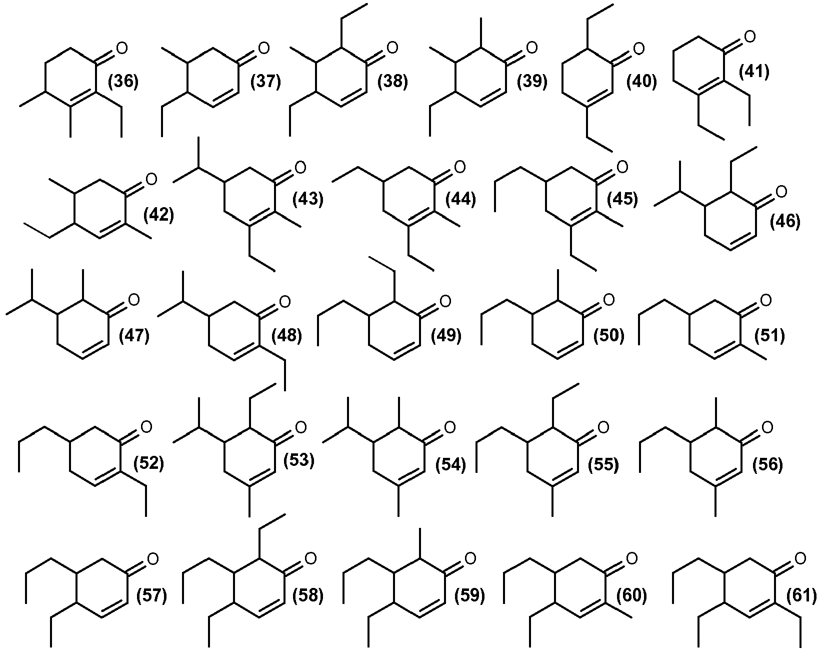

3. Verwendung nach Anspruch 1 oder 2 zum überadditiven Verstärken eines Geruchseindrucks ausgewählt aus der Gruppe bestehend aus floralen und/oder fruchtigen Geruchseindrücken.

4. Verwendung nach einem der einem der vorangehenden Ansprüche, wobei der zu verstärkende Geruchseindruck nicht ausgewählt ist aus der Gruppe bestehend aus nussig, haselnussig, pistazieartig, kakaoartig, karamellig, fleischig, röstgetreideartig, schokoladig und gewürzartig und/oder wobei die Verbindung der Formel (I) oder die entsprechende Mischung keinen Geruchseindruck vermittelt, der ausgewählt ist aus der Gruppe bestehend aus nussig, haselnussig, pistazieartig, kakaoartig, karamellig, fleischig, röstgetreideartig, schokoladig und gewürzartig.

5. (A) Verbindung der Formel (I) 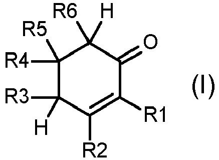 wie in einem der vorangehenden Ansprüche definiert oder
(B) Mischung bestehend aus oder umfassend zwei, drei, vier, fünf, sechs oder mehr verschiedene Verbindungen der Formel (I) wie in einem der vorangehenden Ansprüche definiert,
wobei (A) die Verbindung der Formel (I) bzw. (B) eine, mehrere oder sämtliche Verbindungen der Formel (I) in der Mischung ausgewählt ist bzw. jeweils unabhängig voneinander ausgewählt sind aus der Gruppe bestehend aus den folgenden Verbindungen: 6-Ethyl-4-isopropylcyclohex-2-en-1-on (1), 5,6-Diethyl-4-methylcyclohex-2-en-1-on (6), 5-Ethyl-4,6-dimethylcyclohex-2-en-1-on (7), 2,5-Diethyl-4-methylcyclohex-2-en-1-on (9), 3-Ethyl-5-methylcyclohex-2-en-1-on (10), 3,6-Diethyl-5-methylcyclohex-2-en-1-on (11), 3-Ethyl-5,6-dimethylcyclohex-2-en-1-on (12), 2,3-Diethyl-5-methylcyclohex-2-en-1-on (14), 6-Ethyl-4,5-dimethylcyclohex-2-en-1-on (16), 4,5,6-Trimethylcyclohex-2-en-1-on (17), 2,4,5-Trimethylcyclohex-2-en-1-on (18), 2-Ethyl-4,5-dimethylcyclohex-2-en-1-on (19), 6-Ethyl-3,5-dimethylcyclohex-2-en-1-on (20), 6-Ethyl-3,4,5-trimethylcyclohex-2-en-1-on (21), 3,4,5,6-Tetramethylcyclohex-2-en-1-on (22), 2,3,4,5-Tetramethylcyclohex-2-en-1-on (23), 2-Ethyl-3,4,5-trimethylcyclohex-2-en-1-on (24), 5,6-Diethylcyclohex-2-en-1-on (25), 5-Ethyl-6-methylcyclohex-2-en-1-on (26), 6-Ethyl-5,5-dimethylcyclohex-2-en-1-on (27), 2-Ethyl-5,5-dimethylcyclohex-2-en-1-on (28), 6-Ethyl-4-methylcyclohex-2-en-1-on (31), 4,6-Diethylcyclohex-2-en-1-on (32), 4-Ethyl-6-methylcyclohex-2-en-1-on (33), 2,4-Diethylcyclohex-2-en-1-on (34), 6-Ethyl-3,4-dimethylcyclohex-2-en-1-on (35), 2-Ethyl-3,4-dimethylcyclohex-2-en-1-on (36), 4-Ethyl-5-methylcyclohex-2-en-1-on (37), 4,6-Diethyl-5-methylcyclohex-2-en-1-on (38), 4-Ethyl-5,6-dimethylcyclohex-2-en-1-on (39), 3,6-Diethylcyclohex-2-en-1-on (40), 2,3-Diethylcyclohex-2-en-1-on (41), 4-Ethyl-2,5-dimethylcyclohex-2-en-1-on (42), 3-Ethyl-5-isopropyl-2-methylcyclohex-2-en-1-on (43), 3,5-Diethyl-2-methylcyclohex-2-en-1-on (44), 3-Ethyl-2-methyl-5-n-propyl-cyclohex-2-en-1-on (45), 6-Ethyl-5-isopropylcyclohex-2-en-1-on (46), 5-Isopropyl-6-methylcyclohex-2-en-1-on (47), 2-Ethyl-5-isopropylcyclohex-2-en-1-on (48), 6-Ethyl-5-n-propylcyclohex-2-en-1-on (49), 6-Methyl-5-n-propylcyclohex-2-en-1-on (50), 2-Methyl-5-n-propylcyclohex-2-en-1-on (51), 2-Ethyl-5-n-propylcyclohex-2-en-1-on (52), 6-Ethyl-5-isopropyl-3-methylcyclohex-2-en-1-on (53), 5-Isopropyl-3,6-dimethylcyclohex-2-en-1-on (54), 6-Ethyl-3-methyl-5-n-propylcyclohex-2-en-1-on (55), 3,6-Dimethyl-5-n-propylcyclohex-2-en-1-on (56), 4-Ethyl-5-n-propylcyclohex-2-en-1-on (57), 4,6-Diethyl-5-n-propylcyclohex-2-en-1-on (58), 4-Ethyl-6-methyl-5-n-propylcyclohex-2-en-1-on (59), 4-Ethyl-2-methyl-5-n-propylcyclohex-2-en-1-on (60) und 2,4-Diethyl-5-n-propylcyclohex-2-en-1-on (61),
vorzugsweise ausgewählt sind aus der Gruppe bestehend aus
2,3,4,5-Tetramethylcyclohex-2-en-1-on (23), 3-Ethyl-5-isopropyl-2-methylcyclohex-2-en-1-on (43) und 2-Methyl-5-n-propylcyclohex-2-en-1-on (51).

6. Riech- und/oder Geschmacksstoffkomposition, umfassend oder bestehend aus
(a) einer Verbindung der Formel (I) oder einer Mischung aus zwei, drei, vier, fünf, sechs oder mehr verschiedenen Verbindungen der Formel (I), wie jeweils in einem der vorangehenden Ansprüche definiert, vorzugsweise wie in Anspruch 5 definiert,
und
(b) einem, zwei, drei oder mehr weiteren Riech- und/oder Geschmacksstoffen, wobei der bzw. die weiteren Riech- und/oder Geschmacksstoffe keine Verbindungen der Formel (I) sind,
wobei der bzw. ein, zwei, drei, mehr oder sämtliche der weiteren Riech- und/oder Geschmacksstoffe gemäß Bestandteil (b) einen floralen und/oder einen fruchtigen Geruch aufweisen.

7. Riech- und/oder Geschmacksstoffkomposition nach Anspruch 6, wobei der bzw. ein, zwei, drei, mehr oder sämtliche der weiteren Riech- und/oder Geschmacksstoffe gemäß Bestandteil (b)
- entweder keinen Geruchseindruck vermitteln, der ausgewählt ist aus der Gruppe bestehend aus nussig, haselnussig, pistazieartig, kakaoartig, karamellig, fleischig, röstgetreideartig, schokoladig und gewürzartig, oder einen Geruchseindruck vermitteln, der ausgewählt ist aus der Gruppe bestehend aus nussig, haselnussig, pistazieartig, kakaoartig, karamellig, fleischig, röstgetreideartig, schokoladig und gewürzartig, wobei dieser Geruchseindruck nicht überadditiv verstärkt wird
und/oder
wobei die Verbindung der Formel (I) oder die entsprechende Mischung keinen Geruchseindruck vermittelt, der ausgewählt ist aus der Gruppe bestehend aus nussig, haselnussig, pistazieartig, kakaoartig, karamellig, fleischig, röstgetreideartig, schokoladig und gewürzartig.

8. Riech- und/oder Geschmacksstoffkomposition nach einem der Ansprüche 6 oder 7, wobei der Bestandteil (b) keine Riech- und/oder Geschmacksstoffe enthält, die eine Pyrazingrundstruktur aufweisen.

9. Riech- und/oder Geschmacksstoffkomposition nach einem der Ansprüche 6 bis 8, wobei Bestandteil (a) einen oder mehrere Geruchseindrücke des Bestandteils (b) verstärkt, wobei vorzugsweise ein floraler und/oder ein fruchtiger Geruchseindruck des Bestandteils (b) verstärkt wird.

10. Riech- und/oder Geschmacksstoffkomposition nach einem der Ansprüche 6 bis 9, wobei die Gesamtmenge an Verbindungen der Formel (I) im Bereich von 0,0001 bis 90 Gew.-%, vorzugsweise im Bereich von 0,01 bis 70 Gew.-%, bevorzugt im Bereich von 0,1 bis 50 Gew.-%, besonders bevorzugt im Bereich von 0,1 bis 30 Gew.-%, ganz besonders bevorzugt im Bereich von 0,1 bis 10 Gew.-% liegt, bezogen auf das Gesamtgewicht der Riech- und/oder Geschmacksstoffkomposition.

11. Parfümierte und/oder aromatisierte Artikel, umfassend eine Riech- und/oder Geschmacksstoffkomposition nach einem der Ansprüche 6 bis 10.

12. Artikel nach Anspruch 11, weiter umfassend einen Träger oder ein Substrat, der bzw. das in direktem Kontakt mit der oder den Verbindungen der Formel (I) bzw. der Riech- und/oder Geschmacksstoffkomposition steht.

13. Verfahren zum Verstärken eines Geruchseindruckes, vorzugsweise eines floralen und/oder fruchtigen Geruchseindruckes, umfassend folgenden Schritt:
Inkontaktbringen oder Mischen
- einer Verbindung der Formel (I) wie in einem der Ansprüche 1 bis 4 oder 5 definiert,
- einer Mischung aus zwei, drei, vier, fünf, sechs oder mehr verschiedenen Verbindungen der Formel (I),
wie in einem der Ansprüche 1 bis 4 oder 5 definiert,
oder
- einer Riech- und/oder Geschmacksstoffkomposition nach einem der Ansprüche 6 bis 11, mit einem Erzeugnis.

## Claims

1. Use of
• a compound of formula (I) 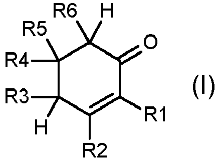 or
• a mixture consisting of or comprising two, three, four, five, six or more various compounds of formula (I),
wherein in each of the compounds of formula (I), the residues R1, R2, R3, R4, R5 and R6 respectively, independently of one another have the following meaning:
R1 denotes hydrogen, methyl or ethyl,
R2 denotes hydrogen, methyl or ethyl,
R3 denotes hydrogen, methyl, ethyl or isopropyl,
R4 denotes hydrogen, methyl, ethyl, n-propyl or isopropyl,
R5 denotes hydrogen or methyl,
R6 denotes hydrogen, methyl or ethyl,
with the provisio that R1 is not methyl or ethyl when R6 is methyl or ethyl for the superadditive enhancement of an olfactory impression.

2. Use of a single compound or a mixture as claimed in claim 1, wherein the single compound of formula (I) respectively one, several or all compounds of formula (I) is selected from, respectively is independently from each other selected from the group consisting of the following compounds (1) to (61), preferably selected from the group consisting of the compounds (2), (4), (13), (23), (29), (43) and (51):

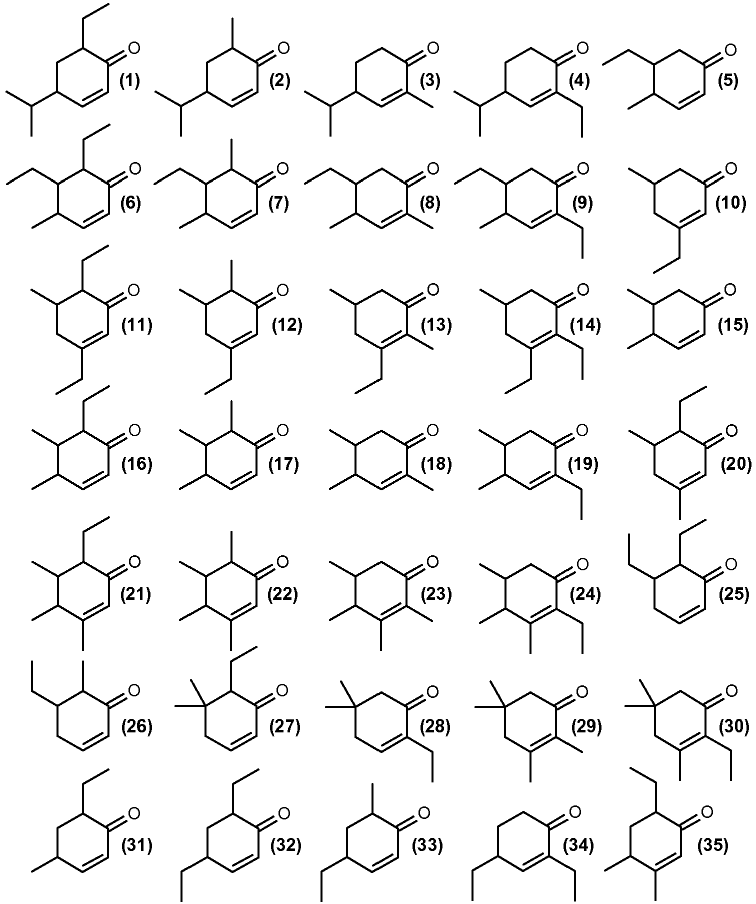

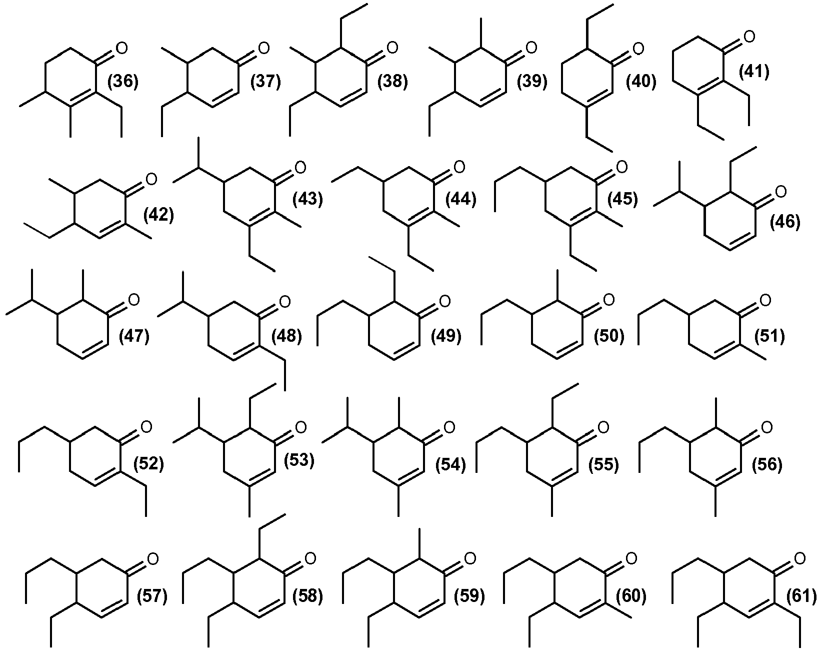

3. Use as claimed in claim 1 for the superadditive enhancement of an olfactory impression selected from the group consisting of floral and/or fruity impressions.

4. Use as claimed in any of the preceding claims, wherein the olfactory impression to be enhanced is not selected from the group consisting of nutty, hazelnut, pistachio, cacao, caramel, meat, roasted grain, chocolate and spicy and/or the compound of formula (I) or the respective mixture does not impart an olfactory impression selected from the group consisting of nutty, hazelnut, pistachio, cacao, caramel, meat, roasted grain, chocolate and spicy.

5. (A) Compound of formula (I) 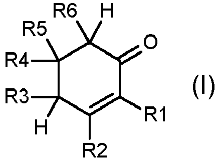 as defined in any of the preceding claims or
(B) mixture consisting of or comprising two, three, four, five, six or more various compounds of formula (I) as defined in any of the preceding claims.
wherein (A) is the compound of formula (I) respectively (B) is one, several or all compounds of formula (I) selected in a mixture respectively each independently from each other selected from the group consisting of the flowing compounds: 6-ethyl-4-isopropylcyclohex-2-en-1-one (1), 5,6-diethyl-4-methylcyclohex-2-en-1-one (6), 5-ethyl-4,6-dimethylcyclohex-2-en-1-one (7), 2,5-diethyl-4-methylcyclohex-2-en-1-one (9), 3-ethyl-5-methylcyclohex-2-en-1-one (10), 3,6-diethyl-5-methylcyclohex-2-en-1-one (11), 3-ethyl-5,6-dimethylcyclohex-2-en-1-one (12), 2,3-diethyl-5-methylcyclohex-2-en-1-one (14), 6-ethyl-4,5-dimethylcyclohex-2-en-1-one (16), 4,5,6-trimethylcyclohex-2-en-1-one (17), 2,4,5-trimethylcyclohex-2-en-1-one (18), 2-ethyl-4,5-dimethylcyclohex-2-en-1-one (19), 6-ethyl-3,5-dimethylcyclohex-2-en-1-one (20), 6-ethyl-3,4,5-trimethylcyclohex-2-en-1-one (21), 3,4,5,6-tetramethylcyclohex-2-en-1-one (22), 2,3,4,5-tetramethylcyclohex-2-en-1-one (23), 2-ethyl-3,4,5-trimethylcyclohex-2-en-1-one (24), 5,6-diethylcyclohex-2-en-1-one (25), 5-ethyl-6-methyl-cyclohex-2-en-1-one (26), 6-ethyl-5,5-dimethylcyclohex-2-en-1-one (27), 2-ethyl-5,5-dimethyl-cyclohex-2-en-1-one (28), 6-ethyl-4-methylcyclohex-2-en-1-one (31), 4,6-diethylcyclohex-2-en-1-one (32), 4-ethyl-6-methylcyclohex-2-en-1-one (33), 2,4-diethylcyclohex-2-en-1-one (34), 6-ethyl-3,4-dimethylcyclohex-2-en-1-one (35), 2-ethyl-3,4-dimethylcyclohex-2-en-1-one (36), 4-ethyl-5-methylcyclohex-2-en-1-one (37), 4,6-diethyl-5-methylcyclohex-2-en-1-one (38), 4-ethyl-5,6-dimethylcyclohex-2-en-1-one (39), 3,6-diethylcyclohex-2-en-1-one (40), 2,3-diethylcyclohex-2-en-1-one (41), 4-ethyl-2,5-dimethylcyclohex-2-en-1-one (42), 3-ethyl-5-isopropyl-2-methylcyclohex-2-en-1-one (43), 3,5-diethyl-2-methylcyclohex-2-en-1-one (44), 3-ethyl-2-methyl-5-n-propylcyclohex-2-en-1-one (45), 6-ethyl-5-isopropylcyclohex-2-en-1-one (46), 5-isopropyl-6-methylcyclohex-2-en-1-one (47), 2-ethyl-5-isopropylcyclohex-2-en-1-one (48), 6-ethyl-5-n-propylcyclohex-2-en-1-one (49), 6-methyl-5-n-propylcyclohex-2-en-1-one (50), 2-methyl-5-n-propylcyclohex-2-en-1-one (51), 2-ethyl-5-n-propylcyclohex-2-en-1-one (52), 6-ethyl-5-isopropyl-3-methylcyclohex-2-en-1-one (53), 5-isopropyl-3,6-dimethylcyclohex-2-en-1-one (54), 6-ethyl-3-methyl-5-n-propylcyclohex-2-en-1-one (55), 3,6-dimethyl-5-n-propylcyclohex-2-en-1-one (56), 4-ethyl-5-n-propylcyclohex-2-en-1-one (57), 4,6-diethyl-5-n-propylcyclohex-2-en-1-one (58), 4-ethyl-6-methyl-5-n-propylcyclohex-2-en-1-one (59), 4-ethyl-2-methyl-5-n-propylcyclohex-2-en-1-one (60) and 2,4-diethyl-5-n-propylcyclohex-2-en-1-one (61),
preferably selected from the group consisting of
2,3,4,5-tetramethylcyclohex-2-en-1-one (23), 3-ethyl-5-isopropyl-2-methylcyclohex-2-en-1-one (43) and 2-methyl-5-n-propylcyclohex-2-en-1-one (51).

6. Fragrance and/or flavor material composition comprising or consisting of
(a) a compound of formula (I) or a mixture of two, three, four, five, six or more various compounds of formula (I) as defined in any of the preceding claims, preferably as defined in claim 5,
and
(b) one, two, three or more further fragrance and/or flavor, wherein the one respectively the further fragrances and/or flavors are not compounds of formula (I),
wherein the one, respectively one, two, three, more or all of the fragrances and/or flavors according as component (b) exhibit a floral and/or fruity odor impression.

7. Fragrance and/or flavor material composition as claimed in claim 6, wherein the one respectively one, two, three, more or all of the further fragrances and/or flavors according as component (b)
• either does not impart an olfactory impression selected from the group consisting of nutty, hazelnut, pistachio, cacao, caramel, meat, roasted grain, chocolate and spicy, or impart an olfactory impression selected from the group consisting of nutty, hazelnut, pistachio, cacao, caramel, meat, roasted grain, chocolate and spicy, wherein the olfactory impression is not being enhanced superadditively
and/or
wherein the compound of formula (I) or the correspond mixture does not impart an odor impression selected from the group consisting of nutty, hazelnut, pistachio, cacao, caramel, meat, roasted grain, chocolate and spicy.

8. Fragrance and/or flavor material composition as claimed in claims 6 or 7, wherein the component (b) does not contain fragrances and/or flavors materials that have a pyrazine basic structure.

9. Fragrance and/or flavor material composition as claimed in claims 6 to 8, wherein component (a) enhances one or several of the olfactory impressions of component (b), wherein preferably a floral and/or fruity odor impression of component (b) is enhanced.

10. Fragrance and/or flavor material composition as claimed in claims 6 to 9, wherein the total amount of compounds of formula (I) is in the range from 0,0001 to 90 wt.%, preferably in the range from 0,01 to 70 wt.%, preferably in the range from 0,1 to 50 wt.%, more preferred in the range from 0,1 to 30 wt.%, most preferred in the range from 0,1 to 10 wt.%, based on the total weight of the fragrance and/or flavor material.

11. Perfumed and/or aromatized article comprising the fragrance and/ or flavor material composition according to any claims 6 to 10.

12. Article of claim 11, further comprising a carrier or a substrate that is in direct contact with at least one of the one or more compounds of formula (I) respectively of the fragrance and/or flavor material composition.

13. Method for the enhancement of an olfactory impression, preferably of a floral and/or fruity odor impression, comprising the following step:
bringing in contact or mixing
• a compound of formula (I), as defined in any claims 1 to 4 or 5,
• a mixture of two, three, four, five, six, or more various compounds of formula (I),
as defined in any claims 1 to 4 or 5,
or
• a fragrance and/or flavor material composition as claimed in claims 6 to 11,
with a product.

## Revendications

1. Utilisation
• d'un composé de formule (I) 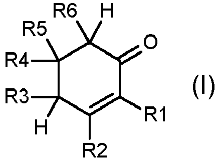 ou
• d'un mélange consistant de ou comprenant deux, trois, quatre, cinq, six ou plus composés différents de formule (I)
dans lequel les restes R1, R2, R3, R4, R5 et R6 de chaque composé de formule (1) ont chacun indépendamment les uns des autres la signification suivante
R1 signifie l'hydrogène, le méthyle ou l'éthyle,
R2 signifie l'hydrogène, le méthyle ou l'éthyle,
R3 signifie l'hydrogène, le méthyle, l'éthyle ou l'isopropyle,
R4 signifie l'hydrogène, le méthyle, l'éthyle, le n-propyle ou l'isopropyle,
R5 signifie l'hydrogène ou le méthyle
R6 signifie l'hydrogène, le méthyle ou l'éthyle,
à condition que R1 n'est pas le méthyle ou l'éthyle si R6 est le méthyle ou l'éthyle pour un renforcement significatif d'une impression d'odeur.

2. Utilisation d'un composé en soi ou d'un mélange selon la revendication 1, dans lequel le composé en soi de formule (I) ou une, plusieurs ou tous les composés de formule (I) est choisi ou sont choisis chacun indépendamment les uns des autres parmi le groupe consistant des composés (1) à (61), de préférence choisis parmi le groupe consistant des composés (2), (4), (13), (23), (29), (43) et (51):

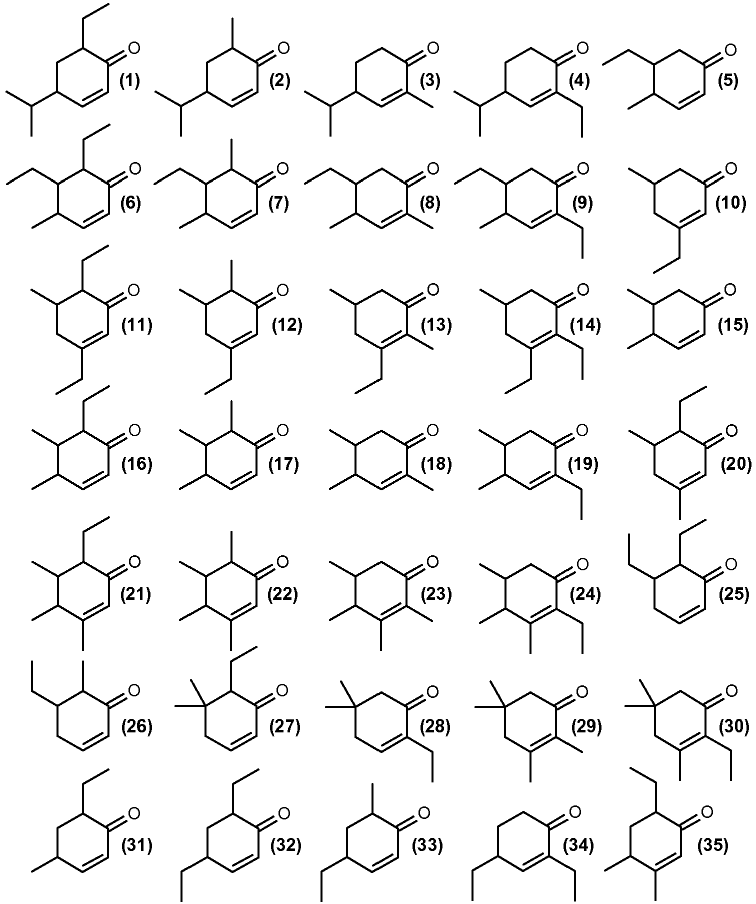

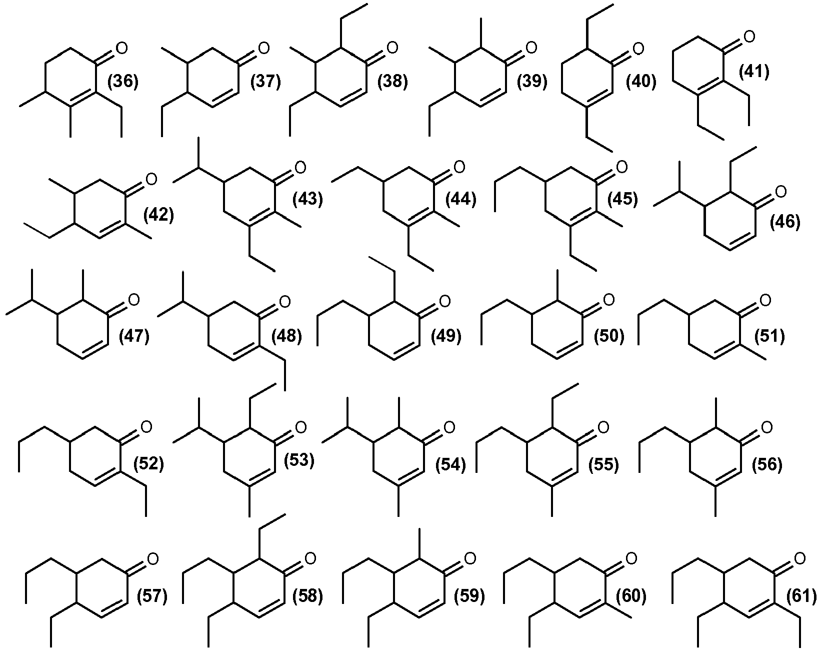

3. Utilisation selon les revendications 1 ou 2 pour un renforcement significatif d'une impression d'odeur choisi parmi le groupe consistant d'une impression d'odeur florale et/ou fruitée.

4. Utilisation selon l'une quelconque des revendications précédentes, dans lequel l'impression d'odeur à renforcer n'est pas choisi parmi le groupe consistant de noix, de pistache, de cacao, de caramel, de chair, de céréales soufflées, de chocolat et d'épice et/ou dans lequel le composé de formule (I) ou le mélange correspondant ne transmet aucune impression d'odeur qui est choisi parmi le groupe consistant de noix, de pistache, de cacao, de caramel, de chair, de céréales soufflées, de chocolat et d'épice.

5. (A) Composé de formule (I) 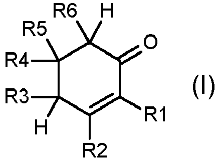 comme défini selon l'une quelconque des revendications précédentes ou
(B) un mélange consistant de ou contenant deux, trois, quatre, cinq, six ou plus composés différents de formule (I) comme défini selon l'une quelconque des revendications précédentes,
dans lequel (A) du composé de formule (I) ou (B) un, plusieurs ou tous les composés de formule (I) du mélange est choisi ou sont choisis chacun indépendamment les uns des autres parmi le groupe consistant des composés suivants : 6-éthyle-4-isopropylcyclohex-2-en-1-on (1), 5,6-diéthyle-4-méthylecyclohex-2-en-1-on (6), 5-éthyle-4,6-diméthylecyclohex-2-en-1-on (7), 2,5-diéthyle-4-méthyelcyclohex-2-en-1-on (9), 3-éthyle-5-méthylecyclohex-2-en-1-on (10), 3,6-diéthyle-5-méthylecyclohex-2-en-1-on (11), 3-éthyle-5,6-diméthylecyclohex-2-en-1-on (12), 2,3-diéthyle-5-méthylecyclohex-2-en-1-on (14), 6-éthyle-4,5-diméthylecyclohex-2-en-1-one (16), 4,5,6-triméthylecyclohex-2-en-1-on (17), 2,4,5-triméthylecyclohex-2-en-1-on (18), 2-éthyle-4,5-diméthylecyclohex-2-en-1-on (19), 6-éthyle-3,5-diméthylcyclohex-2-en-1-on (20), 6-éthyle-3,4,5-triméthylecyclohex-2-en-1-on (21), 3,4,5,6-tetraméthylecyclohex-2-en-1-on (22), 2,3,4,5-tetraméthylecyclohex-2-en-1-on (23), 2-éthyle-3,4,5-triméthylecyclohex-2-en-1-on (24), 5,6-diéthylecyclohex-2-en-1-on (25), 5-éthyle-6-méthylecyclohex-2-en-1-on (26), 6-éthyle-5,5-diméthylecyclohex-2-en-1-on (27), 2-éthyle-5,5-diméthylecyclohex-2-en-1-on (28), 6-éthyle-4-méthylecyclohex-2-en-1-on (31), 4,6-di-éthylecyclohex-2-en-1-on (32), 4-éthyle-6-méthylecyclohex-2-en-1-on (33), 2,4-diéthyle-cyclohex-2-en-1-on (34), 6-éthyle-3,4-diméthylecyclohex-2-en-1-on (35), 2-éthyle-3,4-diméthylecyclohex-2-en-1-on (36), 4-éthyle-5-méthylecyclohex-2-en-1-on (37), 4,6-diéthyle-5-méthylecyclohex-2-en-1-on (38), 4-éthyle-5,6-diméthylecyclohex-2-en-1-on (39), 3,6-diéthylecyclohex-2-en-1-on (40), 2,3-diéthylecyclohex-2-en-1-on (41), 4-éthyle-2,5-diméthylecyclohex-2-en-1-on (42), 3-éthyle-5-isopropyl-2-méthylecyclohex-2-en-1-on (43), 3,5-diéthye-2-méthylecyclohex-2-en-1-on (44), 3-éthyle-2-méthyle-5-n-propylcyclohex-2-en-1-on (45), 6-éthyle-5-isopropylcyclohex-2-en-1-on (46), 5-isopropyl-6-méthylecyclohex-2-en-1-on (47), 2-éthyle-5-isopropylcyclohex-2-en-1-on (48), 6-éthyle-5-n-propylcyclohex-2-en-1-on (49), 6-méthyle-5-n-propylcyclohex-2-en-1-on (50), 2-méthyle-5-n-propylcyclohex-2-en-1-on (51), 2-éthyle-5-n-propylcyclohex-2-en-1-on (52), 6-éthyle-5-isopropyl-3-méthylecyclohex-2-en-1-on (53), 5-isopropyl-3,6-diméthylecyclohex-2-en-1-on (54), 6-éthyle-3-méthyle-5-n-propylcyclohex-2-en-1-on (55), 3,6-diméthyle-5-n-propylcyclohex-2-en-1-on (56), 4-éthyle-5-n-propylcyclohex-2-en-1-on (57), 4,6-diéthyle-5-n-propylcyclohex-2-en-1-on (58), 4-éthyle-6-méthyle-5-n-propylcyclohex-2-en-1-on (59), 4-éthyle-2-méthyle-5-n-propylcyclohex-2-en-1-on (60) and 2,4-diéthyle-5-n-propylcyclohex-2-en-1-on (61),
de préférence choisi parmi le groupe consistant de
2,3,4,5-tetraméthylecyclohex-2-en-1-on (23), 3-éthyle-5-isopropyl-2-méthylecyclohex-2-en-1-on (43) et 2-méthyle-5-n-propylcyclohex-2-en-1-on (51).

6. Une composition d'arôme et/ou de saveur contenant ou consistant de (a) un composé de formule (I) ou un mélange de deux, trois, quatre, cinq, six ou plus composés différents de formule (I) comme défini selon l'une quelconque des revendications précédentes, de préférence comme défini selon la revendication 5,
et
(b)un, deux, trois ou plus autres substances d'arôme et/ou de saveur dans lequel l'autre substance ou les autres substances d'arôme et/ou de saveur ne sont pas de formule (I),
dans lequel un, deux, trois, plus ou tous les substances d'arôme et/ou de saveur ont une odeur florale et/ou fruitée selon le composé (b).

7. Une composition d'arôme et/ou de saveur selon la revendication 6 dans lequel l'un ou deux, trois, plus ou tous les substances d'arôme et/ou de saveur selon le composé (b)
- ne transmettent pas de l'impression d'odeur qui est choisi parmi le groupe consistant de noix, de pistache, de cacao, de caramel, de chair, de céréales soufflées, de chocolat et d'épice ou transmettent une impression d'odeur qui est choisi parmi le groupe consistant de noix, de pistache, de cacao, de caramel, de chair, de céréales soufflées, de chocolat et d'épice dans lequel cette impression d'odeur n'est pas renforcée significativement
et/ou
dans lequel le composé de formule (I) ou le mélange correspondant ne transmet pas de l'impression d'odeur qui est choisi parmi le groupe consistant de noix, de pistache, de cacao, de caramel, de chair, de céréales soufflées, de chocolat et d'épice.

8. Une composition d'arôme et/ou de saveur selon une des revendications 6 ou 7 dans lequel le composé (b) ne comporte pas des substances d'arôme et/ou de saveur qui ont une structure générale de pyrazine.

9. Une composition d'arôme et/ou de saveur selon une des revendications 6 à 8 dans lequel le composé (a) renforce une ou plusieurs impressions d'odeur de composé (b) dans lequel une impression d'odeur florale et/ou fruitée du composé (b) est renforcée.

10. Une composition d'arôme et/ou de saveur selon une des revendications 6 à 9 dans lequel la quantité totale de composés de formule (I) est dans la gamme de 0,0001 à 90 % en poids, de préférence dans la gamme de 0,01 à 70 % en poids, de préférence dans la gamme de 0,1 à 50 % en poids, de façon particulièrement préférée dans la gamme de 0,1 à 30 % en poids, de façon plus particulièrement préférée dans la gamme de 0,1 à 10 % en poids par rapport au poids total des compositions d'arôme et/ou de saveur.

11. Des articles parfumés et/ou aromatisé comprenant une composition d'arôme et/ou de saveur selon une des revendications 6 à 10.

12. Des articles selon la revendication 11 comprenant en outre un support ou un substrat qui est en contact direct avec le composé ou les composés de formule (I) ou des compositions d'arôme et/ou de saveur.

13. Un procédé pour le renforcement d'une impression d'odeur, de préférence d'une impression d'odeur florale et/ou fruitée comprenant les étapes suivantes :
La mise en contact ou le mélange de
• un composé de formule (I)
comme défini selon une des revendications 1 à 4 ou 5,
• un mélange de deux, trois, quatre, cinq, six ou plus composés différents de formule (I) comme défini selon une des revendications 1 à 4 ou 5,
ou
• une composition d'arôme et/ou de saveur selon une des revendications 6 à 11 avec un produit.
